(19) Europäisches Patentamt
European Patent Office
Office européen des brevets

(11) EP 3 907 280 A1

(12) EUROPEAN PATENT APPLICATION
published in accordance with Art. 153(4) EPC

(43) Date of publication:
**10.11.2021 Bulletin 2021/45**

(21) Application number: **18943085.3**

(22) Date of filing: **13.12.2018**

(51) Int Cl.:
*C12N 5/10* (2006.01) *C12N 15/867* (2006.01)

(86) International application number:
**PCT/CN2018/120938**

(87) International publication number:
**WO 2020/118634 (18.06.2020 Gazette 2020/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Carsgen Therapeutics Co., Ltd.**
  **Xuhui District**
  **Shanghai 200231 (CN)**
- **Shanghai Cancer Institute**
  **Shanghai 200032 (CN)**

(72) Inventors:
- **LI, Zonghai**
  **Shanghai 200231 (CN)**
- **JIANG, Hua**
  **Shanghai 200231 (CN)**

(74) Representative: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(54) **IMMUNE EFFECTOR CELL TARGETING GPC3 AND APPLICATION THEREOF**

(57) The present invention relates to an immune effector cell which expresses a chimeric antigen receptor targeting GPC3 and exogenous IL12. The present invention further provides a pharmaceutical composition containing the immune effector cell and a method for treating tumor, particularly GPC3 positive tumor by using the immune effector cell or the pharmaceutical composition. The immune effector cell in the present invention is effective to an entity tumor cell in vitro, and is outstanding in effect of extinguishing the entity tumor cell in vivo.

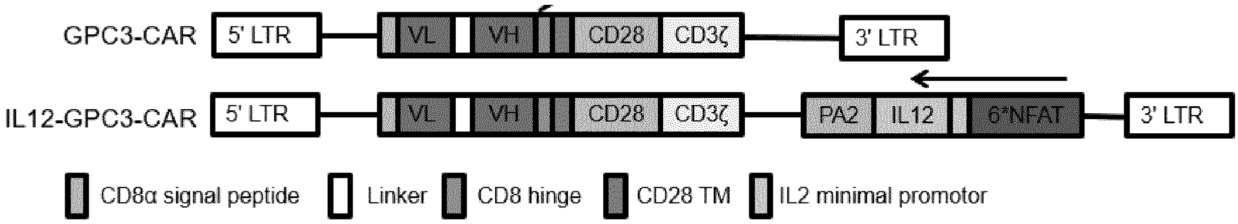


Fig. 1A

EP 3 907 280 A1

## Description

### Technical field

The present invention relates to the field of cellular immunotherapy, and in particular, to a genetically modified immune effector cell targeting GPC3.

### Background

Chimeric antigen receptor engineered T lymphocyte (CAR-T) technology is a new strategy for tumor biotherapy that has emerged in recent years. Chimeric antigen receptor (CAR) combines a single-chain antibody recognizing a tumor-associated antigen and an activating motif of immune cells, and T lymphocytes can express chimeric antigen receptors *in vitro* by means of genetic engineering, thereby rendering T cells the ability to specifically recognize and kill tumor cells. At present, good results in the treatment of hematological malignancies have been achieved by CAR-T. For example, great success has been achieved in patients of CD19-positive B lymphocytic leukemia. However, CAR-T is still faced with great challenges in the treatment of solid tumors.
Interleukin-12 (IL12) is an important immunostimulatory cytokine that can promote the proliferation of helper T cells and induce NK cells and T cells to produce interferon-gamma. However, in clinical trials, it was found that serious side effects occurred in multiple organs during systemic administration of IL12, such as abnormal liver function, high fever, severe hemodynamic instability, etc. The severe cases resulted in death, so that the application of IL12 is restricted.
In addition, due to the particularity of tumors, especially solid tumors, the microenvironment of each tumor is completely different. It is unknown whether immune effector cells co-expressing IL12 can have enhanced anti-tumor effects, especially the significantly enhanced anti-tumor effects, and certain side effects will be caused, such as weight loss of experimental mice. In addition, the combination of CAR T cells and other cytokines also exhibits disadvantages, such as great side effects or poor *in vivo* effects. Therefore, effects produced by specific chimeric antigen receptor immune effector cells in combination with specific cytokines cannot be reasonably expected.
Therefore, there is an urgent need in the art for immune effector cells haing significant anti-tumor effects but low side effects.

### Summary of the invention

The object of the present invention is to provide an immune effector cell with significant anti-tumor effects but low side effects.
In the first aspect, the present invention provides an immune effector cell expressing a receptor and exogenous IL12, wherein the receptor specifically binds to GPC3.
In a specific embodiment, the receptor and the IL12 are operably linked.
In a specific embodiment, the immune effector cells include: T cells, natural killer cells, cytotoxic T lymphocytes, natural killer T cells, DNT cells, and/or regulatory T cells.
In a specific embodiment, the exogenous IL12 is constitutively or inductively expressed; preferably, the promoter used to express the IL12 includes: an immune cell inducible promoter; and preferably, the immune cell inducible promoter is NFAT6 promoter.
In a specific embodiment, the receptor has an extracellular domain specifically binding to GPC3, a transmembrane domain, and an intracellular signal domain.
In a specific embodiment, the receptor is a chimeric antigen receptor, wherein the intracellular domain contains a cell stimulating signal molecule or a combination of a cell stimulating signal molecule and a cell activating co-stimulatory molecule;

Preferably, the cell stimulating signal molecule is selected from the functional signaling domain of a protein: CD3ζ, CD3γ, CD3δ, CD3ε, FcεRIγ, FcRβ, CD79a, CD79b, FcyRIIa, DAP10, or DAP 12; more preferably CD3ζ; or
Preferably, the cell activating co-stimulatory molecule is selected from the functional signaling domain of the following proteins: CD27, CD28, CD137, CD134, ICOS, OX40, CD30, CD40, PD-1, lymphocyte function related antigen -1 (LFA-1), CD2, CD7, LIGHT, NKG2C, B7-H3, a ligand that specifically binds to CD83, CDS, ICAM-1, GITR, BAFFR, HVEM (LIGHTR), SLAMF7, NKp80 (KLRF1), CD160 , CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1 , ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, and NKG2D, more preferably, CD27, CD28, CD137, CD134, ICOS.

In a specific embodiment, the extracellular domain of the receptor contains an amino acid sequence that is at least 90%, 95%, 96%, 97%, 98%, or 99% identical with the amino acid sequence shown in SEQ ID NO: 25.
In a specific embodiment, the receptor contains the amino acid sequence shown in SEQ ID NO: 21, 22, 23, or 24; or contains an amino acid sequence that is at least 90%, 95%, 96%, 97%, 98%, or 99% identical with the amino acid sequence shown in SEQ ID NO: 21, 22, 23 or 24.
In a specific embodiment, the receptor and the exogenous IL12 are encoded by a nucleotide sequence that is at least 90%, 95%, 96%, 97%, 98%, or 99% identity with SEQ ID NO: 27, 28, or 29.
In a specific embodiment, the immune effector cells do not contain exogenous co-stimulatory ligands.
In a specific embodiment, the receptor and/or the IL12 are constitutively or inducibly expressed on the surface of immune effector cells.
In a specific embodiment, the immune effector cell contains an expression construct, which includes: an expression cassette of the antigen-binding receptor; and an expression cassette of the IL12.
In a specific embodiment, the receptor and/or IL12 are expressed by using a viral vector; and preferably, the viral vector includes: a lentiviral vector, retroviral vector or adenoviral vector.
In a specific embodiment, after the immune effector cells are administered to an individual, the number of CAR-T cells in the peripheral blood of the individual is increased by at least 50%, compared with the case where the exogenous IL12 is not present.
In a specific embodiment, about 7 days after the immune effector cells are administered to the individual, the sum of the number of CAR-T cells in the individual's peripheral blood is greater than 6,000/μL; and about 10 days after the immune effector cells are administered, the sum of the number of CAR-T cells in the individual's peripheral blood is greater than 6,000/μL.
In a preferred embodiment, the immune effector cells of the present invention exhibit excellent killing effects on tumors that cannot be controlled by a low dose, such as a dose of less than $1*10^7$ cells/L of immune effector cells not of the present invention (that is, immune effector cells which do not express a receptor specifically binding to GPC3 and exogenous IL12 simultaneously), or tumors that are too large (such as tumors larger than 300 $mm^3$ in the mouse tumor model, or even larger than 600 $mm^3$).
In the second aspect, the present invention provides an expression construct, including an expression cassette of a receptor and an expression cassette of IL12, which are connected in sequence; wherein the antigen-binding receptor and IL12 are described as in the first aspect of the present invention.
In a third aspect, the present invention provides the use of the immune effector cells described in the first aspect of the present invention in the preparation of a pharmaceutical composition for treating tumors in an individual in need thereof.
In a specific embodiment, the tumor includes: liver cancer, stomach cancer, lung cancer, breast cancer, head and neck cancer, bladder cancer, ovarian cancer, cervical cancer, kidney cancer, pancreatic cancer, cervical cancer, liposarcoma, melanoma, adrenal carcinoma, Schwannoma, malignant fibrous histiocytoma, esophageal cancer; and preferably, the tumor is liver cancer, gastric cancer, lung cancer, or breast cancer.
In a specific embodiment, the immune effector cells reduce the tumor by at least 50%.
In a specific embodiment, the immune effector cells reduce the tumor by at least 70%, and more preferably, the immune effector cells reduce the tumor by at least 80%.
In the fourth aspect, the present invention provides a pharmaceutical composition, comprising:

the immune effector cell of the first aspect of the present invention; and
a pharmaceutically acceptable carrier or excipient.

In the fifth aspect, the present invention provides a kit comprising:

the immune effector cell of the first aspect of the present invention; and
instructions on how to administer the immune effector cell to an individual.

It should be understood that, within the scope of the present invention, the above-mentioned technical features of the present invention and the technical features specifically described in the following contents (such as the Examples) can be combined with each other to form a new or preferred technical solution, which will not be repeated one by one due to the limited contents herein.

## Description of drawings

Figure 1A is a schematic diagram of the structure of a plasmid prepared for CAR-T cells; and Figure 1B shows the expression of CAR on the surface of GPC3-CAR-T/IL12-GPC3-CAR-T cells detected by FACS;
Figure 2 shows the experimental results of *in vitro* toxicity of CAR-T cells;
Figure 3A shows the secretion of IL-12 when CAR-T cells and tumor cells are co-incubated for 24 hours; and Figure 3B shows the secretion of IL-2/TNF-α/IFN-γ when CAR-T cells and tumor cells are co-incubated for 24 hours;
Figure 4A shows the growth of Huh-7 subcutaneously transplanted tumors in different experimental groups; and Figure 4B shows changes in the weight of mice with Huh-7 subcutaneously transplanted tumors in different experimental groups;
Figure 5 shows the number of surviving cells of different CAR-T in peripheral blood on day 7 and day 10;
Figure 6 shows the amount of IFN-γ in serum samples on day 10 after the injection of CAR-T cells;
Figure 7 shows the results of immunohistochemical detection of infiltrating T cells and Ki67 in tumor tissues;
Figures 8A and 8B show the growth of subcutaneous tumors in mice at the end of the experiment;
Figure 9 shows the expression of CAR on the surface of mCAR-T/mIL12-GPC3-CAR T cells detected by FACS;
Figure 10 shows the experimental results of *in vitro* toxicity of mCAR-T;
Figure 11A shows the secretion of mIL-12 when UTD, mGPC3-CAR, mGPC3-m28Z-mNFAT6-mIL12 are incubated with tumor cells for 24 hours; and Figure 11B shows the release of cytokines mIL-2, mTNF-α and mIFN-γ when UTD/mGPC3-m28Z/mIL12-GPC3-CAR T and tumor cells are co-incubated for 24 hours;
Figure 12A and Figure 12B show therapeutic effects on E0771-GPC3 subcutaneous transplantation tumor;
Figure 13A shows the experimental results of the group, in which mIL12-GPC3-T 5×106/animal was injected through vein injection, and the group, in which mIL12-GPC3-T 2×106/animal was injected through vein injection; and Figure 13B shows the body weights of mice in mGPC3-CAR-T group and blank control group, after mIL12-GPC3-T were administered.

## Modes for carrying out the invention

After extensive and in-depth research, the inventors unexpectedly discovered that the expression of a receptor that specifically binds to GPC3 and exogenous IL12 in immune effector cells will have significant anti-tumor effects, but with low side effects, based on which the present invention has been completed.

The following detailed description shows the embodiments disclosed herein in detail. It should be understood that this specification is not intended to be limited to the specific embodiments disclosed herein, which may be changed. A skilled person in the art will understand that there may be various changes or modifications of the contents disclosed in this specification, all of which shall fall within the scope and principle of the disclosure. Unless otherwise specified, each embodiment can be arbitrarily combined with any other embodiment.

Certain embodiments disclosed herein include numerical ranges, and certain aspects of the present invention may be described in terms of ranges. Unless otherwise specified, it should be understood that a numerical range or the description of a range is provided only for brevity and convenience, while should not be considered as a strict limitation on the scope of the present invention. Therefore, the description of a range should be regarded as specifically disclosing all possible sub-ranges and all possible specific numerical points within the range, just as these sub-ranges and numerical points have been clearly written herein. For example, the description of a range from 1 to 6 should be considered as specifically disclosing sub-ranges from 1 to 3, 1 to 4, 1 to 5, 2 to 4, 2 to 6, 3 to 6, and the like, and specific numerical points within these ranges, such as 1, 2, 3, 4, 5, 6. Regardless of the width of the stated range, the above principles are equally applicable. When a range is described, the endpoints of the range are also included in the range.

**术语术语术语术语 Terms**

As used herein, the terms "activation" and "activating" can bee used interchangeably, and they and other grammatical forms can refer to a process by which a cell changes from a resting state to an active state. The process may include a response to phenotypic or genetic changes of antigen, migration, and/or functional activity status. For example, the term "activation" can refer to a process of gradual activation of T cells. For example, at least two signals may be required to fully activate T cells. The first signal can occur after the conjugation of TCR with antigen-MHC complex, and the second signal can occur through the conjugation of co-stimulatory molecules (see the costimulatory molecules listed in Table 3). *In vitro,* anti-CD3 can simulate the first signal, and anti-CD28 can simulate the second signal. For example, engineered T cells can be activated by expressed CAR. T cell activation or T cell triggering as used herein may refer to the state of T cells that have been sufficiently stimulated to induce detectable cell proliferation, cytokine production, and/or detectable effector functions.

The term "receptor" as used herein refers to a special type of protein that exists in the cell membrane or inside the cell, and can bind to a specific signal molecule outside the cell to activate a series of biochemical reactions in the cell, so that the cell produces corresponding effects on external stimuli. protein. Biologically active substances that bind to the receptor are collectively referred to as ligands. The binding of a receptor and a ligand results in a molecular conformational change, which causes cellular responses, for example mediating a process, such as intercellular signaling, intercellular adhesion, and endocytosis.

The term "co-stimulatory ligand" as used herein includes molecules on antigen-presenting cells (e.g., aAPC, dendritic cells, B cells, etc.) that specifically bind to identical co-stimulatory molecules on T cells, thereby providing signals, and mediating the T cell response together with the first signal provided by, for example, the binding of the TCR/CD3 complex and the peptide-loaded MHC molecule, including but not limited to proliferation, activation, differentiation, and the like. Co-stimulatory ligands can include but are not limited to CD7, B7-1 (CD80), B7-2 (CD86), PD-L, PD-L2, 4-1BBL, OX40L, inducible co-stimulatory ligand (ICOS-L), intercellular adhesion molecule (ICAM), CD30L, CD40, CD70, CD83, HLA-G, MICA, MICB, HVEM, lymphotoxin β receptor, 3/TR6, ILT3, ILT4, HVEM, agonists or antibodies binding to Toll ligand receptors and ligands that specifically binds to B7-H3. Co-stimulatory ligands also specifically include antibodies that specifically bind to co-stimulatory molecules present on T cells, such as but not limited to CD27, CD28, 4-1BB, OX40, CD30, CD40, PD-1, ICOS, lymphocyte function related Antigen-1 (LFA-1), CD2, CD7, LIGHT, NKG2C, B7-H3 and ligands that specifically bind to CD83.

The term "co-stimulatory molecule" as used herein refers to an identical binding partner on a T cell that specifically binds to a co-stimulatory ligand, thereby mediating a co-stimulatory response of the T cell, such as but not limited to proliferation. Co-stimulatory molecules include but are not limited to MHC class I molecules, BTLA and Toll ligand receptors.

"Co-stimulatory signal" as used herein refers to a signal that binds to cell stimulatory signal molecules, such as TCR/CD3, thereby leading to up- or down-regulation of T cell proliferation and/or key molecules.

The term "chimeric antigen receptor" or "CAR" as used herein refers to an engineered molecule that can be expressed by immune cells including but not limited to T cells. CAR is expressed in T cells and can redirect T cells to induce the specific killing of target cells determined by artificial receptors. The extracellular binding domain of CAR can be derived from murine, humanized or fully human monoclonal antibodies.

The term "engineered" and other grammatical forms thereof as used herein can refer to one or more changes in a nucleic acid, such as a nucleic acid within the genome of an organism. The term "engineered" can refer to changes, additions, and/or deletions of genes. Engineered cells can also refer to cells with added, deleted and/or changed genes.

As used herein, the term "cell" or "engineered cell" and other grammatical forms thereof can refer to cells of human or non-human animal origin. Engineered cells can also refer to cells expressing CAR

The term "transfection" as used herein refers to the introduction of an exogenous nucleic acid into eukaryotic cells. Transfection can be achieved by various means known in the art, including calcium phosphate-DNA co-precipitation, DEAE-dextran-mediated transfection, polybrene-mediated transfection, electroporation, microinjection, liposome fusion, lipofection, protoplast fusion, retroviral infection and biolistics.

The term "stable transfection" or "stably transfect" refers to the introduction and integration of a exogenous nucleic acid, DNA or RNA into the genome of the transfected cell. The term "stable transfectant" refers to a cell having a foreign DNA stably integrated into its genomic DNA.

As used herein, terms "nucleic acid molecule encoding", "encoding DNA sequence" and "encoding DNA" refer to a sequence or order of deoxyribonucleotides along a deoxyribonucleic acid chain. The order of these deoxyribonucleotides determines the order of amino acids along a polypeptide (protein) chain. Therefore, the nucleic acid sequence encodes an amino acid sequence.

The term "individual" as used herein refers to any animal, such as a mammal or a marsupial. The individual of the present invention includes, but not limited to, a human, non-human primate (such as rhesus monkey or other types of macaque), mouse, pig, horse, donkey, cattle, sheep, rat, and any kind of poultry.

The term "peripheral blood lymphocytes" (PBL) as used herein, and other grammatical forms thereof can refer to lymphocytes circulating in blood (e.g., peripheral blood). Peripheral blood lymphocytes may refer to lymphocytes that are not limited to organs. The peripheral blood lymphocytes may comprise T cells, NK cells, B cells, or any combination thereof.

The term "immune effector cell" or "immune responsive cell" as used herein may refer to cells that can trigger an immune response, including but not limited to T cells, B cells, NK cells, NKT cells, DNT cells, etc., their respective precursor cells and descendants. Immune effector cells can also refer to cells of the lymphoid or bone marrow lineage.

The term "T cell" and other grammatical forms thereof as used herein can refer to T cells of any origin. For example, the T cell may be a primary T cell, such as an autologous T cell or the like. T cells can also be of human or non-human.

As used herein, the term "T cell activation" or "T cell stimulation" and other grammatical forms thereof may refer to T cells that are sufficiently stimulated to induce detectable cell proliferation, production of cytokine, and/or detectable effector function status. In some cases, "complete T cell activation" can be similar to triggering of T cell cytotoxicity. T cell activation can be measured by using various assays known in the art. The assay may be ELISA, ELISPOT to measure cytokine secretion, flow cytometry assay (CD107) to measure intracellular cytokine expression, flow cytometry assay to measure proliferation, and cytotoxicity assay (51Cr release assay) to determine target cell elimination. In the assay, a control (non-engineered cell) is usually used to be compared with an engineered cell (CAR T) to determine the relative activation of the engineered cell relative to the control. In addition, the assay can be compared with engineered cells incubated or contacted with target cells that do not express the target antigen. For example, the comparison may be a comparison with GPC3-CART cells incubated with target cells that do not express GPC3.

When used to refer to a nucleotide sequence, the term "sequence" and other grammatical forms thereof as used herein may include DNA or RNA, and may be single-stranded or double-stranded. The nucleic acid sequence can be mutated. The nucleic acid sequence can be of any length.

The term "effective amount" as used herein refers to an amount providing therapeutic or preventive benefits.

The term "expression vector" as used herein refers to a vector containing a recombinant polynucleotide, which contains an expression control sequence operatively linked to the nucleotide sequence to be expressed. The expression vector contains sufficient cis-acting elements for expression; other elements for expression can be provided by host cells or *in vitro* expression systems. Expression vectors include those known in the art, such as cosmids, plasmids (e.g. naked or contained in liposomes), and viruses (e.g., lentivirus, retrovirus, adenovirus, and adeno-associated virus).

The term "lentivirus" as used herein refers to the genus of the Retroviridae family. Retroviruses are unique among retroviruses in their ability to infect non-dividing cells; they can deliver a large amount of genetic information into the DNA of host cells, thereof they are one of the most effective vehicles for gene delivery. HIV, SIV and FIV are examples of lentiviruses. Vectors derived from lentiviruses provide a means to achieve significant gene transfer *in vivo.*

As used herein, the term "operably linked" refers to a functional linkage between a regulatory sequence and other nucleic acid sequences, and the linkage, for example results in the expression of the latter. For example, when the first nucleic acid sequence and the second nucleic acid sequence are in a functional relationship, the first nucleic acid sequence and the second nucleic acid sequence are operably linked.

The term "promoter" as used herein is defined as a DNA sequence recognized by a synthetic mechanism of the cell or an introduced synthetic mechanism required to initiate the specific transcription of a polynucleotide sequence.

The term "vector" as used herein is a composition that contains an isolated nucleic acid and can be used to deliver the isolated nucleic acid inside a cell. Many vectors are known in the art, including but not limited to linear polynucleotides, polynucleotides related to ionic or amphiphilic compounds, plasmids, and viruses. Therefore, the term "vector" includes autonomously replicating plasmids or viruses. The term should also be interpreted to include non-plasmid and non-viral compounds facilitating the transfer of nucleic acids into cells, such as polylysine compounds, liposomes, and the like. Examples of viral vectors include, but are not limited to, adenovirus vectors, adeno-associated virus vectors, retroviral vectors, and the like.

As used herein, the term sequence "identity" determines the percent identity by comparing two best-matched sequences over a comparison window (for example, at least 20 positions), where the portion of the polynucleotide or polypeptide sequence in the comparison window may include additions or deletions (i.e. gaps), such as 20% or less gaps (e.g., 5 to 15%, or 10 to 12%) for the two sequences that best match, compared with the reference sequence (which does not contain additions or deletions). The percentage is usually calculated by determining the number of positions where the same nucleic acid base or amino acid residue occurs in the two sequences to produce the number of correct matching positions. The number of correct matching positions is divided by the total number of positions in the reference sequence (i.e., the window size), and multiply the result by 100 to produce the percentage of sequence identity.

The terms "IL12", "interleukin-12" used herein can be used interchangeably and have the same meaning. The term "IL12" as used herein is an immunomodulatory factor with multiple biological activities. For example, the term "IL12" can refer to human IL12 as defined in SEQ ID NO: 26 or active fragments thereof, or can refer to murine IL12 as defined in SEQ ID NO: 27 or active fragments thereof, or may also be of other species.

In some embodiments, the element used to construct the receptor specifically binding to GPC3 or IL12 may be naturally occurring, for example, it may be isolated or purified from mammals; or it may also be artificially prepared, for example, recombinant elements or IL12 can be produced according to conventional genetic engineering recombination techniques. Preferably, recombinant elements or IL12 can be used in the present invention.

Based on the aforementioned elements or IL12 polypeptide sequences, amino acid sequences formed by substitution, deletion or addition of one or more amino acid residues are also included in the present invention. Appropriate replacement of amino acids is a well-known technique in the art, which can be readily implemented and ensures that biological activities of the resulting molecule will not be altered. Based on such techniques, a skilled person in the art will realize that generally, changing a single amino acid in a non-essential region of a polypeptide does not substantially change the biological activity.

Biologically active fragments of all the elements or IL12 polypeptides can be used in the present invention. The biologically active fragment mentioned herein means a polypeptide that is a part of the full-length polypeptide, while still retains all or part of the functions of the full-length polypeptide. Normally, the biologically active fragment retains at least 50% of the activity of the full-length polypeptide. Under preferred conditions, the active fragment can retain 60%, 70%, 80%, 90%, 95%, 99%, or 100% of the activity of the full-length polypeptide.

Based on the various elements or IL12 polypeptide sequences, modified or improved polypeptides can also be used in the present invention. For example, a polypeptide can be modified or improved to promote half-life, effectiveness, metabolism, and/or polypeptide effectiveness thereof. In other words, any variation that does not affect the biological activity of a polypeptide can be used in the present invention.

The term "tumor" as used herein refers to a disease characterized by the pathological proliferation of cells or tissues, and subsequent migration or invasion into other tissues or organs. Tumor growth is usually uncontrolled and progressive, and does not induce or inhibit normal cell proliferation. Tumors can affect a variety of cells, tissues or organs, including but not limited to bladder, breast, esophagus, intestine, kidney, liver, lung, lymph nodes, nerve tissue, ovary, pancreas, prostate, skeletal muscle, skin, spinal cord, spleen, stomach, uterine organs, or tissues or corresponding cells. The tumor of the present invention may include, but is not limited to, liver cancer, stomach cancer, lung cancer, breast cancer, head and neck cancer, bladder cancer, ovarian cancer, cervical cancer, kidney cancer, pancreatic cancer, cervical cancer, liposarcoma, melanoma, and adrenal carcinoma, schwannoma, malignant fibrous histiocytoma, esophageal cancer. Preferably, the "tumor" includes, but is not limited to: liver cancer, gastric cancer, lung cancer, and breast cancer.

The term "enhancement of immune effector cell function" as used herein includes, for example, enhancement of T cell function. Taking T cells as an example, enhancing T cell functions includes inducing, causing or stimulating T cells to have sustained or enhanced biological functions, or renewing or reactivating exhausted or inactive T cells. Examples of enhancing T cell function include: compared with the pre-intervention level, increased interferon secretion by CD8+ T cells, increased proliferation, and increased antigen reactivity (e.g., virus or pathogen clearance rate). In one embodiment, the enhancement is at least 50%, or 60%, 70%, 80%, 90%, 100%, 120%, 150%, 200%. The way to measure such enhancement is known to a skill person in the art.

The term "exogenous" as used herein refers to a nucleic acid molecule or polypeptide not endogenously expressed in a cell, or the expression level of which is insufficient to achieve the function that it exhibits when it is overexpressed. Thus, "exogenous" includes recombinant nucleic acid molecules or polypeptides expressed in cells, such as exogenous, heterologous and overexpressed nucleic acid molecules and polypeptides.

The term "receptor" as used herein refers to a polypeptide, or part thereof, selectively binding to one or more ligands on the cell membrane.

In some embodiments, the antigen-binding receptor of the present invention is a chimeric antigen receptor. The term "Chimeric Antigen Receptor (CAR)" as used herein refers to a tumor antigen binding domain fused to an intracellular signaling domain that can activate T cells. The extracellular binding domain of CAR is generally derived from mouse or humanized or human monoclonal antibodies.

Chimeric antigen receptors usually contain extracellular antigen binding regions. In some embodiments, the extracellular antigen binding region may be of full human. In other cases, the extracellular antigen binding region can be humanized. In other cases, the extracellular antigen binding region may be of murine origin, or the chimera in the extracellular antigen binding region consists of amino acid sequences from at least two different animals. In some embodiments, the extracellular antigen binding region may be of non-human.

A variety of antigen binding regions can be designed. Non-limiting examples include single chain variable fragments (scFv) derived from antibodies, antigen binding regions (Fab) selected from libraries, single domain fragments, or natural ligands that engage cognate receptors thereof. In some embodiments, the extracellular antigen binding region may comprise scFv, Fab, or natural ligands, and any derivatives thereof. The extracellular antigen binding region may refer to a molecule other than the intact antibody, which may comprise a part of the intact antibody and can bind to the antigen to which the intact antibody binds. Examples of antibody fragments may include, but are not limited to, Fv, Fab, Fab', Fab'-SH, F(ab')2; bifunctional antibodies, linear antibodies; single-chain antibody molecules (such as scFv); and multispecific antibodies formed from antibody fragments.

Extracellular antigen binding regions, such as scFv, Fab, or natural ligands, can be part of a CAR that determines antigen specificity. The extracellular antigen binding region can bind to any complementary target. The extracellular antigen binding region can be derived from antibodies with known variable region sequences. The extracellular antigen binding region can be obtained from available antibody sequences from mouse hybridomas. Alternatively, the extracellular antigen binding region can be obtained from total extracellular cleavage sequencing of tumor cells or primary cells, such as tumor infiltrating lymphocytes (TIL).

In some cases, the binding specificity of the extracellular antigen binding region can be determined by complementarity determining regions or CDRs, such as light chain CDRs or heavy chain CDRs. In many cases, the binding specificity can be determined by the light chain CDR and the heavy chain CDR. Compared with other reference antigens, the combination of a given heavy chain CDRs and light chain CDRs can provide a given binding pocket, which can confer greater affinity and/or specificity to the antigen (e.g., GPC3). For example, CDRs specific to Glypican-3 can be expressed in the extracellular binding region of the CAR, so that the GPC3-targeted CAR can target immune effector cells to tumor cells expressing GPC3.

In certain aspects of any embodiments disclosed herein, the extracellular antigen binding region, such as a scFv, may comprise a light chain CDR specific for the antigen. The light chain CDR may be the complementarity determining region of the scFv light chain of an antigen binding unit, such as a CAR. The light chain CDR may comprise a sequence of consecutive amino acid residues, or a sequence of two or more consecutive amino acid residues separated by a non-complementarity determining region (e.g., a framework region). In some cases, light chain CDR may include two or more light chain CDRs, which may be referred to as light chain CDR-1, CDR-2, and the like. In some cases, the light chain CDR may comprise three light chain CDRs, which may be referred to as light chain CDR-1, light chain CDR-2, and light chain CDR-3, respectively. In some examples, a group of CDRs present on a common light chain can be collectively referred to as light chain CDRs.

In certain aspects of any embodiments disclosed herein, the extracellular antigen binding region, such as a scFv, may comprise a heavy chain CDR specific for an antigen. The heavy chain CDR may be the heavy chain complementarity determining region of an antigen binding unit, such as a scFv. The heavy chain CDR may comprise a continuous sequence of amino acid residues, or a continuous sequence of two or more amino acid residues separated by a non-complementarity determining region (such as a framework region). In some cases, a heavy chain CDR may comprise two or more heavy chain CDRs, which may be referred to as heavy chain CDR-1, CDR-2, and the like. In some cases, the heavy chain CDR may include three heavy chain CDRs, which may be referred to as heavy chain CDR-1, heavy chain CDR-2, and heavy chain CDR-3, respectively. In some cases, a group of CDRs present on a common heavy chain can be collectively referred to as a heavy chain CDR.

By using genetic engineering, the extracellular antigen binding region can be modified in various ways. In some cases, the extracellular antigen binding region can be mutated, so that the extracellular antigen binding region can be selected to have a higher affinity for its target. In some cases, the affinity of the extracellular antigen binding region for its target can be optimized for targets that can be expressed at low levels on normal tissues. Such optimization can be performed to minimize potential toxicity. In other cases, clones of extracellular antigen-binding regions with higher affinity for the membrane-bound form of the target may be superior to their soluble form counterparts. Such modification can be performed since different levels of targets in a soluble form can still be detected, and targeting thereof can cause undesirable toxicity.

In some cases, the extracellular antigen binding region includes a hinge or spacer. The terms hinge and spacer can be used interchangeably. The hinge can be considered as part of the CAR used to render flexibility to the extracellular antigen binding region. In some cases, hinges can be used to detect CARs on the cell surface, especially when antibodies that detect extracellular antigen binding regions are ineffective or unavailable. For example, it may be necessary to optimize the length of the hinge derived from immunoglobulin, depending on the location of the epitope on the target targeted by the extracellular antigen binding region.

In some cases, the hinge may not belong to immunoglobulin, but belong to another molecule, such as the natural hinge of CD8$\alpha$ molecule. The CD8$\alpha$ hinge may contain cysteine and proline residues that are known to play a role in the interaction of CD8 co-receptors and MHC molecules. The cysteine and proline residues can affect the performance of the CAR.

The CAR hinge can be adjustable in size. The morphology of the immune synapse between the immune effector cell and the target cell also defines the distance that cannot be functionally bridged by CAR due to the distal membrane epitopes of the target molecule on the cell surface, therefore, the distance between synapses cannot reach the approximate value of signal transmission even with the short hinge CAR. Similarly, for the CAR targeting epitope at the proximal end of the membrane, signal output can be observed only in the context of the long hinge CAR. The hinge can be adjusted according to the used extracellular antigen binding region. The hinge can be of any length.

The transmembrane domain can anchor the CAR to the plasma membrane of the cell. The natural transmembrane portion of CD28 can be used in a CAR. In other cases, the natural transmembrane portion of CD8$\alpha$ can also be used in a CAR. "CD8" can be a protein that has at least 85, 90, 95, 96, 97, 98, 99, or 100% identity with NCBI reference number: NP_001759 or a fragment having stimulating activity. "CD8 nucleic acid molecule" can be a polynucleotide encoding a CD8 polypeptide. In some cases, the transmembrane region can be the natural transmembrane portion of CD28. "CD28" can refer to a protein having at least 85, 90, 95, 96, 97, 98, 99, or 100% identity with the reference number of NCBI: NP_006130 or a fragment thereof with stimulating activity. A "CD28 nucleic acid molecule" may be a polynucleotide encoding a CD28 polypeptide. In some cases, the transmembrane portion may comprise a CD8$\alpha$ region.

The intracellular signal domain of the CAR may be responsible for activating at least one of the effector functions of the immune effector cells in which the CAR has been placed. CAR can induce effector functions of T cells, for example, the effector function is cytolytic activity or auxiliary activity, including secretion of cytokines. Therefore, the term "intracellular signal domain" refers to the part of a protein that transduces effector function signals and guides cells to perform specific functions. Although the entire intracellular signaling region can usually be used, in many cases it is not necessary to use the entire chain of signaling domains. In some cases, truncated portions of intracellular signaling regions are used. In some cases, the term intracellular signaling domain is therefore intended to include any truncated portion of the intracellular signaling region which is sufficient to transduce effector function signals.

Preferred examples of signal domains used in CAR may include cytoplasmic sequences of T cell receptor (TCR) and co-receptors synergistically acting to initiate signal transduction after target-receptor binding, as well as any derivatives or variant sequence thereof and any synthetic sequence with the same functionality of these sequences.

In some cases, the intracellular signaling domain may contain a known immunoreceptor tyrosine activation motif (ITAM) signaling motif. Examples of ITAMs containing cytoplasmic signaling sequences include functional signaling domains derived from proteins of TCRζ, FcRy, FcRβ, CD3γ, CD3δ, CD3ε, CD5, CD22, CD79a, CD79b, CD66d DAP10, or DAP12. However, in a preferred embodiment, the intracellular signaling domain is derived from CD3ζ chain.

An example of a T cell signaling domain containing one or more ITAM motifs is CD3ζ domain, also known as T cell receptor T3ζ chain or CD247. This domain is a part of the T cell receptor-CD3 complex, and plays an important role in combining the antigen recognition of several intracellular signal transduction pathways with the main effect activation of T cells. As used herein, CD3ζ mainly refers to human CD3ζ and isoforms thereof, as known from Swissprot entry P20963, including proteins with substantially the same sequence. As a part of the chimeric antigen receptor, it shall be noticed once again that the whole T cell receptor T3ζ chain is not required, and any derivative containing the signaling domain of the T cell receptor T3ζ chain may be suitable, including any functional equivalents thereof.

The intracellular signaling domain can be selected from any one of the domains in Table 1. In some cases, the domain can be modified so that the identity with the reference domain can be about 50% to about 100%. Any one of the domains in Table 1 can be modified so that the modified form can comprise about 50, 60, 70, 80, 90, 95, 96, 97, 98, 99 or up to about 100% identity.

The intracellular signaling region of the CAR may further include one or more co-stimulatory domains. The intracellular signaling region may contain a single co-stimulatory domain, such as the ζ chain (first-generation of CAR) or in combination with CD28 or 4-1BB (second-generation of CAR). In other examples, the intracellular signaling region may contain two co-stimulatory domains, such as CD28/OX40 or CD28/4-1BB (third generation).

Together with intracellular signaling domains such as CD8, these co-stimulatory domains can produce downstream activation of the kinase pathway, thereby supporting gene transcription and functional cellular responses. The co-stimulatory domain of a CAR can activate CD28 (phosphatidylinositol-4,5-bisphosphate 3-kinase) or 4-1BB/OX40 (TNF-receptor-related factor adaptor protein) pathways, as well as proximal signaling proteins related to MAPK and Akt activation.

In some cases, the signal generated by the CAR may be combined with auxiliary or co-stimulatory signals. For co-stimulatory signal domains, the chimeric antigen receptor-like complex can be designed to contain several possible co-stimulatory signal domains. As is well known in the art, in naive T cells, T cell receptor engagement alone is not sufficient to induce the complete activation of T cells into cytotoxic T cells. The activation of intact producer T cells requires a second co-stimulatory signal. Several receptors that provide co-stimulation to T cell activation have been reported, including but not limited to CD28, OX40, CD27, CD2, CD5, ICAM-1, LFA-1 (CD11a/CD18), 4-1BBL, MyD88 and 4- 1BB. All of the signaling pathways used by these co-stimulatory molecules can synergistically act with the master T cell receptor activation signal. The signals provided by these co-stimulatory signaling regions can synergistically act with main effect activation signals derived from one or more ITAM motifs (such as the CD3zeta signaling domain), and can complete the requirement of T cell activation.

In some cases, the addition of co-stimulatory domains to chimeric antigen receptor-like complexes can enhance the efficacy and durability of engineered cells. In another embodiment, the T cell signaling domain and the co-stimulatory domain are fused to each other to form a signaling region.

Table 1. Co-stimulatory domain

| Gene Marker | Abbreviation | Name |
|---|---|---|
| CD27 | CD27; T14; S152; Tp55; TNFRSF7; S152. LPFS2 | CD27 molecule |
| CD28 | Tp44; CD28; CD28 antigen | CD28 molecule |
| TNFRSF9 | ILA; 4-1BB; CD137; CDw137 | Tumor Necrosis Factor Receptor Superfamily Member 9 |
| TNFRSF 4 | OX40; ACT35; CD134; IMD16; TXGP1L | Tumor Necrosis Factor Receptor Superfamily Member 4 |
| TNFRSF8 | CD30; Ki-1; D1S166E | Tumor Necrosis Factor Receptor Superfamily Member 8 |

(continued)

| Gene Marker | Abbreviation | Name |
|---|---|---|
| CD40LG | IGM; IMD3; TRAP; gp39; CD154; CD40L; HIGM1; T-BAM; TNFSF5; hCD40L | CD40 ligand |
| ICOS | AILIM; CD278; CVID1 | Inducible T cell costimulator |
| ITGB2 | LAD; CD18; MF17; MFI7; LCAMB; LFA-1; MAC-1 | Integrin β2 (complement component 3 receptor 3 and 4 subunits) |
| CD2 | T11; SRBC; LFA-2 | CD2 molecule |
| CD7 | GP40; TP41; Tp40; LEU-9 | CD7 molecule |
| KLRC2 | NKG2C; CD159c; NKG2-C | Killer cell lectin-like receptor subfamily C, Member 2 |
| TNFRSF 18 | AITR; GITR; CD357; GITR-D | Tumor Necrosis Factor Receptor |
| | | Superfamily Member 18 |
| TNFRSF 14 | TR2; ATAR; HVEA; HVEM; CD270; LIGHTR | Tumor Necrosis Factor Receptor Superfamily Member 14 |
| HAVCR1 | TIM; KIM1; TIM1; CD365; HAVCR; KIM-1; TIM-1; TIMD1; TIMD-1; HAVCR-1 | Hepatitis A Virus Cell Receptor 1 |
| LGALS9 | HUAT; LGALS9A, Galectin-9 | Lectin, galactoside binding, soluble, 9 |
| CD83 | BL11; HB15 | CD83 molecule |

The term "modulation" as used herein refers to a positive or negative change. Examples of adjustments include changes of 1%, 2%, 10%, 25%, 50%, 75%, or 100%.

The term "treatment" as used herein refers to clinical intervention in the process of trying to change a person or treating a disease caused by cells, which can be used for prevention or intervention in the clinical pathological process. The therapeutic effects include, but not limited to, preventing the occurrence or recurrence of the disease, reducing the symptoms, reducing the direct or indirect pathological consequences of any disease, preventing metastasis, slowing down the progression of the disease, improving or relieving the condition, relieving or improving the prognosis, etc.

The term "immune-compromised" as used herein means that a subject has immunodeficiency and is easily infected. Organisms that cause opportunistic infections usually do not cause illness with a healthy immune system, but can infect people with a weakened or suppressed immune system.

The term "constitutive expression" as used herein refers to the expression under all physiological conditions.

The term "induced expression" as used herein refers to the expression under certain conditions, such as when T cells bind to an antigen. A person skilled in the art will knoe how to perform a conventional "induction of expression".

**Immune effector cells**

In some embodiments, the present invention provides an immune effector cell expressing exogenous IL12 and a receptor that specifically binds to GPC3.

In some embodiments, the exogenous IL12 is constitutively expressed or inducibly expressed; preferably inducibly expressed. For example, an inducible promoter; preferably NFAT6 promoter is used.

In some embodiments, the transmembrane region of the antigen-binding receptor may be selected from the transmembrane region of a protein such as CD8 or CD28. The human CD8 protein is a heterodimer, composed of two chains, αβ or γδ. In some embodiments, the transmembrane region is selected from the transmembrane region of CD8α or CD28. In addition, the CD8α hinge region (hinge) is a flexible region. Therefore, CD8 or CD28 and the transmembrane region as well as the hinge region are used to connect target recognition domain scFv of the antigen-binding receptor CAR and the intracellular signal region.

The intracellular signal domain of the present invention can be selected from CD3ζ, FcεRIγ, CD28 co-stimulatory signal domain, CD137 co-stimulatory signal domain, and combinations thereof. The CD3 molecule is composed of five subunits, in which the CD3ζ subunit (also known as CD3 zeta, abbreviated as Z) contains three ITAM motifs, which are important signaling region in the TCR-CD3 complex. In addition, as mentioned earlier, CD28 and CD137 are co-stimulatory signal molecules, and after binding to respective ligands, the co-stimulation effect produced by their intracellular signal segment can cause the continuous proliferation of immune effector cells (mainly T lymphocytes), increase the level of cytokines such as IL-2 and IFN-γ secreted by immune effector cells, and improve the survival and anti-tumor effects of CAR immune effector cells in the body. In some embodiments, the intracellular signaling domain is a CD3ζ signal domain or a combination of a CD3ζ signal domain and other co-stimulatory signals, such as CD28.

In some embodiments, the immune effector cell of the present invention may include an expression construct, in which there are elements connected sequentially in the following manner: antibody, CD28 co-stimulatory signal domain, CD3ζ, and NFAT6, IL12 expression unit reversely linked with the foregoing elements. Preferably, the antibody and the CD28 co-stimulatory signal domain are connected through the CD8α transmembrane region and the CD8α hinge region.

In some embodiments, the nuclear factor of activated T cells (NFAT) plays an important role in the transcriptional expression of cytokines during the activation of T cell. Therefore, the present inventors placed the IL12 encnoding sequence under the control of the NFAT6 promoter, so that only when CAR-T cells contact an antigen and trigger T cell activation, IL12 can be expressed at a high level.

The NFAT6 promoter is a promoter composed of 6 NFAT binding sites and the minimal promoter of IL2 linked in tandem (Hooijberg E, Bakker AQ, Ruizendaal JJ, Spits H. NFAT-controlled expression of GFP permits visualization and Isolation of antigen-stimulated primary human Tcells. Blood. 2000 Jul 15; 96(2):459-66), which can be used to regulate the expression of cytokines, such as IL12 in T lymphocytes, such as TCR-T (Zhang L, Kerkar SP , Yu Z, Zheng Z, Yang S, RestifoNP, Rosenberg SA, Morgan RA. Improving adoptive T cell therapy by targeting and controlling IL-12 expression to the tumor environment. Mol Ther.2011 Apr;19(4):751-9 ).

According to one aspect of the present invention, the present invention also includes a nucleic acid encoding the antigen-binding receptor. The present invention also relates to variants of the above-mentioned polynucleotides, which encode polypeptides having the same amino acid sequence as the present invention, or polypeptide fragments, analogs and derivatives.

The present invention also provides a vector containing the nucleic acid encoding the antigen-binding receptor protein expressed on the surface of immune effector cells. In a specific embodiment, the vector used in the present invention is a lentiviral plasmid vector pRRLSIN-cPPT.PGK-GFP.WPRE. It should be understood that other types of viral vectors and non-viral vectors are also applicable.

The present invention also includes a virus containing the above-mentioned vectors. The virus of the present invention includes a packaged virus with infectivity, and also includes a virus to be packaged that contains necessary components for packaging as a virus with infectivity. Other viruses known in the art that can be used to transfer a foreign gene into immune effector cells and corresponding plasmid vectors can also be used in the present invention.

The immune effector cell of the present invention is transduced with a construct capable of expressing an antigen-binding receptor and exogenous IL12, or an expression vector, or a virus containing the plasmid. Conventional nucleic acid transduction methods in the art, including non-viral and viral transduction methods, can be used in the present invention.

The immune effector cell of the present invention can also express another antigen-binding receptor besides the above-mentioned antigen-binding receptor.

The immune effector cell of the present invention can also express a safety switch; and preferably, the safety switch includes: iCaspase-9, Truncated EGFR or RQR8.

## Nucleic Acid

A transgene encoding a target binding antigen receptor or CAR can be incorporated into the cell. For example, the transgene can be incorporated into immune effector cells, such as T cells. When inserted into a cell, the transgene can be a complementary DNA (cDNA) fragment, which is a copy of messenger RNA (mRNA); or the gene itself (with or without introns) located in the original region of its genomic DNA.

Nucleic acids encoding transgenic sequences, such as DNA, can be randomly inserted into the chromosomes of cells. The random integration can be achieved by any method of introducing nucleic acid (e.g., DNA) into cells. For example, the method may include, but not limited to, electroporation, ultrasound, using gene guns, lipofection, calcium phosphate transfection, using dendrimers, microinjection, and using virus vectors including adenovirus, AAV and retroviral vectors, and/or type II ribozyme.

The DNA encoding the transgene can also be designed to include a reporter gene, so that the presence of the transgene or its expression product can be detected by the activation of the reporter gene. Any reporter gene can be used, such as those described above. By selecting cells in the cell culture in which the reporter gene has been activated, cells containing the transgene can be selected.

The expression of CAR can be verified by expression assays, such as qPCR or by measuring the level of RNA. The expression level can also indicate copy number. For example, if the expression level is very high, this can indicate that more than one copy of the CAR is integrated into the genome. Alternatively, the high expression can indicate that the transgene is integrated in a highly transcribed region, such as a region near a highly expressed promoter. The expression can also be verified by measuring protein levels, for example by Western blotting.

In some embodiments, the immune effector cells of the present invention may contain one or more transgenes. The one or more transgenes can express the CAR protein, and the CAR protein recognizes and binds to at least one epitope on the antigen or binds to a mutant epitope on the antigen. The CAR can be a functional CAR. In some embodiments, the immune effector cell of the present invention may comprise one or more CARs, or it may comprise a single CAR and a secondary engineered receptor.

As mentioned above, the transgene can be inserted into the genome of immune resposive cells in a random or site-specific manner. For example, the transgene can be inserted into a random site in the genome of an immune cell. These transgenes can be functional, for example, fully functional when inserted any site in the genome. For example, the transgene can encode its own promoter, or can be inserted into a position controlled by its internal promoter. Alternatively, the transgene can be inserted into a gene, such as an intron of a gene or an exon, a promoter, or a non-coding region of a gene. Transgenes can be inserted, so that genes, such as endogenous immune checkpoints, can be disrupted by the insertion.

In some cases, more than one copy of the transgene can be inserted into multiple random sites within the genome. For example, multiple copies can be inserted at random locations in the genome. This may result in an increase in overall expression compared with one random insertion of the transgene. Alternatively, a copy of the transgene can be inserted into the gene, and another copy of the transgene can be inserted into a different gene. The transgene can be targeted, so that it can be inserted into a specific site in the genome of immune responsive cells.

In some cases, a polynucleic acid containing a sequence encoding a receptor binding to an antigen may take the form of a plasmid vector. The plasmid vector may contain a promoter. In some cases, the promoter can be constitutive. In some cases, the promoter is inducible. The promoter can be or can be derived from CMV, U6, MND or EF1a. In some cases, the promoter may be adjacent to the CAR sequence. In some cases, the plasmid vector also contains a splice acceptor. In some cases, the splice acceptor may be adjacent to the CAR sequence. The promoter sequence can be a PKG or MND promoter. The MND promoter may be a synthetic promoter containing the U3 region of MoMuLV LTR modified with an enhancer of myeloproliferative sarcoma virus.

In some cases, polynucleotide encoding target receptors can be designed for delivery to cells by non-viral techniques. In some cases, the polynucleotide can be a compatible reagent of good manufacturing practice (GMP).

The expression of the polynucleotide encoding the target binding antigen receptor or CAR can be controlled by one or more promoters. Promoters can be ubiquitous, constitutive (unrestricted promoters that allow continuous transcription of related genes), tissue-specific promoters or inducible promoters. The expression of transgenes inserted adjacent or close to the promoter can be regulated. For example, the transgene can be inserted near or beside a ubiquitous promoter. Some ubiquitous promoters can be CAGGS promoter, hCMV promoter, PGK promoter, SV40 promoter or ROSA26 promoter.

The promoter can be endogenous or exogenous. For example, one or more transgenes can be inserted in the vicinity or proximity of the endogenous or exogenous ROSA26 promoter. In addition, the promoter may be specific for immune resposive cells. For example, one or more transgenes can be inserted in the vicinity or proximity of the porcine ROSA26 promoter.

Tissue-specific promoters or cell-specific promoters can be used to control the location of expression. For example, one or more transgenes can be inserted in the vicinity or proximity of a tissue-specific promoter. The tissue-specific promoter can be FABP promoter, Lck promoter, CamKII promoter, CD19 promoter, keratin promoter, albumin promoter, aP2 promoter, insulin promoter, MCK promoter, MyHC promoter, WAP Promoter, or Col2A promoter.

Inducible promoters can also be used. If necessary, these inducible promoters can be turned on and off by adding or removing inducers. It is expected that the inducible promoter may be, but not limited to, Lac, tac, trc, trp, araBAD, phoA, recA, proU, cst-1, tetA, cadA, nar, PL, cspA, T7, VHB, Mx and/or Trex.

**Immune cell inducible promoter**

As used herein, the term "inducible promoter" is a controlled promoter that does not express or under-expresses the gene operably linked to it before the expected condition is achieved, and when the expected condition is achieved, expresses or expresses at a high level the gene to which it is operably linked. For example, in some embodiments, the inducible promoter of the present application does not express or under-expresses the gene operably linked to it under normal or high oxygen content conditions in the cell, while expresses or expresses at a high level the gene to which it is operably linked under hypoxic conditions in the response to the decreased oxygen content in the cell. In some embodiments, the inducible promoter used herein includes hypoxia-inducible transcription factor-la (HIF-1$\alpha$). In some embodiments, the term "inducible promoter" as used herein refers to an "immune cell inducible promoter", which does not express or expresses at low level the gene to which it is operably linked before the immune effector cell contacts the antigen or when the immune effector cell is not activated, while only drives high-level expression of the gene to which it is operably linked only when the immune effector cells contact antigen or immune effector cells are activated or under conditions such as hypoxia. In some embodiments, the "immune cell inducible promoter" includes a NFAT (nuclear factor of activated T cell)-type promoter.

As used herein, "NFAT-type promoter" refers to a type of promoter that regulates the expression of a gene to which it is operably linked based on the NFAT binding activity.

NFAT is a collective name for a family of transcription factors that play an important role in the immune response. One or more members of the NFAT family are expressed in most cells of the immune system. NFAT is also involved in the development of the heart, skeletal muscle and nervous system.

The NFAT transcription factor family consists of five members, NFATc1, NFATc2, NFATc3, NFATc4 and NFAT5. NFATc1 to NFATc4 are regulated by calcium signals. The Calcium signal is essential for NFAT activation, since calmodulin (CaM) activates serine/threonine phosphatase calcineurin (CN). The activated CN rapidly dephosphorylates the serine-rich region (SRR) and SP repeats at the amino terminus of the NFAT protein, leading to conformational changes, exposing nuclear localization signals, and leading to the import of NFAT into the nucleus.

Based on the role of NFAT in the transcriptional expression of cytokines during the activation of T cells, it can be used to regulate the immune cell inducible promoter described herein, so that when immune effector cells are exposed to antigen and activated, the gene to which it is operably linked can be expressed or high-level expressed. In some embodiments, the "NFAT-type promoter" described herein may include more than one NFAT binding site. For example, the "NFAT-type promoter" may include 2, 3, 4, 5, 6, 7, 8, 9, 10 or more NFAT binding sites. In some embodiments, the "NFAT-type promoter" may be a promoter composed of a plurality of NFAT binding sites and a promoter, such as the IL2 minimal promoter linked in tandem. In some embodiments, the NFAT-type promoter described herein includes 6 NFAT binding sites, designated as (NFAT)6. For convenience, the (NFAT)6 is also referred to as NFAT6. In some embodiments, the NFAT6 also represents 6 repeated NFAT binding sites in the NFAT-type promoter.

In addition, the transgene sequence may also include transcription or translation regulatory sequences, such as promoters, enhancers, insulators, internal ribosome entry sites, sequences encoding 2A peptides and/or polyadenylation signals, although not required for expression.

In some cases, retroviral vectors (γ-retrovirus or lentiviral vectors) can be used to introduce transgenes into immune responsive cells. For example, the transgenes encoding a CAR or any antigen-binding receptors or variants or fragments thereof can be cloned into retroviral vectors, and can be drived bythe endogenous promoters, retroviral long terminal repeats, or promotes specific to the type of the target cell. Non-viral vectors can also be used. The non-viral vector delivery system may include DNA plasmids, naked nucleic acids, and nucleic acids complexed with delivery vehicles, such as liposomes or poloxamers.

Many virus-based systems have been developed for transfering genes into mammalian cells. For example, retroviruses provide a convenient platform for gene delivery systems. The selected gene can be inserted into a vector and packaged in a retroviral particle using techniques known in the art. Vectors derived from retroviruses, such as lentiviruses are suitable tools to achieve long-term gene transfer, since the long-term and stable integration of the transgene and the propagation thereof in daughter cells thereof can be allowed. Lentiviral vectors have additional advantages over vectors derived from retroviruses, such as murine leukemia virus, since they can transduce non-proliferating cells. They also have added advantages of low immunogenicity. The advantage of adenoviral vectors is that they do not fuse into the genome of target cells, thereby bypassing negative integration-related events.

Cells can be transfected with a transgene encoding the receptor that binds to the antigen. The concentration of the transgene can range from about 100 picograms to about 50 micrograms. In some cases, the amount of nucleic acid (e.g., ssDNA, dsDNA, or RNA) introduced into the cell can be changed to optimize transfection efficiency and/or cell viability. For example, 1 microgram of dsDNA can be added to each cell sample for electroporation. In some cases, the amount of nucleic acid (e.g., double-stranded DNA) required for optimal transfection efficiency and/or cell viability varies according to cell type. In some cases, the amount of nucleic acid (e.g., dsDNA) used for each sample can directly correspond to transfection efficiency and/or cell viability. For example, a series of transfection concentrations. The transgene encoded by the vector can be integrated into the genome of the cell. In some cases, the transgene encoded by the vector is forward integrated. In other cases, the transgene encoded by the vector is reversely integrated.

In some cases, the immune responsive cells may be stem memory TSCM cells composed of CD45RO(-), CCR7(+), CD45RA(+), CD62L+(L-selectin), CD27+, CD28+ and/or IL-7Rα+, and the stem memory cells can also express CD95, IL-2Rβ, CXCR3 and/or LFA-1, and show many functional properties different from the stem memory cells. Alternatively, the immune responsive cell may also be a central memory TCM cell containing L-selectin and CCR7, where the central memory cell can secrete, for example, IL-2, but not IFNγ or IL-4. The immune responsive cells can also be effector memory TEM cells containing L-selectin or CCR7, and produce, for example, effector cytokines, such as IFNγ and IL-4.

The vector is usually dilivered into an individual by systemic administration (e.g., intravenous, intraperitoneal, intramuscular, subcutaneous, or intracranial infusion) or topical application, as described below. Alternatively, the vector can be delivered to cells *ex vivo*. For example, cells are removed from an individual patient (e.g., lymphocytes, T cells, bone marrow aspirate, tissue biopsy), and then the cells are usually selected, have the vector incorporated and implanted into the patient's body. The cells can be expanded before or after the selection.

Suitable immune responsive cells for expressing receptors that bind to an antigen may be cells that are autologous or non-autologous to the individual in need thereof.

A suitable source of immune effector cells can be obtained from an individual. In some cases, T cells can be obtained. The T cells can be obtained from many sources, including PBMC, bone marrow, lymph node tissue, umbilical cord blood, thymus tissue, and tissue from infection sites, ascites, pleural effusion, spleen tissue, and tumors. In some cases, any techniques known to a skilled person in the art, such as FicollTM isolation, can be used to obtain T cells from blood collected from the individual. In one embodiment, cells from the circulating blood of the individual are obtained by Apheresis. Apheresis products usually contain lymphocytes, including T cells, monocytes, granulocytes, B cells, other nucleated white blood cells, red blood cells and platelets. In one embodiment, the cells collected by apheresis can be washed to remove the plasma fraction and placed in a suitable buffer or medium for subsequent processing steps.

Alternatively, cells can be derived from healthy donors, from patients diagnosed with cancer, or patients diagnosed with infection. In some embodiments, the cell may be a part of a mixed cell population with different phenotypic characteristics. Cell lines can also be obtained from transformed T cells according to the aforementioned method. Cells can also be obtained from cell therapy banks. Modified cells resistant to immunosuppressive therapy can be obtained by any of the methods described herein. It is also possible to select a suitable cell population before modification. Engineered cell populations can also be selected after modification. Engineered cells can be used for autologous transplantation. Alternatively, the cells can be used for allogeneic transplantation. In some cases, the cells are administered to a patient, whose sample is used to identify cancer-related target sequences. In other cases, the cells are administered to a patient other than those whose samples are used to identify cancer-related target sequences.

In some cases, suitable primary cells include peripheral blood mononuclear cells (PBMC), peripheral blood lymphocytes (PBL) and other blood cell subpopulations, such as but not limited to T cells, natural killer cells, monocytes, natural Killer T cells, monocyte precursor cells, hematopoietic stem cells or non-pluripotent stem cells. In some cases, the cells can be any immune cells, including any T cells, such as tumor infiltrating cells (TIL), such as CD3 + T cells, CD4 + T cells, CD8 + T cells, or T cells of any other type. T cells may also include memory T cells, memory stem T cells, or effector T cells. It is also possible to select T cells from a large population, for example from whole blood. T cells can also be expanded from large populations. T cells may also be of a specific population and phenotype. For example, T cells can be of a phenotype including CD45RO(-), CCR7(+), CD45RA(+), CD62L(+), CD27(+), CD28(+), and/or IL-7R$\alpha$(+). Suitable cells can comprise one or more markers selected from the following list: CD45RO(-), CCR7(+), CD45RA(+), CD62L(+), CD27(+), CD28(+) and/or IL-7R$\alpha$(+). Suitable cells also include stem cells, such as, embryonic stem cells, induced pluripotent stem cells, hematopoietic stem cells, neuronal stem cells, and mesenchymal stem cells. Suitable cells may include any number of primary cells, such as human cells, non-human cells, and/or mouse cells. Suitable cells may be progenitor cells. Suitable cells can be derived from a subject (e.g., patient) to be treated.

The amount of therapeutically effective cells required in a patient can vary depending on the viability of the cells and the efficiency with which the cells are genetically modified (for example, the efficiency with which the transgene is integrated into one or more cells, or the expression level of the protein encoded by the transgene ). In some cases, the product (e.g., multiplication) of cell viability after genetic modification and the integration efficiency of transgene may correspond to the therapeutic amount of cells available for administration to a subject. In some cases, the increase in cell viability after genetic modification may correspond to a decrease in the necessary amount of cells that is effective for the patient when the treatment is administered. In some cases, an increase in the efficiency of integration of the transgene into one or more cells may correspond to a decrease in the necessary therapeutically effective number of cells administered in a patient. In some cases, the determination of the necessary therapeutically effective amount of cells can include the determination of functions related to changes in the cells over time. In some cases, the determination of the necessary therapeutically effective amount of cells may include the determination of functions corresponding to changes in the efficiency of integrating the transgene into one or more cells based on time-related varibles (e.g., culture time for cells, electroporation time, stimulation time for cells). In some cases, a therapeutically effective cell may be a cell population that contains about 30% to about 100% expression of antigen-binding receptors on the cell surface. In some cases, as measured by flow cytometry, therapeutically effective cells can express the antigen-binding receptor on the cell surface by about 30%, 35%, 40%, 45%, 50%, 55%, 60% , 65%, 70%, 75%, 80%, 85%, 90%, 91%, 92%, 93%, 94%, 95%, 96%, 97%, 98%, 99%, 99.5%, 99.9% or more than about 99.9%.

In some cases, when the antigen-binding receptor is present on the plasma membrane of a cell, and when it is activated by binding to a target, the toxicity to target cells with the target expressed in the cell surface will be caused. For example, in some cases, the cell may be a cytotoxic cell (e.g., NK cell or cytotoxic T lymphocyte). When the antigen-binding receptor as described herein is activated by binding to its target, it can increase the cytotoxic activity of cytotoxic cells to target cells. For example, in some cases, when the antigen-binding receptor described herein is activated by binding to its target, it can increase cytotoxicity by at least 10%, at least 15%, at least 20%, at least 25%, at least 30%, at least 40%, at least 50%, at least 75%, at least 2 times, at least 2.5 times, at least 5 times, at least 10 times or more 10 times, compared with the cytotoxicity to cells without the target.

## Pharmaceutical composition

The immune effector cells of the present invention can be used to prepare a pharmaceutical composition. In addition to including an effective amount of immune effector cells, the pharmaceutical composition may also include a pharmaceutically acceptable carrier. The term "pharmaceutically acceptable" means that when the molecular entity and composition are properly administered to animals or humans, they will not produce adverse, allergic or other undesired reactions.

Specific examples of some substances that can be used as pharmaceutically acceptable carriers or components thereof are antioxidants; preservatives; pyrogen-free water; isotonic salt solutions; and phosphate buffers and the like.

The composition of the present invention can be prepared into various dosage forms according to needs, and a doctor can determine the beneficial dosage for a patient according to factors, such as the type of the patient, age, weight, general disease condition, and administration method. The mode of administration can be, for example, parenteral administration (such as injection) or other treatment modes.

"Parenteral" administration of an immunogenic composition includes, for example, subcutaneous (s.c.), intravenous (i.v.), intramuscular (i.m.) or intrasternal injection or infusion techniques.

The formulation containing a population of immune responsive cells administered to an individual contains a plurality of immune responsive cells effective in treating and/or preventing a specific indication or disease. Therefore, a therapeutically effective population of immune responsive cells can be administered to an individual. Generally, a preparation containing about $1\times10^4$ to about $1\times10^{10}$ immune responsive cells is administered. In most cases, the formulation will contain about $1\times10^5$ to about $1\times10^9$ immune responsive cells, about $5\times10^5$ to about $5\times10^8$ immune responsive cells, or about $1\times10^6$ to about $1\times10^7$ immune responsive cells. However, depending on the location, source, identity, degree and severity of the cancer, the age and physical condition of an individual to be treated, and the like, the number of CAR immune responsive cells administered to the individual will vary within a wide range. The doctor will finally determine the appropriate dosage to be used.

In some embodiments, chimeric antigen receptors are used to stimulate immune cell-mediated immune responses. For example, a T cell-mediated immune response is an immune response involving T cell activation. Activated antigen-specific cytotoxic T cells can induce apoptosis in target cells displaying foreign antigen epitopes on the surface, such as cancer cells displaying tumor antigens. In another embodiment, chimeric antigen receptors are used to provide anti-tumor immunities in mammals. A subject will develop anti-tumor immunities, due to the T cell-mediated immune response.

In some cases, the method for treating a subject with cancer may involve administering one or more immune effector cells of the present invention to the subject in need of treatment. The immune effector cells can bind tumor target molecules and induce the death of cancer cells. As described above, the present invention also provides a method for treating pathogen infection in an individual, which comprises administering to the individual a therapeutically effective amount of the immune effector cells of the present invention.

The frequency of administration of the immune responsive cells of the present invention will be based on factors, including the disease to be treated, the elements of the specific immune responsive cells, and the mode of administration. As described herein, the immune effector cells of the present application have improved viability, therefore they can not only be administered in a lower therapeutically effective amount as compared with similar immune effector cells that do not express exogenous IL12, but also can be administered in a lower frequency of administration to obtain at least similar, and preferably more significant therapeutic effects.

## Combination with anti-tumor drugs

In some embodiments, the immune effector cells of the present invention may be administered in combination with another therapeutic agent. In some embodiments, the other therapeutic agent is a chemotherapeutic agent. The chemotherapeutic drugs that can be used in combination with the immune effector cells of the present invention include but are not limited to mitotic inhibitors (vinca alkaloids), including vincristine, vinblastine, vindesine and novibine (TM) (vinorelbine) , 5'-dehydrogen sulfide); topoisomerase I inhibitors, such as camptothecin compounds, including CamptosarTM (irinotecan HCL), HycamtinTM (topotecan HCL) and other compounds derived from camptothecin and analoges thereof; podophyllotoxin derivatives, such as etoposide, teniposide and midoxizoz; alkylating agents, cisplatin, cyclophosphamide, nitrogen mustard, trimethylene thiophosphoramide, carmustine, busulfan, chlorambucil, briquiazine, uracil mustard, cloprofen, and dacarbazine; antimetabolites, including cytarabine, fluorouracil, methotrexate, mercaptopurine, azathioprine, and procarbazine; antibiotics, including but not limited to doxorubicin, bleomycin, dactinomycin, daunorubicin, mycinomycin, mitomycin, sarcomycin C, and doxorubicin; and other chemotherapy drugs, including but not limited to anti-tumor antibodies, dacarbazine, azacytidine, amsacam, melphalan, ifosfamide and mitoxantrone.

In some embodiments, chemotherapeutic drugs that can be used in combination with the immune effector cells of the present invention include, but are not limited to, anti-angiogenic agents, including anti-VEGF antibodies (including humanized and chimeric antibodies, anti-VEGF aptamers and antisense oligonucleotides) and other angiogenesis inhibitors, such as angiostatin, endostatin, interferon, retinoic acid, and tissue inhibitors of metalloproteinase-1 and -2.

Kit

The present invention also provides a kit containing the immune effector cells of the present invention. The kit can be used to treat or prevent cancer, pathogen infection, immune disorder, or allogeneic transplantation. In one embodiment, the kit may include a therapeutic or preventive composition containing an effective amount of immune effector cells of one or more unit dosage forms.

In some embodiments, the kit includes a sterile container that can contain a therapeutic or preventive composition.

In some cases, the kit may include about $1\times10^4$ cells to about $1\times10^6$ cells. In some cases, the kit may include at least about $1\times10^5$ cells, at least about $1\times10^6$ cells, at least about $1\times10^7$ cells, at least about $4\times10^7$ cells, at least about $5\times10^7$ cells, at least about $6\times10^7$ cells, at least about $6\times10^7$ cells, $8\times10^7$ cells, at least about $9\times10^7$ cells, at least about $1\times10^8$ cells, at least about $2\times10^8$ cells, at least about $3\times10^8$ cells, at least about $4\times10^8$ cells, at least about $5\times10^8$ cells, at least about $6\times10^8$ cells, at least about $6\times10^8$ cells, at least about $8\times10^8$ cells, at least about $9\times10^8$ cells, at least about $1\times10^9$ cells, at least about $2\times10^9$ cells, at least about $3\times10^9$ cells, at least about $4\times10^9$ cells, at least about $5\times10^9$ cells, at least about $6\times10^9$ cells, at least about $8\times10^9$ cells, at least about $9\times10^9$ cells, at least about $1\times10^{10}$ cells, at least about $2\times10^{10}$ cells, at least about $3\times10^{10}$ cells, at least about $4\times10^{10}$ cells, at least about $5\times10^{10}$ cells, at least about $6\times10^{10}$ cells, at least about $9\times10^{10}$ cells, at least about $9\times10^{10}$ cells, at least about $1\times10^{11}$ cells, at least about $2\times10^{11}$ cells, at least about $3\times10^{11}$ cells, at least about $4\times10^{11}$ cells, at least about $5\times10^{11}$ cells, at least about $8\times10^{11}$ cells, at least about $9\times10^{11}$ cells, or at least about $1\times10^{12}$ cells. For example, about $5\times10^{10}$ cells can be included in the kit.

In some cases, the kit may include allogeneic cells. In some cases, the kit can include cells with genomic modifications. In some cases, the kit may contain "ready-for-use" cells. In some cases, the kit can include cells that can be expanded for clinical use. In some cases, the kit may contain contents for research purposes.

Autologous lymphocyte infusion can be used for treatment. Autologous peripheral blood mononuclear cells (PBMC) can be collected from a patient in need of treatment, and T cells can be activated and expanded using methods described herein and known in the art, and then injected into the patient. In other cases, allogeneic cells can be used to treat patients.

The methods disclosed herein can include transplantation. Transplantation can refer to the adoptive transplantation of cell products. The transplantation can be autologous transplantation, allogeneic transplantation, xenotransplantation or any other transplantation. For example, the transplantation can be a xenotransplantation. The transplantation can also be an allogeneic transplantation.

**Example 1. Preparation of GPC3-CAR-T cells/IL12-GPC3-CAR T cells**

1. Construction of plasmid

The chimeric antigen receptor used in this example is a second-generation of chimeric antigen receptor, and the encoding nucleotide sequence of the scFv targeting the extracellular domain of the GPC3 receptor is shown in SEQ ID NO:1, which also comprises the transmembrane domain of CD28, the intracellular domain of CD28, and CD3ζ. Plasmids for GPC3-CAR-T cells and IL12-GPC3-CAR T cells were constructed, respectively (referring to Figure 1A), as follows:
The GPC3-CAR-T sequence consists of CD8α signal peptide (SEQ ID NO: 2), scFv targeting GPC3 (SEQ ID NO: 1), hinge region of CD8 (SEQ ID NO: 3), CD28 transmembrane region (SEQ ID NO: 6) and intracellular signaling domain (SEQ ID NO: 4) as well as intracellular segment CD3ζ of CD3 (SEQ ID NO: 5).

For the plasmid of IL12-GPC3-CAR, the NFAT6-IL12 sequence was inserted into the GPC3-CAR-T plasmid to construct a lentiviral plasmid expressing a second-generation of chimeric antigen receptor of GPC3 and IL12. The NFAT6-IL12 sequence is composed of 6*NFAT binding motif (SEQ ID NO: 7), IL2 minimal promoter (SEQ ID NO: 8), IL12 signal peptide and IL12 p40 (SEQ ID NO: 10), (G4S)3 Linker (SEQ ID NO: ID NO: 9), IL12 p35 (SEQ ID NO: 11), PA2 (SEQ ID NO: 20).

2. Lentivirus packaging, virus concentration and titer determination

a. Lentivirus packaging

1) 293T cells cultured to the $6^{th}$ to $10^{th}$ passage at a density of $5\times10^6$ were inoculated in a petri dish, and cultured overnight at 37°C, 5% CO2, and the medium was DMEM containing 10% fetal bovine serum (Gibico);
2) target gene plasmids GPC3-CAR-T and IL12-GPC3-CAR T (5.4 μg) and packaging plasmids pRsv-REV (6.2 μg), RRE-PMDLg (6.2 μg), and Vsvg (2.4 μg) were dissolved into 800 μL of blank DMEM culture medium, and mixed well, so as to obtain a plasmid mixture;
3) 60 μg of PEI (1 μg/μl) was dissolved in 800 μl of serum-free DMEM culture medium, and incubated at room temperature for 5 minutes to obtain a PEI mixture;
4) the plasmid mixture was added into the PEI mixture, mixed, and incubated at room temperature for 20 minutes to form a transfection complex;
5) 1.6 ml of the transfection complex was added dropwise to a 10cm petri dish containing 11 ml of DMEM medium. After 4-5h hours, the medium of the transfected 293T cells was changed to DMEM medium with 10% FBS, and incubated at 37°C for 72h, and the virus supernatant was collected.

b. Lentivirus concentration

1) preparation of 5XPEG8000 NaCl: 8.766 g of NaCl and 50 g of PEG8000 were weighted and dissolved in 200 ml of Milli-Q pure water; sterilized at 121°C for 30min and stored at 4°C;
2) a 0.45μm filter was used to filter the virus supernatant, and 7.5 ml of 5X PEG-8000 NaCl stock solution was added into every 30 ml of the filtered virus initial solution; mixed once every 20-30 min for a total of 3-5 times; palced at 4°C overnight. After centrifugation, the supernatant was aspirated and discarded, the precipitate was left stood and the remaining liquid was removed; an appropriate amount of lentivirus lysis solution was added to dissolve the lentivirus precipitation; and the concentrated virus suspension was stored at -80°C.

The titer of lentivirus was detected by flow cytometry (preferably, the number of cells with a positive rate of 5-20%), the titer (U/mL) was calculate as = number of cells × positive rate/virus volume, and the titer of the concentrated virus were:

GPC3-CAR-T: 2.4×108 U/ml

IL12-GPC3-CAR T: 1×108 U/ml

3. Preparation of GPC3-CAR-T cells and IL12-GPC3-CAR T cells

1) anti-CD3 and CD28 antibody magnetic beads (Invitrogen) were used for T lymphocyte activation;
2) The day before infection, a 24-well plate was coated with RetroNectin: 380 μl of 5 μg/ml RetroNectin solution (PBS) was added to each well, and incubated overnight at 4°C;
3) the RetroNectin solution (PBS) in the 24-well plate was discarded, the plate was washed twice with 1 ml PBS; the activated T cells were inoculated in a 24-well plate coated with retronectin at $5\times10^5$ cells per well and 500 μl of culture medium; concentrated lentivirus was added into PBMC cells at MOI=15, centrifuged at 32°C, 1800 rpm, for 40 min, then transferred to a cell incubator;
4) Expansion culture: the infected cells were passaged every other day at a density of $5\times10^5$/mL, and the lymphocyte culture medium was supplemented with recombinant human IL-2 at a final concentration of 500 U/mL.

GPC3-CAR-T cells/IL12-GPC3-CAR T positive rate (purity) were determined by using conventional flow cytometry (the determination method is a conventional method in the art, such as the method disclosed in Kowolik et al., 2006) The results are shown in Figure 1B, in which UTD is T cells not transfected with CAR, GPC3-CAR is T cells transfected with pRRLSIN.cPPT.EF-1α-9F2-28Z, and IL12-GPC3-CAR is T cells transfected with pRRLSIN.cPPT.EF-1α-9F2-28Z-NFAT6-IL12. The results show that both of 9F2-28Z / 9F2-28Z-NFAT6-IL12 have a higher level of CAR expression on the surface.

**Example 2. Cytotoxicity assay of CAR-T/IL12-GPC3-CAR T cells targeting GPC3**

CytoTox 96 non-radioactive cytotoxicity detection kit (Promega) was used to detect cytotoxicity. Details can be found in the instructions of CytoTox 96 non-radioactive cytotoxicity detection kit.

1) Target cells: Huh-7, PLC/PRF/5, SK-HEP-1 cells were selected as target cells, among which Huh-7 cells and PLC/PRF/5 cells were GPC3-positive, and SK-HEP-1 cells were GPC3 negative;
2) Effector cells: UTD, GPC3-CAR-T and IL12-GPC3-CAR T cells were added at an effector target ratio of 3: 1, 1: 1 or 1: 3;

The experimental results are shown in Figure 2. Compared with the UTD group, both of GPC3-CAR-T / IL12-GPC3-CAR T showed stronger cytotoxicity in the *in vitro* toxicity experiment, and there was no significant difference. For GPC3-negative liver cancer cells, GPC3-CAR-T / IL12-GPC3-CAR T exhibited no enhanced killing effects.

**Example 3. Secretion of cytokines of GPC3-CAR-T/IL12-GPC3-CAR T cells**

The UTD/GPC3-CAR-T/IL12-GPC3-CAR T were incubated with the liver cancer cell line Huh-7, PLC/PRF/5, SK-HEP-1 cells at 1:1 for 24 hours, and then the supernatant was collected. The supernatant was detected by ELISA for secretion levels of cytokines.

The samples for detecting TNF-$\alpha$ and IL12 were not diluted, and the samples for detecting IL2 and IFN-$\gamma$ were diluted 20 times and 25 times respectively. Double-antibody sandwich enzyme-linked immunosorbent detection technology was used for ELISA kit. The results are shown in Figures 3A and 3B. Figure 3A shows that a higher level of IL12 secretion was detected for IL12-GPC3-CAR T, when co-incubated with GPC3+ tumor cells Huh-7, PLC/PRF/5, and IL12 was almost undetectable, when co-incubated with GPC3- tumor cells SK-HEP-1, which means that the CAR-T cell can only secrete IL12 when it recognizes GPC3. Figure 3B shows the release of cytokines IL-2, TNF-$\alpha$, IFN-$\gamma$ when UTD/GPC3- CAR-T/IL12-GPC3-CAR T were incubated with tumor cells for 24 hours, showing that IL12-GPC3-CAR T obviously secreted more IFN-$\gamma$.

**Example 4. Therapeutic effects of GPC3-CAR-T/IL12-GPC3-CAR T on Huh-7 subcutaneous xenograft tumors with medium and high endogenous expression of GPC3**

1. NOD/SCID mice were inoculated with Huh-7 subcutaneous transplantation tumor
Huh-7 cells were subcutaneously inoculated, $2\times10^6$/mouse, and the tumor volume reached 300 $mm^3$ after about 10 days. The mice were randomly grouped (n = 6);
2. Preparation and expansion of UTD/GPC3-CAR-T/IL12-GPC3-CAR T cells;
3. UTD/GPC3-CAR-T/IL12-GPC3-CAR T cell adoptive immunotherapy for Huh-7 transplanted tumors

1) for subcutaneously transplanted tumors, cyclophosphamide (100 mg/kg) was intraperitoneally injected within 10 days;
2) On Day 10, the tumor volume of NOD/SCID mice was measured and the mice were randomly grouped. The mice with subcutaneous transplanted tumor were divided into 3 groups, including: UTD group, GPC3-CAR-T group, IL12-GPC3-CAR T group (n = 6);
3) On Day 12, NOD/SCID mice were subjected to adoptive immunotherapy after being grouped. Low-dose of CAR-T were given through tail vein injection at a dose of $1\times10^7$ cells/mouse;
4) the volume of Huh-7 subcutaneously transplanted tumor was measured every 3-4 days, changes in the tumor volume in each group of mice were recorded, and the growth curves of tumor volume over time were plotted. The results are shown in Figure 4A and Figure 4B.

Figure 4A shows the growth of Huh-7 subcutaneously xenografted tumors. After CAR-T cells were administered for 2 days, IL12-GPC3-CAR T showed significant tumor suppression and killing effects, while in the GPC3-CAR-T group, the tumors showed an increasing trend starting from the $14^{th}$ day, which is not significantly different from the blank control group. It shows that IL12-GPC3-CAR T still shows excellent killing activity for tumors that cannot be controlled by GPC3-CAR.

Figure 4B shows that after IL12-GPC3-CAR T was administered, there was no significant difference in the body weight of mice in the GPC3-CAR-T group and the blank control group, indicating that although IL12-GPC3-CAR T showed significant anti-tumor effects, it did not cause side effects of treatment-related weight loss. In addition, the observation of mice in the experimental group showed that, after IL12-GPC3-CAR T were administered, the mice did not show obvious side effects.

Blood was taken from the tail vein on the 7th day and 10th day after the CAR-T injection, and the survival of CAR-T cells was detected by flow cytometry. The results are shown in Figure 5, which shows that on the 7th day, the survival number of CAR-T cells in the GPC3-CAR-T group was lower than 6000 cells/μL, and reached nearly 8000 cells/μL in the IL12-GPC3-CAR T group. On the 10th day, CAR-T cells in the GPC3-CAR-T group were significantly reduced (lower than 2000 cells/μL), while high cell survival was maintained in IL12-GPC3-CAR T group. Compared with the data of IL12-GPC3-CAR T on day 7, there is little difference. Compared with the GPC3-CAR-T group, in the IL12-GPC3-CAR T group, the number of CAR-T cells in peripheral blood is at least 75% higher (calculation method: IL12-GPC3-CAR T group-GPC3-CAR-T group/IL12-GPC3-CAR T group), indicating that the *in vivo* survival of T cells for IL12-GPC3-CAR T is far superior to CAR-T products not comprising IL12.

The peripheral blood of mice was collected on Day10, and the serum samples were tested for IFN-γ by ELISA. The detection method was the same as that in Example 3. The results are shown in Figure 6, which shows that the IFN-γ in the blood of mice in the IL12-GPC3-CAR T group was much higher than that in the GPC3-CAR-T group.

At the end of the experiment, the subcutaneous tumors were taken, and the infiltrating T cells and Ki67 in the tumor tissues were tested by immunohistochemistry. The results are shown in Figure 7. The CAR-T infiltration in IL12-GPC3-CAR T group was significantly more than that in GPC3-CAR-T group. In the IL12-GPC3-CAR T group, the nuclear proliferation of Marker Ki67 was significantly less than that of the other two groups. The decrease in Ki67 indicates that the proliferation of cells in the tumor is reduced, which means that IL12-GPC3-CAR T can effectively eliminate the GPC3+ tumor cells and inhibit the proliferation of tumor cells.

At the end of the experiment, the mice were euthanized and their subcutaneous tumors were weighed. As shown in Figure 8A and Figure 8B, the tumor volume and weight of the IL12-GPC3-CAR T group were significantly lower than those of the second-generation CAR-T group at the end of the experiment, and there are significant statistical differences.

**Example 5. Preparation of mouse CAR-T (mGPC3-CAR) and m-IL12-GPC3-CAR T (mouse CAR-IL12) cells**

In order to further verify the role of IL12 in mobilizing innate immunity in immunocompetent mice, mouse-derived CAR-T were prepared.

1. Construction of plasmid

Gene sequences of the mouse CD8α signal peptide (SEQ ID NO: 12), the anti-GPC3 monoclonal antibody (SEQ ID NO: 1), the mouse CD8α hinge region and transmembrane region (SEQ ID NO: 13), the mouse CD28 intracellular domain (SEQ ID NO: ID NO: 14) and the mouse CD3ζ intracellular domain (SEQ ID NO: 15) were connected in sequence, and the GPC3-CAR-T gene fragments were obtained by *in vitro* gene synthesis. The IRES-GFP fragment in the retroviral vector MSCV-IRES-GFP was replaced with Mlu I and Sal I restriction sites to obtain the recombinant vector MSCV-GPC3-CAR-T. For mouse CAR-IL12 (m-IL12-GPC3-CAR T), the mNFAT6-mIL12 sequence was inserted into the MSCV-GPC3-CAR-T plasmid to construct a plasmid expressing a second-generation of chimeric antigen receptor of GPC3 and mIL12 , MSCV-IL12-GPC3-CAR T. The mNFAT6-mIL12 sequence is composed of 6*mNFAT binding motif (SEQ ID NO: 16), mIL2 minimal promoter (SEQ ID NO: 17), mIL12 signal peptide and mIL12 p40 (SEQ ID NO: 18), (SG4)3 Linker (SEQ ID NO: ID NO: 9), mIL12 p35 (SEQ ID NO: 19), PA2 (SEQ ID NO: 20).

2. Packaging of retrovirus

1) 293T cells cultured to the 6th to 10th passage were inoculated at a density of 5×106 in a 10cm petri dish, and cultured overnight at 37°C, 5% $CO_2$, and the medium is DMEM containing 10% fetal bovine serum (Gibico);
2) 9 μg of MSCV-GPC3-CAR-T or MSCV-GPC3-CAR-T-mNFAT6-mIL12 and 9 μg of packaging plasmid pCL-Eco were dissolved into 800 μL of serum-free DMEM culture medium and mixd to obtain a plasmid mixture; and 54 μg of PEI (1 μg/μl) was dissolved in 800 μl of serum-free DMEM medium, mixed well, and incubated at room temperature for 5 minutes to obtain a PEI mixture;
3) a plasmid mixture was added to a PEI mixture and mixed, and incubated at room temperature for 20 minutes to form a transfection complex;
4) 1.6 ml of the transfection complex was added dropwise to a 10cm petri dish containing 11 ml of DMEM medium. After 4-5h hours, the medium of the transfected 293T cells was exchanged with 10% FBS DMEM medium, and incubated at 37°C for 72h. The virus supernatant was collected to obtain the retrovirus carrying mGPC3-CAR-T/mGPC3-CAR-T-mNFAT6-mIL12.

4. Preparation of mouse CAR-T lymphocytes

Healthy Balb/c mice were taken and Cell isolation kit (STEMCELL Technologies) was used to isolate mouse spleen T lymphocytes according to the instructions.

The purified mouse CD3+ T lymphocytes were added to Dynabeads Mouse T-activator CD3/CD28 (washed once with PBS) at a ratio of 1:1, and cultured in an incubator. The medium was RPMI 1640 complete medium for activation.

Mouse splenic T lymphocytes activated for 24 hours were added in a 48-well plate coated with retronectin at a cell number of $1\times10^6$ per well. 1 ml of retrovirus of mGPC3-CAR-T/mGPC3-CAR-T-mNFAT6-mIL12 was added, the medium was supplemented to 2 mL. After centrifuged at 2000 g, 32°C for 90 minutes, the cells were transferred an incubator for further culture. On the next day, the medium was exchanged to a fresh medium, and the cell density was adjusted to $5\times10^5$/mL, and passaged every 2-3 days.

Assay on mCAR-T cell chimeric antigen receptor expression:

$1\times10^6$ mCAR-T/mIL12-GPC3-CAR T cells were taken, divided into 2 portions, added in a 2 ml centrifuge tube, and centrifuged at 4°C, 400 g for 5min. The supernatant was discarded, and the precipitate was washed twice with PBS. 50 μl of PE-SA (diluted 1:200) was added into cells in the control group and incubated on ice for 45 min, washed twice with PBS (2% NBS); and resuspended as a control. 50μl of 1:50 diluted biotin-Goat anti Human IgG, F(ab')$_2$ antibody was added into cells in the test group, incubated on ice for 45 min; and washed twice with PBS (2% NBS); and 50 μl of PE-SA (diluted 1:200) antibody was added and incubated on ice for 45 min.

2 ml PBS (2% NBS) was added to resuspend the cells, and centrifuged at 4°C, 400 g for 5 minutes to discard the supernatant (repeated twice); 500 μl of PBS (2% NBS) was added, and FACS detection was performed. The results are shown in Figure 9, which show that there is a higher expression level of CAR on the surface of m9F2-m28Z/m9F2-m28Z-mNFAT6-mIL12, and UTD is mT cells without being transfected with CAR.

**Example 6. Cytotoxicity** assay **of mCAR-T/mIL12-GPC3-CAR T cells targeting GPC3**

CytoTox 96 non-radioactive cytotoxicity detection kit (Promega) was used, and details can be found in the instructions of CytoTox 96 non-radioactive cytotoxicity detection kit.

1) Target cells: 50 μL of $2\times10^5$/mL E0771-Parental, E0771-Parental-GPC3, E0771-Recurrent (a cell line isolated and obtained from tumor tissue, after E0771 were inoculated into mice and tumor formed), E0771-Recurrent-GPC3 cells were inoculated into 96 well plate, respectively;
2) Effector cells: UTD, mGPC3-m28Z and mGPC3-m28Z-mNFAT6-mIL12 cells were added at an effector target ratio of 3:1, 1:1 or 1:3;

The experimental results are shown in Figure 10. Compared with the UTD group, both of mGPC3-CAR and mGPC3-m28Z-mNFAT6-mIL12 (mIL12-GPC3-CAR T) showed stronger cytotoxicity in *in vitro* toxicity experiments, and there is no obvious difference. For GPC3-negative tumor cells, mGPC3-CAR and mIL12-GPC3-CAR T have no enhanced killing effects.

**Example 7. Secretion of cytokines of mCAR-T/mIL12-GPC3-CAR T cell**

UTD/mGPC3-CAR/ mIL12-GPC3-CAR T were co-incubated with E0771-Parental, E0771-Parental-GPC3, E0771-Recurrent, E0771-Recurrent-GPC3 cells at 1:1 for 24 hours. The supernatant was collected, and the supernatant was detect by ELISA for cytokine secretion level. The samples for detecting mTNF-a, mIL12, and mIL2 were not diluted, and the samples for detecting mIFN-γ were diluted 50 times. Double antibody sandwich enzyme-linked immunosorbent detection technology was used for the ELISA kit. Specific anti-mouse IL-2, TNF-α, IFN-γ, IL-12 monoclonal antibodies were pre-coated on high-affinity ELISA plates, respectively. The standard, sample to be tested and biotinylated detection antibody were added to the wells of a microtiter plate. After incubation, the mIL-2, mTNF-a, mIFN-γ, and mIL-12 present in a sample will be combined with solid-phase antibody and detection antibody, respectively. After unbound substances were washed and removed, horseradish peroxidase-labeled streptavidin (streptavidin-HRP) was added. The plate was washed and TMB-developped. The intensity of the color response is directly proportional to the concentration of the above-mentioned cytokines in the sample. A quench solution was added to stop the reaction, and the absorbance value was measured at 450 nm wavelength (reference wavelength of 570-630 nm).

The results are shown in Figures 11A and 11B. Figure 11A shows the secretion of mIL-12 when UTD, mGPC3-CAR, mGPC3-m28Z-mNFAT6-mIL12 were co-incubated with tumor cells for 24 hours, and when co-incubated with GPC3+ tumor cells, mGPC3-m28Z-mNFAT6-mIL12 (mIL12-GPC3-CAR T) exhibited a higher secretion level of IL12, indicating that IL12 secretion in mice can also be inducible. Figure 11B shows the release of cytokines mIL-2, mTNF-a, and mIFN-γ when UTD/mGPC3-m28Z/mIL12-GPC3-CAR T was co-incubated with tumor cells for 24 hours, and mIL12-GPC3-CAR T secreted significantly more IFN-γ.

**Example 8. Therapeutic effects of mGPC3-CAR-T/mIL12-GPC3-T on E0771-GPC3 subcutaneous xenograft tumor**

1. C57BL/6 mice were inoculated with E0771-GPC3 subcutaneously transplanted tumor
E0771-GPC3 cells were subcutaneously inoculated at 1×10[6]/mouse, and the tumor volume reached 300mm[3] after about 10 days; and the mice were randomly grouped (n = 6);
2. Preparation and expansion of mUTD/mGPC3-CAR-T/mIL12-GPC3-T cells;
3. Adoptive immunotherapy of MUTD/mGPC3-CAR-T/mIL12-GPC3-CAR-T cell on E0771-GPC3 transplanted tumor

    1) on Day 10, E0771-GPC3 mice were measured for the volume of transplantation tumor and randomly grouped. The mice with subcutaneous transplanted tumor were divided into 5 groups, including: UTD group, mGPC3-CAR-T 5×10[6] group, mGPC3-CAR-T 2×10[6] group, mIL12-GPC3-T 5×10[6] group, mIL12-GPC3-T 2×10[6] group (n = 6);
    2) the volume of E0771-GPC3 subcutaneously transplanted tumor was measured every 3-4 days, changes in the tumor volume in each group of mice were recorded, and the growth curves of tumor volume over time was plotted. The results are shown in Figure 12A and Figure 12B.

Figure 12A shows that the group administered with mIL12-GPC3-T 5×10[6]/animal through tail vein injection and the group administered with mIL12-GPC3-T 2×10[6]/animal through tail vein injection exhibited similar tumor-inhibiting effects at the end of the experiment, and both exhibited good tumor-inhibiting effects with a tumor-inhibiting rate over 90%, which shows that the ideal effects can still be achieved by infusing a small dosage of CAR-T for the large tumor load with the assistance of IL12. In contrast, in the group of mGPC3-CAR-T 5×10[6]/animal, the tumor volume is smaller than that in the mUTD group, however there is no statistical difference, which is due to the larger tumor volume (300mm[3]) when CAR-T was injected.
Figure 12B shows that after administration of mIL12-GPC3-T, there is no significant difference in the body weight of mice in the mGPC3-CAR-T group and the blank control group, indicating that IL12-GPC3-T exhibited significant anti-tumor effects, however side effects of treatment-related weight loss is not caused.
At the end of the experiment, the mice were euthanized and their subcutaneous tumors were weighed. The results are shown in Figure 13A and Figure 13B. At the end of the experiment, both of the tumor volume and the tumor weight in the mIL12-GPC3-T group were significantly lower than those of the second-generation of CAR-T group with a significant statistical difference.
The sequences involved in the present invention are summarized in the following table

| SEQ ID NO | Construct | Sequence |
|---|---|---|
| 1 | GPC3-scFv | Gaggtgcagctggtgcagagcggcgccgaggtgaagaagcccggcgccagcgtgaaggtgag ctgcaAggccagcggctacaccttcagcgactacgagatgcactgggtgcggcaggcccccggc cagggcctGgagtggatgggcgccatccaccccggcagcggcgacaccgcctacaaccagcgg ttcaagggccggGtgaccatcaccgccgacaagagcaccagcaccgcctacatggagctgagca gcctgcggagcgaggAcaccgccgtgtactactgcgcccggttctacagctacgcctactggggc cagggcaccctggtgacCgtgagcgccggtggaggcggttcaggcggaggtggttctggcggtg gcggatcggacatcgtgatgAcccagacccccctgagcctgcccgtgacccccggcgagcccgc cagcatcagctgccggagcagccAgagcctggtgcacagcaacggcaacacctacctgcagtgg tacctgcagaagcccggccagagcccCcagctgctgatctacaaggtgagcaaccggttcagcgg cgtgcccgaccggttcagcggcagcggcAgcggcaccgacttcaccctgaagatcagccgggt ggaggccgaggacgtgggcgtgtactactgcagccagagcatctacgtgccctacaccttcggcc agggcaccaagctggagatcaaacgt |
| 2 | human CD8α signal peptide | Atggccttaccagtgaccgccttgctcctgccgctgccttgctgctccacgccgccaggccg |

(continued)

| SEQ ID NO | Construct | Sequence |
|---|---|---|
| 3 | human CD8α hinge region | Accacgacgccagcgccgcgaccaccaacaccggcgcccaccatcgcgtcgcagcccctgtcc ctgcGcccagaggcgtgccggccagcggcggggggcgcagtgcacacgagggggctggactt cgcctgtgat |
| 4 | human CD28 intracellular domain | AggagtaagaggagcaggctcctgcacagtgactacatgaacAtgactccccgccgccccgg gccaacccgcaagcattaccagccctatgccccaccacgcgacttcgcagcctatcgctcc |
| 5 | human CD3ζ intracellular domain | Agagtgaagttcagcaggagcgcagacgcccccgcgtaccagcagggccagaaccagctcta taacgAgctcaatctaggacgaagagaggagtacgatgttttggacaagagacgtggccggga ccctgagatGgggggaaagccgcagagaaggaagaaccctcaggaaggcctgtacaatgaac tgcagaaagataagAtggcggaggcctacagtgagattgggatgaaaggcgagcgccggagg ggcaaggggcacgatggccTttaccagggtctcagtacagccaccaaggacacctacgacgcc cttcacatgcaggccctgccccctcgc |
| 6 | human CD28 transmembrane domain | Ttttgggtgctggtggtggttggtggagtcctggcttgctatagcttgctagtaacagtggcctttatt attttctgggtg |
| 7 | NFAT6 binding motif | Ggaggaaaaactgtttcatacagaaggcgtggaggaaaaactgtttcatacagaaggcgtggagg aaaAactgtttcatacagaaggcgtcaattgtcctcgacggaggaaaaactgtttcatacagaaggc gtggaggaaaaactgtttcatacagaaggcgtggaggaaaaactgtttcatacagaaggcgt |
| 8 | IL2 minimal promoter | AcattttgacacccccataatatttttccagaattaacagtataaattgcatctcttgttcaagagttCcc tatcactctctttaatcactactcacagtaacctcaactcctg |
| 9 | $(G_4S)_3$ linker | Ggtggaggcggttcaggcggaggtggttctggcggtggcggatcg |
| 10 | IL12 signal peptide and IL12p40 | AtgtgtcaccagcagttggtcatctcttggttttccctggtttttctggcatctcccctcgtggccatAtg ggaactgaagaaagatgtttatgtcgtagaattggattggtatccggatgcccctggagaaatggTgg tcctcacctgtgacacccctgaagaagatggtatcacctggaccttggaccagagcagtgaggtcTta ggctctggcaaaaccctgaccatccaagtcaaagagtttggagatgctggccagtacacctgtcaCaa aggaggcgaggttctaagccattcgctcctgctgcttcacaaaaaggaagatggaatttggtccaCtga tattttaaaggaccagaaagaacccaaaaataagacctttctaagatgcgaggccaagaattatTctgg acgtttcacctgctggtggctgacgacaatcagtactgatttgacattcagtgtcaaaagcagCagagg ctcttctgacccccaaggggtgacgtgcggagctgctacactctctgcagagagagtcagagGgga caacaaggagtatgagtactcagtggagtgccaggaggacagtgcctgcccagctgctgaggagA gtctgcccattgaggtcatggtggatgccgttcacaagctcaagtatgaaaactacaccagcagcttC ttcatcagggacatcatcaaacctgacccacccaagaacttgcagctgaagccattaaagaattctcG gcaggtggaggtcagctgggagtaccctgacacctggagtactccacattcctacttctccctgaca Ttctgcgttcaggtccagggcaagagcaagagagaaaagaaagatagagtcttcacggacaagac ctcAgccacggtcatctgccgcaaaaatgccagcattagcgtgcgggcccaggaccgctactatag ctcatcttggagcgaatgggcatctgtgccctgcagt |
| 11 | IL12p35 | Agaaacctccccgtggccactccagacccaggaatgttcccatgccttcaccactcccaaaacct gctGagggccgtcagcaacatgctccagaaggccagacaaactctagaattttacccttgcacttc tgaagAgattgatcatgaagatatcacaaaagataaaaccagcacagtggaggcctgtttaccatt ggaattaAccaagaatgagagttgcctaaattccagagagacctctttcataactaatgggagttgc ctggcctcCagaaagacctcttttatgatggccctgtgccttagtagtatttatgaagacttgaagatg taccaggTggagttcaagaccatgaatgcaaagcttctgatggatcctaagaggcagatctttcta gatcaaaacAtgctggcagttattgatgagctgatgcaggccctgaatttcaacagtg |
| | | agactgtgccacaaaaatcCtcccttgaagaaccggatttttataaaactaaaatcaagctctgcata cttcttcatgctttcagaattcgggcagtgactattgatagagtgatgagctatctgaatgcttcctaa |
| 12 | mouse CD8α signal peptide | Atggcctcaccgttgacccgctttctgtcgctgaacctgctgctgctgggtgagtcgattatcctgg ggagtggagaagct |

(continued)

| SEQ ID NO | Construct | Sequence |
|---|---|---|
| 13 | mouse CD8α hinge region and transmembrane region | ActactaccaagccagtgctgcgaactccctcacctgtgcaccctaccgggacatctcagccccagagAccagaagattgtcggccccgtggctcagtgaaggggaccggattggacttcgcctgtgatatttacaTctgggcacccttggccggaatctgcgtggcccttctgctgtccttgatcatcactctcatctgctaccacaggagccga |
| 14 | mouse CD28 intracellular domain | AatagtagaaggaacagactccttcaaagtgactacatgaacAtgactccccggaggcctgggctcactcgaaagccttaccagccctacgcccctgccagagactttgcagcgtaccgcccc |
| 15 | mouse CD3ζ intracellular domain | AgcaggagtgcagagactgctgccaacctgcaggaccccaaccagctctacaatgagctcaatctaggGcgaagagaggaatatgacgtcttggagaagaagcgggctcgggatccagagatgggaggcaaacagcAgaggaggaggaacccccaggaaggcgtatacaatgcactgcagaaagacaagatggcagaagcctacAgtgagatcggcacaaaaggcgagaggcggagaggcaagg ggcacgatggcctttaccagggtctcagCactgccaccaaggacacctatgatgccctgcatatgcagaccctggcc |
| 16 | mNFAT6 binding motif | AagaggaaaatttgtttcatacagaaggcgttaagaggaaaatttgtttcatacagaaggcgttaagaGgaaaatttgtttcatacagaaggcgttaagaggaaaatttgtttcatacagaaggcgttaagaggaaaatttgtttcatacagaaggcgttaagaggaaaatttgtttcatacagaaggcgtt |
| 17 | mIL2 minimal promoter | AacatcgtgacacccccatattatttttccagcattaacagtAtaaattgcctcccatgctgaagagctgcctatcacccttgctaatcactcctcacagtgacctcaagtcct |
| 18 | mIL12 signal peptide and mIL12p40 | AtgtgtcctcagaagctaaccatctcctggtttgccatcgttttgctggtgtctccactcatggccatGtgggagctggagaaagacgtttatgttgtagaggtggactggactcccgatgcccctggagaaacagTgaacctcacctgtgacacgcctgaagaagatgacatcacctggacctcagaccagagacatggagtcAtaggctctggaaagaccctgaccatcactgtcaaagagtttctagatgctggccagtacacctgccaCaaaggaggcgagactctgagccactcacatctgctgctccacaagaaggaaaatggaatttggtccaCtgaaattttaaaaaatttcaaaaacaagactttcctgaagtgtgaagcaccaaattactccggacggTtcacgtgctcatggctggtgcaaagaaacatggacttgaagttcaacatcaagagcagtagcagttcCcctgactctcgggcagtgacatgtggaatggcgtctctgtctgcagagaaggtcacactggaccaaaGggactatgagaagtattcagtgtcctgccaggaggatgtcacctgcccaactgccgaggagaccctgCccattgaactggcgttggaagcacggcagcagaataaatatgagaactacagcaccagcttcttcatCagggacatcatcaaaccagacccgcccaagaacttgcagatgaagcctttgaagaactcacaggtggAggtcagctgggagtaccctgactcctggagcactccccattcctacttctccctcaagttctttgttCgaatccagcgcaagaaagaaaagatgaaggagacagaggaggggtgtaaccagaaaggtgcgttcctCgtagagaagacatctaccgaagtccaatgcaaaggcgggaatgtctgcgtgcaagctcaggatcgctattacaattcctcatgcagcaagtgggcatgtgttccctgcagggtccgatcc |
| 19 | mIL12p35 | AgggtcattccagtctctggacctgccaggtgtcttagccagtcccgaaacctgctgaagaccacagaTgacatggtgaagacggccagagaaaaactgaaacattattcctgcactgctgaagacatcgatcatgAagacatcacacgggaccaaaccagcacattgaagacctgtttaccactggaactacacaagaacgagAgttgcctggctactagagagacttcttccacaacaagagggagctgcctgcccccacagaagacgtcTttgatgatgaccctgtgccttggtagcatctatgaggacttgaagatgtaccagacagagttccaggCcatcaacgcagcacttcagaatcacaaccatcagcagatcattctagacaagggcatgctggtggccAtcgatgagctgatgcagtctctgaatcataatggcgagactctgcgccagaaacctcctgtgggagaAgcagacccttacagagtgaaaatgaagctctgcatcctgcttcacgccttcagcacccgcgtcgtgaccatcaacagggtgatgggctatctgagctccgcc |
| 20 | PA2 | AataaaatatctttattttcattacatctgtgtgttggttttTtgtgtgag |

(continued)

| SEQ ID NO | Construct | Sequence |
|---|---|---|
| 21 | 9F2-28Z | EVQLVQSGAEVKKPGASVKVSCKASGYTFSDYEMHWVRQAPGQGLEWMGAIHPGSGDTAYNQRFKGRVTITADKSTSTAYMELSSLRSEDTAVYYCARFYSYAYWGQGTLVTVSAGGGGSGGGGSGGGGSDIVMTQTPLSLPVTPGEPASISCRSSQSLVHSNGNTYLQWYLQKPGQSPQLLIYKVSNRFSGVPDRFSGSGSGTDFTLKISRVEAEDVGVYYCSQSIYVPYTFGQGTKLEIKRTTTPAPRPPTPAPTIASQPLSLRPEACRPAAGGAVHTRGLDFACDFWVLVV |
| | | VGGVLACYSLLVTVAFIIFWVRSKRSRLLHSDYMNMTPRRPGPTRKHYQPYAPPRDFAAYRSRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| 22 | GPC3-BBZ | EVQLVQSGAEVKKPGASVKVSCKASGYTFSDYEMHWVRQAPGQGLEWMGAIHPGSGDTAYNQRFKGRVTITADKSTSTAYMELSSLRSEDTAVYYCARFYSYAYWGQGTLVTVSAGGGGSGGGGSGGGGSDIVMTQTPLSLPVTPGEPASISCRSSQSLVHSNGNTYLQWYLQKPGQSPQLLIYKVSNRFSGVPDRFSGSGSGTDFTLKISRVEAEDVGVYYCSQSIYVPYTFGQGTKLEIKRTTTPAPRPPTPAPTIASQPLSLRPEACRPAAGGAVHTRGLDFACDIYIWAPLAGTCGVLLLSLVITLYCKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |
| 23 | GPC3-28BBZ | EVQLVQSGAEVKKPGASVKVSCKASGYTFSDYEMHWVRQAPGQGLEWMGAIHPGSGDTAYNQRFKGRVTITADKSTSTAYMELSSLRSEDTAVYYCARFYSYAYWGQGTLVTVSAGGGGSGGGGSGGGGSDIVMTQTPLSLPVTPGEPASISCRSSQSLVHSNGNTYLQWYLQKPGQSPQLLIYKVSNRFSGVPDRFSGSGSGTDFTLKISRVEAEDVGVYYCSQSIYVPYTFGQGTKLEIKRTTTPAPRPPTPAPTIASQPLSLRPEACRPAAGGAVHTRGLDFACDFWVLVVVGGVLACYSLLVTVAFIIFWVRSKRSRLLHSDYMNMTPRRPGPTRKHYQPYAPPRDFAAYRSKRGRKKLLYIFKQPFMRPVQTTQEEDGCSCRFPEEEEGGCELRVKFSRSADAPAYQQGQNQLYNELNLGRREEYDVLDKRRGRDPEMGGKPQRRKNPQEGLYNELQKDKMAEAYSEIGMKGERRRGKGHDGLYQGLSTATKDTYDALHMQALPPR |

(continued)

| SEQ ID NO | Construct | Sequence |
|---|---|---|
| 24 | GPC3-z | EVQLVQSGAEVKKPGASVKVSCKASGYTFSDYEMH<br>WVRQAPGQGLEWMGAIHPGSGDTAYNQRFKGRVT<br>ITADKSTSTAYMELSSLRSEDTAVYYCARFYSYAYW<br>GQGTLVTVSAGGGGSGGGGSGGGGSDIVMTQTPLS<br>LPVTPGEPASISCRSSQSLVHSNGNTYLQWYLQKPG<br>QSPQLLIYKVSNRFSGVPDRFSGSGSGTDFTLKISR<br>VEAEDVGVYYCSQSIYVPYTFGQGTKLEIKRrvkfsrsa<br>dApayqqgqnqlynelnlgrreeydvldkrrgrdpemggkpqrrKnpqeg<br>lynelqkdkmaeayseigmkgerrrgkghdglyqglstatkdtydalhmqalp<br>Pr |
| 25 | GPC3-scFv | EVQLVQSGAEVKKPGASVKVSCKASGYTFSDYEMH<br>WVRQAPGQGLEWMGAIHPGSGDTAYNQRFKGRVT<br>ITADKSTSTAYMELSSLRSEDTAVYYCARFYSYAYW<br>GQGTLVTVSAGGGGSGGGGSGGGGSDIVMTQTPLS<br>LPVTPGEPASISCRSSQSLVHSNGNTYLQWYLQKPG<br>QSPQLLIYKVSNRFSGVPDRFSGSGSGTDFTLKISRV<br>EAEDVGVYYCSQSIYVPYTFGQGTKLEIKR |
| 26 | amino acid sequence of human IL12 | Mchqqlviswfslvflasplvaiwelkkdvyvveldwypdapgemvvltcd<br>tpeedgitWtldqssevlgsgktltiqvkefgdagqytchkggevlshsllllhk<br>kedgiwstdilkDqkepknktflrceaknysgrftcwwlttistdltfsvkssrg<br>ssdpqgvtcgaatlsaErvrgdnkeyeysvecqedsacpaaeeslpievmv<br>davhklkyenytssffirdiikpdPpknlqlkplknsrqvevsweypdtwst<br>phsyfsltfcvqvqgkskrekkdrvftdktsAtvicrknasisvraqdryysss<br>wsewasvpcsggggsggggsggggsrnlpvatpdpgMfpclhhsqnllra<br>vsnmlqkarqtlefypctseeidheditkdktstveaclpleltkNesclnsrets<br>fitngsclasrktsfmmalclssiyedlkmyqvefktmnakllmdpkrQifld<br>qnmlavidelmqalnfnsetvpqkssleepdfyktkiklcillhafriravtidrv<br>msylnas* |
| 27 | amino acid sequence of mouce IL12 | Mcpqkltiswfaivllvsplmamwelekdvyvvevdwtpdapgetvnltc<br>dtpeedditWtsdqrhgvigsgktltitvkefldagqytchkggetlshshllllh<br>kkengiwsteilkNfknktflkceapnysgrftcswlvqrnmdlkfnikssss<br>spdsravtcgmaslsaekvTldqrdyekysvscqedvtcptaeetlpielalea<br>rqqnkyenystsffirdiikpdppKnlqmkplknsqvevsweypdswstph<br>syfslkffvriqrkkekmketeegcnqkgaflVektstevqckggnvcvqaq<br>dryynsscskwacvpcrvrsggggsggggsggggsrvipVsgparclsqsrn<br>llkttddmvktareklkhysctaedidheditrdqtstlktclpleLhknesclatr<br>etssttrgsclppqktslmmtlclgsiyedlkmyqtefqainaalqnhnhqqiil<br>dkgmlvaidelmqslnhngetlrqkppvgeadpyrvkmklcillhafstrvvt<br>inrvmgylssa* |

(continued)

| SEQ ID NO | Construct | Sequence |
|---|---|---|
| 28 | IL12-9F2-28Z | Cgatggctccggtgcccgtcagtgggcagagcgcacatcgcccacagtcccc<br>gagaagtTggggggaggggtcggcaattgaaccggtgcctagagaaggtgg<br>cgcggggtaaactggGaaagtgatgtcgtgtactggctccgcctttttcccgag<br>ggtgggggagaaccgtatatAagtgcagtagtcgccgtgaacgttctttttcgca<br>acgggtttgccgccagaacacaggTgtcgtgacgcggatccaggcctaagctt<br>acgcgtcctagcgctaccggtcgccaccatGgccttaccagtgaccgccttgct<br>cctgccgctggccttgctgctccacgccgccaggcCggaggtgcagctggtgc<br>agagcggcgccgaggtgaagaagcccggcgccagcgtgaagGtgagctgca<br>aggccagcggctacaccttcagcgactacgagatgcactgggtgcggcaGgcc<br>cccggccagggcctggagtggatgggcgccatccaccccggcagcggcgaca<br>ccgCctacaaccagcggttcaagggccgggtgaccatcaccgccgacaagag<br>caccagcaccGcctacatggagctgagcagcctgcggagcgaggacaccgc<br>cgtgtactactgcgcccgGttctacagctacgcctactggggccagggcaccct<br>ggtgaccgtgagcgccggtggagGcggttcaggcggaggtggttctggcggt<br>ggcggatcggacatcgtgatgacccagaccCccctgagcctgcccgtgaccc<br>ccggcgagcccgccagcatcagctgccggagcagccaGagcctggtgcaca<br>gcaacggcaacacctacctgcagtggtacctgcagaagcccggccAgagcc<br>cccagctgctgatctacaaggtgagcaaccggttcagcggcgtgcccgaccg<br>gTtcagcggcagcggcagcggcaccgacttcaccctgaagatcagccgggt<br>ggaggccgaGgacgtgggcgtgtactactgcagccagagcatctacgtgccc<br>tacaccttcggccaggGcaccaagctggagatcaaacgtaccacgacgccag<br>cgccgcgaccaccaacaccggcgCccaccatcgcgtcgcagcccctgtccc<br>tgcgcccagaggcgtgccggccagcggcgggGggcgcagtgcacacgag<br>ggggctggacttcgcctgtgatttttgggtgctggtggtggTtggtggagtcct<br>ggcttgctatagcttgctagtaacagtggcctttattattttctggGtgaggagta<br>agaggagcaggctcctgcacagtgactacatgaacatgactccccgccgC<br>cccgggccaacccgcaagcattaccagccctatgccccaccacgcgactt<br>cgcagcctAtcgctccagagtgaagttcagcaggagcgcagacgccccc<br>gcgtaccagcagggccagAaccagctctataacgagctcaatctaggacg<br>aagagaggagtacgatgttttggacaaGagacgtggccgggaccctgaga<br>tggggggaaagccgcagagaaggaagaaccctcaggAaggcctgtaca<br>atgaactgcagaaagataagatggcggaggcctacagtgagattgggAtg<br>aaaggcgagcgccggaggggcaaggggcacgatggcctttaccagggtc<br>tcagtacAgccaccaaggacacctacgacgcccttcacatgcaggccctg<br>ccccctcgctaggtcgAcaatcaacctctggattacaaaatttgtgaaagat<br>tgactggtattcttaactatgttGctccttttacgctatgtggatacgctgcttta<br>atgcctttgtatcatgctattgcttcCcgtatggctttcattttctcctccttgtat |

(continued)

| SEQ ID NO | Construct | Sequence |
|---|---|---|
| | | aaatcctggttgctgtctctttatgaggAgttgtggcccgttgtcaggcaacg tggcgtggtgtgcactgtgtttgctgacgcaaccCccactggttggggcattg ccaccacctgtcagctcctttccgggactttcgctttcccCctccctattgccac ggcggaactcatcgccgcctgccttgcccgctgctggacaggggCtcggct gttgggcactgacaattccgtggtgttgtcggggaagctgacgtcctttccaT ggctgctcgcctgtgttgccacctggattctgcgcgggacgtccttctgctacg tcccTtcggccctcaatccagcggaccttccttcccgcggcctgctgccggc tctgcggcctcTtccgcgtcttcgccttcgccctcagacgagtcggatctccc tttgggccgcctccccgCctggaattcgctagcctcgagctcacacaaaaa accaacacacacagatgtaatgaaaatAaagatattttattgcggccgctttag gaagcattcagatagctcatcactctatcaatAgtcactgcccgaattctgaa agcatgaagaagtatgcagagcttgattttagttttatAaaaatccggttcttc aagggaggatttttgtggcacagtctcactgttgaaattcaggGcctgcat cagctcatcaataactgccagcatgttttgatctagaaagatctgcctcttA ggatccatcagaagctttgcattcatggtcttgaactccacctggtacatctt caagtCttcataaatactactaaggcacagggccatcataaaagaggtctt tctggaggccaggCaactcccattagttatgaaagaggtctctctggaattt aggcaactctcattcttggtTaattccaatggtaaacaggcctccactgtgc tggttttatcttttgtgatatcttcatGatcaatctcttcagaagtgcaagggta aaattctagagtttgtctggccttctggagcAtgttgctgacggccctcagc aggttttgggagtggtgaaggcatgggaacattcctggGtctggagtggc cacggggaggtttctagatccgccgccacccgacccaccaccgcccgA gccaccgccaccactgcagggcacagatgcccattcgctccaagatgag ctatagtagCggtcctgggcccgcacgctaatgctggcatttttgcggcag atgaccgtggctgaggtCttgtccgtgaagactctatctttcttttctctcttgc tcttgccctggacctgaacgcAgaatgtcagggagaagtaggaatgtgga gtactccaggtgtcagggtactcccagctgAcctccacctgccgagaattc tttaatggcttcagctgcaagttcttgggtgggtcaggTttgatgatgtccct gatgaagaagctgctggtgtagttttcatacttgagcttgtgaaCggcatcc accatgacctcaatgggcagactctcctcagcagctgggcaggcactgtc cTcctggcactccactgagtactcatactccttgttgtcccctctgactctct ctgcagaGagtgtagcagctccgcacgtcaccccttgggggtcagaag agcctctgctgcttttgaCactgaatgtcaaatcagtactgattgtcgtcag ccaccagcaggtgaaacgtccagaAtaattcttggcctcgcatcttagaa aggtcttatttttgggttctttctggtcctttAaaatatcagtggaccaaattcc atcttcctttttgtgaagcagcaggagcgaatggcTtagaacctcgcctcc tttgtgacaggtgtactggccagcatctccaaactctttgacTtggatggtc agggttttgccagagcctaagacctcactgctctggtccaaggtccagGt gataccatcttcttcaggggtgtcacaggtgaggaccaccatttctccagg ggcatCcggataccaatccaattctacgacataaacatctttcttcagttc ccatatggccacGaggggagatgccagaaaaaccagggaaaacca agagatgaccaactgctggtgacacAtggtggcgaccggtagcgcta ggtcatatgcaggagttgaggttactgtgagtagtgAttaaagagagtg atagggaactcttgaacaagagatgcaatttatactgttaattctGgaaaa atattatgggggtgtcaaaatgtcccgggacaattgacgccttctgtatga aAcagtttttcctccacgccttctgtatgaaacagtttttcctccacgccttc tgtatgAaacagtttttcctccgtcgaggacaattgacgccttctgtatgaa acagtttttcctCcacgccttctgtatgaaacagtttttcctccacgccttctg tatgaaacagtttttcCtcc |

(continued)

| SEQ ID NO | Construct | Sequence |
|---|---|---|
| 29 | IL12-9F2-BBZ | Cgatggctccggtgcccgtcagtgggcagagcgcacatcgcccacagtc<br>cccgagaagTtggggggaggggtcggcaattgaaccggtgcctagaga<br>aggtggcgcggggtaaactGggaaagtgatgtcgtgtactggctccgcc<br>tttttcccgagggtgggggagaaccgtaTataagtgcagtagtcgccgtg<br>aacgttctttttcgcaacgggtttgccgccagaacaCaggtgtcgtgacgcg<br>gatccaggcctaagcttacgcgtcctagcgctaccggtcgccAccatggc<br>cttaccagtgaccgccttgctcctgccgctggccttgctgctccacgccgC<br>caggccggaggtgcagctggtgcagagcggcgccgaggtgaagaag<br>cccggcgccagCgtgaaggtgagctgcaaggccagcggctacacctt<br>cagcgactacgagatgcactggGtgcggcaggcccccggccagggc<br>ctggagtggatgggcgccatccaccccggcagcgGcgacaccgccta<br>caaccagcggttcaagggccgggtgaccatcaccgccgacaagagCa<br>ccagcaccgcctacatggagctgagcagcctgcggagcgaggacaccg<br>ccgtgtacTactgcgcccggttctacagctacgcctactggggccagggc<br>accctggtgaccgtgaGcgccggtggaggcggttcaggcggaggtggt<br>tctggcggtggcggatcggacatcgtGatgacccagacccccctgagcc<br>tgcccgtgacccccggcgagcccgccagcatcagcTgccggagcagc<br>cagagcctggtgcacagcaacggcaacacctacctgcagtggtaccTg<br>cagaagcccggccagagcccccagctgctgatctacaaggtgagcaac<br>cggttcagCggcgtgcccgaccggttcagcggcagcggcagcggca<br>ccgacttcaccctgaagatcAgccgggtggaggccgaggacgtggg<br>cgtgtactactgcagccagagcatctacgtgcCctacaccttcggccag<br>ggcaccaagctggagatcaaacgtaccacgacgccagcgccGcgac<br>caccaacaccggcgcccaccatcgcgtcgcagcccctgtccctgcgcc<br>cagagGcgtgccggccagcggcggggggcgcagtgcacacgaggg<br>ggctggacttcgcctgtgAtttttgggtgctggtggtggttggtggagtcc<br>tggcttgctatagcttgctagtaacAgtggcctttattattttctgggtgaaa<br>cggggcagaaagaaactcctgtatatattcAaacaaccatttatgagacc<br>agtacaaactactcaagaggaagatggctgtagctgccGatttccagaa<br>gaagaagaaggaggatgtgaactgagagtgaagttcagcaggagcg<br>cAgacgcccccgcgtaccagcagggccagaaccagctctataacg<br>agctcaatctaggaCgaagagaggagtacgatgttttggacaagag<br>acgtggccgggaccctgagatggggggGaaagccgcagagaagga<br>agaaccctcaggaaggcctgtacaatgaactgcagaaagaTaagat<br>ggcggaggcctacagtgagattgggatgaaaggcgagcgccgga<br>ggggcaagGggcacgatggcctttaccagggtctcagtacagccac<br>caaggacacctacgacgcccTtcacatgcaggccctgccccctcgc<br>taggtcgacaatcaacctctggattacaaaatTtgtgaaagattgactg<br>gtattcttaactatgttgctccttttacgctatgtggatacGctgctttaat<br>gcctttgtatcatgctattgcttcccgtatggctttcattttctcctCcttgt<br>ataaatcctggttgctgtctctttatgaggagttgtggcccgttgtcaggc<br>aAcgtggcgtggtgtgcactgtgtttgctgacgcaacccccactggtt<br>ggggcattgccAccacctgtcagctcctttccgggactttcgctttccc<br>cctccctattgccacggcggAactcatcgccgcctgccttgcccgct<br>gctggacaggggctcggctgttgggcactgaCaattccgtggtgttg<br>tcggggaagctgacgtcctttccatggctgctcgcctgtgttGccacc<br>tggattctgcgcgggacgtccttctgctacgtcccttcggccctcaat<br>ccagCggaccttccttcccgcggcctgctgccggctctgcggcctc<br>ttccgcgtcttcgccttCgccctcagacgagtcggatctccctttggg<br>ccgcctccccgcctggaattcgctagccTcgagctcacacaaaaa<br>accaacacacagatgtaatgaaaataaagatattttattgcgGccgc<br>tttaggaagcattcagatagctcatcactctatcaatagtcactgccc<br>gaattcTgaaagcatgaagaagtatgcagagcttgattttagtttta<br>taaaaatccggttcttcaAgggaggatttttgtggcacagtctcact<br>gttgaaattcagggcctgcatcagctcatcAataactgccagcatg<br>ttttgatctagaaagatctgcctcttaggatccatcagaagctTtgc<br>attcatggtcttgaactccacctggtacatcttcaagtcttcataaat<br>actactaAggcacagggccatcataaaagaggtctttctggaggc<br>caggcaactcccattagttatGaaagaggtctctctggaatttagg |

(continued)

| SEQ ID NO | Construct | Sequence |
|---|---|---|
| | | caactctcattcttggttaattccaatggtaaacAggcctccactg tgctggttttatcttttgtgatatcttcatgatcaatctcttcagaaGt gcaagggtaaaattctagagtttgtctggccttctggagcatgttg ctgacggccctCagcaggttttgggagtggtgaaggcatggg aacattcctgggtctggagtggccacggGgaggtttctagatc cgccgccacccgacccaccaccgcccgagccaccgccacca ctgCagggcacagatgcccattcgctccaagatgagctatagt agcggtcctgggcccgcacGctaatgctggcatttttgcggc agatgaccgtggctgaggtcttgtccgtgaagactcTatctttc ttttctctcttgctcttgccctggacctgaacgcagaatgtcaggg agaagTaggaatgtggagtactccaggtgtcagggtactccca gctgacctccacctgccgagaAttctttaatggcttcagctgcaa gttcttgggtgggtcaggtttgatgatgtccctgaTgaagaagct gctggtgtagttttcatacttgagcttgtgaacggcatccaccatg accTcaatgggcagactctcctcagcagctgggcaggcactg tcctcctggcactccactgaGtactcatactccttgttgtcccctct gactctctctgcagagagtgtagcagctccgcAcgtcacccctt gggggtcagaagagcctctgctgcttttgacactgaatgtcaaat caGtactgattgtcgtcagccaccagcaggtgaaacgtccagaa taattcttggcctcgcaTcttagaaaggtcttatttttgggttctttct ggtcctttaaaatatcagtggaccaaaTtccatcttcctttttgtga agcagcaggagcgaatggcttagaacctcgcctcctttgTgac aggtgtactggccagcatctccaaactctttgacttggatggtca gggttttgccAgagcctaagacctcactgctctggtccaaggt ccaggtgataccatcttcttcaggggTgtcacaggtgaggac caccatttctccaggggcatccggataccaatccaattctacgA cataaacatctttcttcagttcccatatggccacgaggggagatg ccagaaaaaccagGgaaaaccaagagatgaccaactgctggt gacacatggtggcgaccggtagcgctaggtCatatgcaggag ttgaggttactgtgagtagtgattaaagagagtgatagggaactc ttGaacaagagatgcaatttatactgttaattctggaaaaatattat gggggtgtcaaaatGtcccgggacaattgacgccttctgtatga aacagtttttcctccacgccttctgtatgAaacagtttttcctccac gccttctgtatgaaacagtttttcctccgtcgaggacaattGacg ccttctgtatgaaacagtttttcctccacgccttctgtatgaaacag tttttcctccacgccttctgtatgaaacagtttttcctcc |

(continued)

| SEQ ID NO | Construct | Sequence |
|---|---|---|
| 30 | IL12-9F2-28BBZ | Cgatggctccggtgcccgtcagtgggcagagcgcacatcgcc<br>cacagtccccgagaagtTgggggga ggggtcggcaattgaac<br>cggtgcctagagaaggtggcgcggggtaaactggGaaagtga<br>tgtcgtgtactggctccgcctttttcccgagggtgggggaga<br>accgtatatAagtgcagtagtcgccgtgaacgttctttttcg<br>caacgggtttgccgccagaacacaggTgtcgtgacgcggatc<br>caggcctaagcttacgcgtcctagcgctaccggtcgccacca<br>tGgccttaccagtgaccgccttgctcctgccgctggccttgc<br>tgctccacgccgccaggcCggaggtgcagctggtgcagagcg<br>gcgccgaggtgaagaagcccggcgccagcgtgaagGtgagct<br>gcaaggccagcggctacaccttcagcgactacgagatgcact<br>gggtgcggcaGgcccccggccagggcctggagtggatgggcg<br>ccatccaccccggcagcggcgacaccgCctacaaccagcggt<br>tcaagggccgggtgaccatcaccgccgacaagagcaccagca<br>ccGcctacatggagctgagcagcctgcggagcgaggacaccg<br>ccgtgtactactgcgcccgGttctacagctacgcctactggg<br>gccagggcaccctggtgaccgtgagcgccggtggagGcggtt<br>caggcggaggtggttctggcggtggcggatcggacatcgtga<br>tgacccagaccCccctgagcctgcccgtgacccccggcgagc<br>ccgccagcatcagctgccggagcagccaGagcctggtgcaca<br>gcaacggcaacacctacctgcagtggtacctgcagaagcccg<br>gccAgagcccccagctgctgatctacaaggtgagcaaccggt<br>tcagcggcgtgcccgaccggTtcagcggcagcggcagcggca |

(continued)

| SEQ ID NO | Construct | Sequence |
|---|---|---|
| | | ccgacttcaccctgaagatcagccgggtggaggccgaGgacg<br>tgggcgtgtactactgcagccagagcatctacgtgccctaca<br>ccttcggccaggGcaccaagctggagatcaaacgtaccacga<br>cgccagcgccgcgaccaccaacaccggcgCccaccatcgcgt<br>cgcagcccctgtccctgcgcccagaggcgtgccggccagcgg<br>cgggGggcgcagtgcacacgagggggctggacttcgcctgtg<br>atttttgggtgctggtggtggTtggtggagtcctggcttgct<br>atagcttgctagtaacagtggcctttattattttctggGtga<br>ggagtaagaggagcaggctcctgcacagtgactacatgaaca<br>tgactccccgccgCcccgggccaacccgcaagcattaccagc<br>cctatgccccaccacgcgacttcgcagcctAtcgctccaaac<br>ggggcagaaagaaactcctgtatatattcaaacaaccattta<br>tgagaCcagtacaaactactcaagaggaagatggctgtagct<br>gccgatttccagaagaagaagaAggaggatgtgaactgagag<br>tgaagttcagcaggagcgcagacgcccccgcgtaccagcAgg<br>gccagaaccagctctataacgagctcaatctaggacgaagag<br>aggagtacgatgttTtggacaagagacgtggccgggaccctg<br>agatgggggggaaagccgcagagaaggaagaaCcctcaggaag<br>gcctgtacaatgaactgcagaaagataagatggcggaggcct<br>acagtgAgattgggatgaaaggcgagcgccggaggggcaagg<br>ggcacgatggcctttaccagggtCtcagtacagccaccaagg<br>acacctacgacgcccttcacatgcaggccctgccccctcgCt<br>aggtcgacaatcaacctctggattacaaaatttgtgaaagat<br>tgactggtattcttaActatgttgctccttttacgctatgtg<br>gatacgctgctttaatgcctttgtatcatgctAttgcttccc<br>gtatggctttcattttctcctccttgtataaatcctggttgc<br>tgtctctTtatgaggagttgtggcccgttgtcaggcaacgtg<br>gcgtggtgtgcactgtgtttgctgAcgcaacccccactggtt<br>ggggcattgccaccacctgtcagctcctttccgggactttcG<br>ctttccccctccctattgccacggcggaactcatcgccgcct<br>gccttgcccgctgctgGacaggggctcggctgttgggcactg<br>acaattccgtggtgttgtcggggaagctgacgtCctttccat<br>ggctgctcgcctgtgttgccacctggattctgcgcgggacgt<br>ccttctgcTacgtcccttcggccctcaatccagcggaccttc<br>cttcccgcggcctgctgccggctctGcggcctcttccgcgtc<br>ttcgccttcgccctcagacgagtcggatctccctttgggccg<br>Cctccccgcctggaattcgctagcctcgagctcacacaaaaa<br>accaacacacagatgtaAtgaaaataaagatattttattgcg<br>gccgctttaggaagcattcagatagctcatcactCtatcaat<br>agtcactgcccgaattctgaaagcatgaagaagtatgcagag<br>cttgattttAgttttataaaaatccggttcttcaagggagga<br>tttttgtggcacagtctcactgttgaAattcagggcctgcat<br>cagctcatcaataactgccagcatgttttgatctagaaagat<br>cTgcctcttaggatccatcagaagctttgcattcatggtctt<br>gaactccacctggtacatCttcaagtcttcataaatactact<br>aaggcacagggccatcataaaagaggtctttctggAggccag<br>gcaactcccattagttatgaaagaggtctctctggaatttag<br>gcaactctcaTtcttggttaattccaatggtaaacaggcctc<br>cactgtgctggttttatcttttgtgatAtcttcatgatcaat<br>ctcttcagaagtgcaagggtaaaattctagagtttgtctggc<br>ctTctggagcatgttgctgacggccctcagcaggttttggga<br>gtggtgaaggcatgggaacAttcctgggtctggagtggccac<br>ggggaggtttctagatccgccgccacccgacccaccAccgcc<br>cgagccaccgccaccactgcagggcacagatgcccattcgct<br>ccaagatgagcTatagtagcggtcctgggcccgcacgctaat<br>gctggcatttttgcggcagatgaccgtgGctgaggtcttgtc<br>cgtgaagactctatctttcttttctctcttgctcttgccctg<br>gacCtgaacgcagaatgtcagggagaagtaggaatgtggagt<br>actccaggtgtcagggtactCccagctgacctccacctgccg |

(continued)

| SEQ ID NO | Construct | Sequence |
|---|---|---|
| | | agaattctttaatggcttcagctgcaagttcttgggtGggtc<br>aggtttgatgatgtccctgatgaagaagctgctggtgtagtt<br>ttcatacttgagCttgtgaacggcatccaccatgacctcaat<br>gggcagactctcctcagcagctgggcaggCactgtcctcctg<br>gcactccactgagtactcatactccttgttgtcccctctgac<br>tctcTctgcagagagtgtagcagctccgcacgtcaccccttg<br>ggggtcagaagagcctctgctGcttttgacactgaatgtcaa<br>atcagtactgattgtcgtcagccaccagcaggtgaaacGtcc<br>agaataattcttggcctcgcatcttagaaaggtcttattttt<br>gggttctttctggTcctttaaaatatcagtggaccaaattcc<br>atcttcctttttgtgaagcagcaggagcgaAtggcttagaac<br>ctcgcctcctttgtgacaggtgtactggccagcatctccaaa<br>ctcttTgacttggatggtcagggttttgccagagcctaagac<br>ctcactgctctggtccaaggtcCaggtgataccatcttcttc<br>aggggtgtcacaggtgaggaccaccatttctccaggggcAtc<br>cggataccaatccaattctacgacataaacatctttcttcag<br>ttcccatatggccaCgaggggagatgccagaaaaaccaggga<br>aaaccaagagatgaccaactgctggtgacacAtggtggcgac<br>cggtagcgctaggtcatatgcaggagttgaggttactgtgag<br>tagtgaTtaaagagagtgatagggaactcttgaacaagagat<br>gcaatttatactgttaattctggAaaaatattatgggggtgt<br>caaaatgtcccgggacaattgacgccttctgtatgaaacaGt<br>ttttcctccacgccttctgtatgaaacagtttttcctccacg<br>ccttctgtatgaaacAgtttttcctccgtcgaggacaattga<br>cgccttctgtatgaaacagtttttcctccacgccttctgtat<br>gaaacagtttttcctccacgccttctgtatgaaacagttttt<br>cctcc |

(continued)

| SEQ ID NO | Construct | Sequence |
|---|---|---|
| 31 | IL12-9F2-Z | Cgatggctccggtgcccgtcagtgggcagagcgcacatcgcc<br>cacagtccccgagaagttGgggggaggggtcggcaattgaac<br>cggtgcctagagaaggtggcgcggggtaaactgggaAagtga<br>tgtcgtgtactggctccgcctttttcccgagggtgggggaga<br>accgtatataagTgcagtagtcgccgtgaacgttctttttcg<br>caacgggtttgccgccagaacacaggtgtcGtgacgcggatc<br>caggcctaagcttacgcgtcctagcgctaccggtcgccacca<br>tggcctTaccagtgaccgccttgctcctgccgctggccttgc<br>tgctccacgccgccaggccggaggTgcagctggtgcagagcg<br>gcgccgaggtgaagaagcccggcgccagcgtgaaggtgagct<br>Gcaaggccagcggctacaccttcagcgactacgagatgcact<br>gggtgcggcaggcccccgGccagggcctggagtggatgggcg<br>ccatccaccccggcagcggcgacaccgcctacaaccAgcggt<br>tcaagggccgggtgaccatcaccgccgacaagagcaccagca<br>ccgcctacatggAgctgagcagcctgcggagcgaggacaccg<br>ccgtgtactactgcgcccggttctacagctAcgcctactggg<br>gccagggcaccctggtgaccgtgagcgccggtggaggcggtt<br>caggcgGaggtggttctggcggtggcggatcggacatcgtga<br>tgacccagaccccccctgagcctgcCcgtgacccccggcgagc<br>ccgccagcatcagctgccggagcagccagagcctggtgcaca<br>Gcaacggcaacacctacctgcagtggtacctgcagaagcccg<br>gccagagcccccagctgcTgatctacaaggtgagcaaccggt<br>tcagcggcgtgcccgaccggttcagcggcagcggcaGcggca<br>ccgacttcaccctgaagatcagccgggtggaggccgaggacg<br>tgggcgtgtactActgcagccagagcatctacgtgccctaca<br>ccttcggccagggcaccaagctggagatcaAacgtaccacga<br>cgccagcgccgcgaccaccaacaccggcgcccaccatcgcgt<br>cgcagcCcctgtccctgcgcccagaggcgtgccggccagcgg<br>cggggggcgcagtgcacacgagggGgctggacttcgcctgtg<br>atttttgggtgctggtggtggttggtggagtcctggcttgct<br>Atagcttgctagtaacagtggcctttattattttctgggtga<br>gagtgaagttcagcaggaGcgcagacgcccccgcgtaccagc |

(continued)

| SEQ ID NO | Construct | Sequence |
|---|---|---|
| | | agggccagaaccagctctataacgagctcaatctagGacgaa<br>gagaggagtacgatgttttggacaagagacgtggccgggacc<br>ctgagatgggggGaaagccgcagagaaggaagaaccctcagg<br>aaggcctgtacaatgaactgcagaaagataAgatggcggagg<br>cctacagtgagattgggatgaaaggcgagcgccggaggggca<br>aggggcAcgatggcctttaccagggtctcagtacagccacca<br>aggacacctacgacgcccttcacaTgcaggccctgccccctc<br>gctaggtcgacaatcaacctctggattacaaaatttgtgaaa<br>Gattgactggtattcttaactatgttgctccttttacgctat<br>gtggatacgctgctttaaTgcctttgtatcatgctattgctt<br>cccgtatggctttcattttctcctccttgtataaatCctggt<br>tgctgtctctttatgaggagttgtggcccgttgtcaggcaac<br>gtggcgtggtgtGcactgtgtttgctgacgcaacccccactg<br>gttggggcattgccaccacctgtcagctccTttccgggactt<br>tcgctttccccctccctattgccacggcggaactcatcgccg<br>cctgccTtgcccgctgctggacaggggctcggctgttgggca<br>ctgacaattccgtggtgttgtcggGgaagctgacgtcctttc<br>catggctgctcgcctgtgttgccacctggattctgcgcggga<br>Cgtccttctgctacgtcccttcggccctcaatccagcggacc<br>ttccttcccgcggcctgcTgccggctctgcggcctcttccgc<br>gtcttcgccttcgccctcagacgagtcggatctcccTttggg<br>ccgcctccccgcctggaattcgctagcctcgagctcacacaa<br>aaaaccaacacaCagatgtaatgaaaataaagatattttatt<br>gcggccgctttaggaagcattcagatagctCatcactctatc<br>aatagtcactgcccgaattctgaaagcatgaagaagtatgca<br>gagcttGattttagttttataaaaatccggttcttcaaggga<br>ggatttttgtggcacagtctcactGttgaaattcagggcctg<br>catcagctcatcaataactgccagcatgttttgatctagaaa<br>Gatctgcctcttaggatccatcagaagctttgcattcatggt<br>cttgaactccacctggtaCatcttcaagtcttcataaatact<br>actaaggcacagggccatcataaaagaggtctttctGgaggc<br>caggcaactcccattagttatgaaagaggtctctctggaatt<br>taggcaactctcAttcttggttaattccaatggtaaacaggc<br>ctccactgtgctggttttatcttttgtgatAtcttcatgatc<br>aatctcttcagaagtgcaagggtaaaattctagagtttgtct<br>ggccttCtggagcatgttgctgacggccctcagcaggttttg<br>ggagtggtgaaggcatgggaacatTcctgggtctggagtggc<br>cacggggaggtttctagatccgccgccacccgacccaccacc<br>Gcccgagccaccgccaccactgcagggcacagatgcccattc<br>gctccaagatgagctataGtagcggtcctgggcccgcacgct<br>aatgctggcatttttgcggcagatgaccgtggctgaGgtctt<br>gtccgtgaagactctatctttcttttctctcttgctcttgcc<br>ctggacctgaacGcagaatgtcagggagaagtaggaatgtgg<br>agtactccaggtgtcagggtactcccagctGacctccacctg<br>ccgagaattctttaatggcttcagctgcaagttcttgggtgg<br>gtcaggTttgatgatgtccctgatgaagaagctgctggtgta<br>gttttcatacttgagcttgtgaacGgcatccaccatgacctc<br>aatgggcagactctcctcagcagctgggcaggcactgtcctc<br>Ctggcactccactgagtactcatactccttgttgtcccctct<br>gactctctctgcagagagTgtagcagctccgcacgtcacccc<br>ttgggggtcagaagagcctctgctgcttttgacactGaatgt<br>caaatcagtactgattgtcgtcagccaccagcaggtgaaacg<br>tccagaataattCttggcctcgcatcttagaaaggtcttatt<br>tttgggttctttctggtcctttaaaatatcAgtggaccaaat<br>tccatcttccttttgtgaagcagcaggagcgaatggcttag<br>aacctcGcctcctttgtgacaggtgtactggccagcatctcc<br>aaactctttgacttggatggtcagGgttttgccagagcctaa<br>gacctcactgctctggtccaaggtccaggtgataccatcttc<br>Ttcaggggtgtcacaggtgaggaccaccatttctccaggggc |

(continued)

| SEQ ID NO | Construct | Sequence |
|---|---|---|
| | | atccggataccaatccaaTtctacgacataaacatctttctt<br>cagttcccatatggccacgagggggagatgccagaaaAaccag<br>ggaaaaccaagagatgaccaactgctggtgacacatggtggc<br>gaccggtagcgcTaggtcatatgcaggagttgaggttactgt<br>gagtagtgattaaagagagtgatagggaacTcttgaacaaga<br>gatgcaatttatactgttaattctggaaaaatattatggggg<br>tgtcaaAatgtcccgggacaattgacgccttctgtatgaaac<br>agtttttcctccacgccttctgtaTgaaacagtttttcctcc<br>acgccttctgtatgaaacagtttttcctccgtcgaggacaat<br>Tgacgccttctgtatgaaacagtttttcctccacgccttctg<br>Tatgaaacagtttttcctccacgccttctgtatgaaacagtt<br>tttcctcc |

(continued)

| SEQ ID NO | Construct | Sequence |
|---|---|---|
| 32 | mouse IL12-9F2-Z | Atggcctcaccgttgacccgctttctgtcgctgaacctgctg<br>ctgctgggtgagtcgattAtcctggggagtggagaagctgag<br>gtgcagctggtgcagagcggcgccgaggtgaagaagCccggc<br>gccagcgtgaaggtgagctgcaaggccagcggctacaccttc<br>agcgactacgagAtgcactgggtgcggcaggcccccggccag<br>ggcctggagtggatgggcgccatccaccccGgcagcggcgac<br>accgcctacaaccagcggttcaagggccgggtgaccatcacc<br>gccgacAagagcaccagcaccgcctacatggagctgagcagc<br>ctgcggagcgaggacaccgccgtgTactactgcgcccggttc<br>tacagctacgcctactggggccagggcaccctggtgaccgtg<br>Agcgccggtggaggcggttcaggcggaggtggttctggcggt<br>ggcggatcggacatcgtgAtgacccagacccccctgagcctg<br>cccgtgacccccggcgagcccgccagcatcagctgcCggagc<br>agccagagcctggtgcacagcaacggcaacacctacctgcag<br>tggtacctgcagAagcccggccagagcccccagctgctgatc<br>tacaaggtgagcaaccggttcagcggcgtgCccgaccggttc<br>agcggcagcggcagcggcaccgacttcaccctgaagatcagc<br>cgggtgGaggccgaggacgtgggcgtgtactactgcagccag<br>agcatctacgtgccctacaccttcGgccagggcaccaagctg<br>gagatcaaacgtactactaccaagccagtgctgcgaactccc<br>Tcacctgtgcaccctaccgggacatctcagccccagagacca<br>gaagattgtcggccccgtGgctcagtgaaggggaccggattg<br>gacttcgcctgtgatatttacatctgggcacccttgGccgga<br>atctgcgtggcccttctgctgtccttgatcatcactctcatc<br>tgctaccacaggAgccgaagcaggagtgcagagactgctgcc<br>aacctgcaggaccccaaccagctctacaatGagctcaatcta<br>gggcgaagagaggaatatgacgtcttggagaagaagcgggct<br>cgggatCcagagatgggaggcaaacagcagaggaggaggaac<br>ccccaggaaggcgtatacaatgcaCtgcagaaagacaagatg<br>gcagaagcctacagtgagatcggcacaaaaggcgagaggcgg<br>Agaggcaaggggcacgatggcctttaccagggtctcagcact<br>gccaccaaggacacctatGatgccctgcatatgcagaccctg<br>gcctaggtcgactcacacaaaaaaccaacacacagaTgtaat<br>gaaaataaagatattttattcgtacgttaggcggagctcaga<br>tagcccatcaccCtgttgatggtcacgacgcgggtgctgaag<br>gcgtgaagcaggatgcagagcttcattttcActctgtaaggg<br>tctgcttctcccacaggaggtttctggcgcagagtctcgcca<br>ttatgaTtcagagactgcatcagctcatcgatggccaccagc<br>atgcccttgtctagaatgatctgcTgatggttgtgattctga<br>agtgctgcgttgatggcctggaactctgtctggtacatcttc<br>Aagtcctcatagatgctaccaaggcacagggtcatcatcaaa<br>gacgtcttctgtgggggcAggcagctccctcttgttgtggaa<br>gaagtctctctagtagccaggcaactctcgttcttgTgtagt<br>tccagtggtaaacaggtcttcaatgtgctggtttggtcccgt<br>gtgatgtcttcaTgatcgatgtcttcagcagtgcaggaataa<br>tgtttcagtttttctctggccgtcttcaccAtgtcatctgtg<br>gtcttcagcaggtttcgggactggctaagacacctggcaggt |

(continued)

| SEQ ID NO | Construct | Sequence |
|---|---|---|
| | | ccagagActggaatgaccctagatccgccgccacccgaccca ccaccgcccgagccaccgccaccgGatcggaccctgcaggga acacatgcccacttgctgcatgaggaattgtaatagcgatcc Tgagcttgcacgcagacattcccgcctttgcattggacttcg gtagatgtcttctctacgAggaacgcacctttctggttacac ccctcctctgtctccttcatcttttctttcttgcgcTggatt cgaacaaagaacttgagggagaagtaggaatggggagtgctc caggagtcagggTactcccagctgacctccacctgtgagttc ttcaaaggcttcatctgcaagttcttgggcGggtctggtttg atgatgtccctgatgaagaagctggtgctgtagttctcatat ttattcTgctgccgtgcttccaacgccagttcaatgggcagg gtctcctcggcagttgggcaggtgAcatcctcctggcaggac actgaatacttctcatagtccctttggtccagtgtgaccttc Tctgcagacagagacgccattccacatgtcactgcccgagag tcaggggaactgctactgCtcttgatgttgaacttcaagtcc atgtttctttgcaccagccatgagcacgtgaaccgtCcggag taatttggtgcttcacacttcaggaaagtcttgtttttgaaa ttttttaaaattTcagtggaccaaattccattttccttcttg tggagcagcagatgtgagtggctcagagtcTcgcctcctttg tggcaggtgtactggccagcatctagaaactctttgacagtg atggtcAgggtctttccagagcctatgactccatgtctctgg tctgaggtccaggtgatgtcatctTcttcaggcgtgtcacag gtgaggttcactgtttctccaggggcatcgggagtccagtcc Acctctacaacataaacgtctttctccagctcccacatggcc atgagtggagacaccagcAaaacgatggcaaaccaggagatg gttagcttctgaggacacatggtggcgaccggtagcGctagg acgcgtaggacttgaggtcactgtgaggagtgattagcaagg gtgataggcagcTcttcagcatgggaggcaatttatactgtt aatgctggaaaaataatatgggggtgtcacGatgttcccggg acaattgaacgccttctgtatgaaacaaattttcctcttaac gccttcTgtatgaaacaaattttcctcttaacgccttctgta tgaaacaaattttcctcttaacgcCttctgtatgaaacaaat tttcctcttaacgccttctgtatgaaacaaattttcctctta acgccttctgtatgaaacaaattttcctctt |

(continued)

| SEQ ID NO | Construct | Sequence |
|---|---|---|
| 33 | mouse IL12-9F2-28Z | Atggcctcaccgttgacccgctttctgtcgctgaacctgctg ctgctgggtgagtcgattAtcctggggagtggagaagctgag gtgcagctggtgcagagcggcgccgaggtgaagaagCccggc gccagcgtgaaggtgagctgcaaggccagcggctacaccttc agcgactacgagAtgcactgggtgcggcaggcccccggccag ggcctggagtggatgggcgccatccaccccGgcagcggcgac accgcctacaaccagcggttcaagggccgggtgaccatcacc gccgacAagagcaccagcaccgcctacatggagctgagcagc ctgcggagcgaggacaccgccgtgTactactgcgcccggttc tacagctacgcctactggggccagggcaccctggtgaccgtg Agcgccggtggaggcggttcaggcggaggtggttctggcggt ggcggatcggacatcgtgAtgacccagacccccctgagcctg cccgtgacccccggcgagcccgccagcatcagctgcCggagc agccagagcctggtgcacagcaacggcaacacctacctgcag tggtacctgcagAagcccggccagagcccccagctgctgatc tacaaggtgagcaaccggttcagcggcgtgCccgaccggttc agcggcagcggcagcggcaccgacttcaccctgaagatcagc cgggtgGaggccgaggacgtgggcgtgtactactgcagccag agcatctacgtgccctacaccttcGgccagggcaccaagctg gagatcaaacgtactactaccaagccagtgctgcgaactccc Tcacctgtgcaccctaccgggacatctcagccccagagacca gaagattgtcggccccgtGgctcagtgaaggggaccggattg gacttcgcctgtgatatttacatctgggcacccttgGccgga atctgcgtggcccttctgctgtccttgatcatcactctcatc tgctaccacaggAgccgaaatagtagaaggaacagactcctt |

(continued)

| SEQ ID NO | Construct | Sequence |
|---|---|---|
| | | caaagtgactacatgaacatgactccccggAggcctgggctc<br>actcgaaagccttaccagccctacgcccctgccagagacttt<br>gcagcgTaccgccccagcaggagtgcagagactgctgccaac<br>ctgcaggaccccaaccagctctacAatgagctcaatctaggg<br>cgaagagaggaatatgacgtcttggagaagaagcgggctcgg<br>Gatccagagatgggaggcaaacagcagaggaggaggaacccc<br>caggaaggcgtatacaatGcactgcagaaagacaagatggca<br>gaagcctacagtgagatcggcacaaaaggcgagaggCggaga<br>ggcaaggggcacgatggcctttaccagggtctcagcactgcc<br>accaaggacaccTatgatgccctgcatatgcagaccctggcc<br>taggtcgactcacacaaaaaaccaacacacAgatgtaatgaa<br>aataaagatattttattcgtacgttaggcggagctcagatag<br>cccatcAccctgttgatggtcacgacgcgggtgctgaaggcg<br>tgaagcaggatgcagagcttcattTtcactctgtaagggtct<br>gcttctcccacaggaggtttctggcgcagagtctcgccatta<br>Tgattcagagactgcatcagctcatcgatggccaccagcatg<br>cccttgtctagaatgatcTgctgatggttgtgattctgaagt<br>gctgcgttgatggcctggaactctgtctggtacatcTtcaag<br>tcctcatagatgctaccaaggcacagggtcatcatcaaagac<br>gtcttctgtgggGgcaggcagctccctcttgttgtggaagaa<br>gtctctctagtagccaggcaactctcgttcTtgtgtagttcc<br>agtggtaaacaggtcttcaatgtgctggtttggtcccgtgtg<br>atgtctTcatgatcgatgtcttcagcagtgcaggaataatgt<br>ttcagtttttctctggccgtcttcAccatgtcatctgtggtc<br>ttcagcaggtttcgggactggctaagacacctggcaggtcca<br>Gagactggaatgaccctagatccgccgccacccgacccacca<br>ccgcccgagccaccgccaCcggatcggaccctgcagggaaca<br>catgcccacttgctgcatgaggaattgtaatagcgaTcctga<br>gcttgcacgcagacattcccgcctttgcattggacttcggta<br>gatgtcttctctAcgaggaacgcacctttctggttacacccc<br>tcctctgtctccttcatcttttctttcttgCgctggattcga<br>acaaagaacttgagggagaagtaggaatggggagtgctccag<br>gagtcaGggtactcccagctgacctccacctgtgagttcttc<br>aaaggcttcatctgcaagttcttgGgcgggtctggtttgatg<br>atgtccctgatgaagaagctggtgctgtagttctcatattta<br>Ttctgctgccgtgcttccaacgccagttcaatgggcagggtc<br>tcctcggcagttgggcagGtgacatcctcctggcaggacact<br>gaatacttctcatagtccctttggtccagtgtgaccTtctct<br>gcagacagagacgccattccacatgtcactgcccgagagtca<br>ggggaactgctaCtgctcttgatgttgaacttcaagtccatg<br>tttctttgcaccagccatgagcacgtgaacCgtccggagtaa<br>tttggtgcttcacacttcaggaaagtcttgtttttgaaattt<br>tttaaaAtttcagtggaccaaattccattttccttcttgtgg<br>agcagcagatgtgagtggctcagaGtctcgcctcctttgtgg<br>caggtgtactggccagcatctagaaactctttgacagtgatg<br>Gtcagggtctttccagagcctatgactccatgtctctggtct<br>gaggtccaggtgatgtcaTcttcttcaggcgtgtcacaggtg<br>aggttcactgtttctccaggggcatcgggagtccagTccacc<br>tctacaacataaacgtctttctccagctcccacatggccatg<br>agtggagacaccAgcaaaacgatggcaaaccaggagatggtt<br>agcttctgaggacacatggtggcgaccggtAgcgctaggacg<br>cgtaggacttgaggtcactgtgaggagtgattagcaagggtg<br>ataggcAgctcttcagcatgggaggcaatttatactgttaat<br>gctggaaaaataatatgggggtgtCacgatgttcccgggaca<br>attgaacgccttctgtatgaaacaaattttcctcttaacgcc<br>Ttctgtatgaaacaaattttcctcttaacgccttctgtatga<br>aacaaattttcctcttaaCgccttctgtatgaaacaaatttt<br>cctcttaacgccttctgtatgaaacaaattttcctcttaacg<br>ccttctgtatgaaacaaattttcctctt |

(continued)

| SEQ ID NO | Construct | Sequence |
|---|---|---|
| 34 | mouse IL12-9F2-BBZ | Atggcctcaccgttgacccgctttctgtcgctgaacctgctg ctgctgggtgagtcgattAtcctggggagtggagaagctgag gtgcagctggtgcagagcggcgccgaggtgaagaagCccggc gccagcgtgaaggtgagctgcaaggccagcggctacaccttc agcgactacgagAtgcactgggtgcggcaggcccccggccag ggcctggagtggatgggcgccatccaccccGgcagcggcgac accgcctacaaccagcggttcaagggccgggtgaccatcacc gccgacAagagcaccagcaccgcctacatggagctgagcagc ctgcggagcgaggacaccgccgtgTactactgcgcccggttc tacagctacgcctactggggccagggcaccctggtgaccgtg Agcgccggtggaggcggttcaggcggaggtggttctggcggt ggcggatcggacatcgtgAtgacccagacccccctgagcctg cccgtgacccccggcgagcccgccagcatcagctgcCggagc agccagagcctggtgcacagcaacggcaacacctacctgcag tggtacctgcagAagcccggccagagcccccagctgctgatc tacaaggtgagcaaccggttcagcggcgtgCccgaccggttc agcggcagcggcagcggcaccgacttcaccctgaagatcagc cgggtgGaggccgaggacgtgggcgtgtactactgcagccag agcatctacgtgccctacaccttcGgccagggcaccaagctg gagatcaaacgtactactaccaagccagtgctgcgaactccc Tcacctgtgcaccctaccgggacatctcagccccagagacca gaagattgtcggccccgtGgctcagtgaaggggaccggattg gacttcgcctgtgatatttacatctgggcacccttgGccgga atctgcgtggcccttctgctgtccttgatcatcactctcatc tgctaccacaggAgccgaaaatggatcaggaaaaaattcccc cacatattcaagcaaccatttaagaagaccActggagcagct caagaggaagatgcttgtagctgccgatgtccacaggaagaa gaaggaGgaggaggaggctatgagctgagcaggagtgcagag actgctgccaacctgcaggaccccAaccagctctacaatgag ctcaatctagggcgaagagaggaatatgacgtcttggagaag Aagcgggctcgggatccagagatgggaggcaaacagcagagg aggaggaacccccaggaaGgcgtatacaatgcactgcagaaa gacaagatggcagaagcctacagtgagatcggcacaAaaggc gagaggcggagaggcaaggggcacgatggcctttaccagggt ctcagcactgccAccaaggacacctatgatgccctgcatatg cagaccctggcctaggtcgactcacacaaaAaaccaacacac agatgtaatgaaaataaagatattttattcgtacgttaggcg gagctcAgatagcccatcaccctgttgatggtcacgacgcgg gtgctgaaggcgtgaagcaggatgCagagcttcattttcact ctgtaagggtctgcttctcccacaggaggtttctggcgcaga Gtctcgccattatgattcagagactgcatcagctcatcgatg gccaccagcatgccccttgTctagaatgatctgctgatggttg tgattctgaagtgctgcgttgatggcctggaactctGtctgg tacatcttcaagtcctcatagatgctaccaaggcacagggtc atcatcaaagacGtcttctgtgggggcaggcagctccctctt gttgtggaagaagtctctctagtagccaggCaactctcgttc ttgtgtagttccagtggtaaacaggtcttcaatgtgctggtt tggtccCgtgtgatgtcttcatgatcgatgtcttcagcagtg caggaataatgtttcagtttttctCtggccgtcttcaccatg tcatctgtggtcttcagcaggtttcgggactggctaagacac Ctggcaggtccagagactggaatgaccctagatccgccgcca cccgacccaccaccgcccGagccaccgccaccggatcggacc ctgcagggaacacatgcccacttgctgcatgaggaaTtgtaa tagcgatcctgagcttgcacgcagacattcccgcctttgcat tggacttcggtaGatgtcttctctacgaggaacgcacctttc tggttacacccctcctctgtctccttcatcTtttctttcttg cgctggattcgaacaaagaacttgagggagaagtaggaatgg ggagtgCtccaggagtcagggtactcccagctgacctccacc tgtgagttcttcaaaggcttcatcTgcaagttcttgggcggg |

(continued)

| SEQ ID NO | Construct | Sequence |
|---|---|---|
| | | tctggtttgatgatgtccctgatgaagaagctggtgctgtag Ttctcatatttattctgctgccgtgcttccaacgccagttca atgggcagggtctcctcgGcagttgggcaggtgacatcctcc tggcaggacactgaatacttctcatagtccctttggTccagt gtgaccttctctgcagacagagacgccattccacatgtcact gcccgagagtcaGgggaactgctactgctcttgatgttgaac ttcaagtccatgtttctttgcaccagccatGagcacgtgaac cgtccggagtaatttggtgcttcacacttcaggaaagtcttg tttttgAaatttttaaaatttcagtggaccaaattccattt tccttcttgtggagcagcagatgtGagtggctcagagtctcg cctcctttgtggcaggtgtactggccagcatctagaaactct Ttgacagtgatggtcagggtctttccagagcctatgactcca tgtctctggtctgaggtcCaggtgatgtcatcttcttcaggc gtgtcacaggtgaggttcactgtttctccaggggcaTcggga gtccagtccacctctacaacataaacgtctttctccagctcc cacatggccatgAgtggagacaccagcaaaacgatggcaaac caggagatggttagcttctgaggacacatgGtggcgaccggt agcgctaggacgcgtaggacttgaggtcactgtgaggagtga ttagcaAgggtgataggcagctcttcagcatgggaggcaatt tatactgttaatgctggaaaaataAtatgggggtgtcacgat gttcccgggacaattgaacgccttctgtatgaaacaaatttt Cctcttaacgccttctgtatgaaacaaattttcctcttaacg ccttctgtatgaaacaaaTtttcctcttaacgccttctgtat gaaacaaattttcctcttaacgccttctgtatgaaaCaaatt ttcctcttaacgccttctgtatgaaacaaattttcctctt |

(continued)

| SEQ ID NO | Construct | Sequence |
|---|---|---|
| 35 | mouse IL12-9F2-28BBZ | Atggcctcaccgttgacccgctttctgtcgctgaacctgctg ctgctgggtgagtcgattAtcctggggagtggagaagctgag gtgcagctggtgcagagcggcgccgaggtgaagaagCccggc gccagcgtgaaggtgagctgcaaggccagcggctacaccttc agcgactacgagAtgcactgggtgcggcaggcccccggccag ggcctggagtggatgggcgccatccacccccGgcagcggcgac accgcctacaaccagcggttcaagggccgggtgaccatcacc gccgacAagagcaccagcaccgcctacatggagctgagcagc ctgcggagcgaggacaccgccgtgTactactgcgcccggttc tacagctacgcctactggggccagggcaccctggtgaccgtg Agcgccggtggaggcggttcaggcggaggtggttctggcggt ggcggatcggacatcgtgAtgacccagacccccctgagcctg cccgtgacccccggcgagcccgccagcatcagctgcCggagc agccagagcctggtgcacagcaacggcaacacctacctgcag tggtacctgcagAagcccggccagagcccccagctgctgatc tacaaggtgagcaaccggttcagcggcgtgCccgaccggttc agcggcagcggcagcggcaccgacttcaccctgaagatcagc cgggtgGaggccgaggacgtgggcgtgtactactgcagccag agcatctacgtgccctacaccttcGgccagggcaccaagctg gagatcaaacgtactactaccaagccagtgctgcgaactccc Tcacctgtgcaccctaccgggacatctcagccccagagacca gaagattgtcggccccgtGgctcagtgaaggggaccggattg gacttcgcctgtgatatttacatctgggcacccttgGccgga atctgcgtggcccttctgctgtccttgatcatcactctcatc tgctaccacaggAgccgaaatagtagaaggaacagactcctt caaagtgactacatgaacatgactccccggAggcctgggctc actcgaaagccttaccagccctacgcccctgccagagacttt gcagcgTaccgccccaaatggatcaggaaaaaattcccccac atattcaagcaaccatttaagaagAccactggagcagctcaa gaggaagatgcttgtagctgccgatgtccacaggaagaagaa Ggaggaggaggaggctatgagctgagcaggagtgcagagact gctgccaacctgcaggacCccaaccagctctacaatgagctc aatctagggcgaagagaggaatatgacgtcttggagAagaag cgggctcgggatccagagatgggaggcaaacagcagaggagg |

(continued)

| SEQ ID NO | Construct | Sequence |
|---|---|---|
| | | aggaacccccagGaaggcgtatacaatgcactgcagaaagac aagatggcagaagcctacagtgagatcggcAcaaaaggcgag aggcggagaggcaaggggcacgatggcctttaccagggtctc agcactGccaccaaggacacctatgatgccctgcatatgcag accctggcctaggtcgactcacacAaaaaaccaacacacaga tgtaatgaaaataaagatattttattcgtacgttaggcggag Ctcagatagcccatcaccctgttgatggtcacgacgcgggtg ctgaaggcgtgaagcaggAtgcagagcttcattttcactctg taagggtctgcttctcccacaggaggtttctggcgcAgagtc tcgccattatgattcagagactgcatcagctcatcgatggcc accagcatgcccTtgtctagaatgatctgctgatggttgtga ttctgaagtgctgcgttgatggcctggaacTctgtctggtac atcttcaagtcctcatagatgctaccaaggcacagggtcatc atcaaaGacgtcttctgtgggggcaggcagctccctcttgtt gtggaagaagtctctctagtagccAggcaactctcgttcttg tgtagttccagtggtaaacaggtcttcaatgtgctggtttgg Tcccgtgtgatgtcttcatgatcgatgtcttcagcagtgcag gaataatgtttcagttttTctctggccgtcttcaccatgtca tctgtggtcttcagcaggtttcgggactggctaagaCacctg gcaggtccagagactggaatgaccctagatccgccgccaccc gacccaccaccgCccgagccaccgccaccggatcggaccctg cagggaacacatgcccacttgctgcatgagGaattgtaatag cgatcctgagcttgcacgcagacattcccgcctttgcattgg acttcgGtagatgtcttctctacgaggaacgcacctttctgg ttacacccctcctctgtctccttcAtcttttctttcttgcgc tggattcgaacaaagaacttgagggagaagtaggaatgggga Gtgctccaggagtcagggtactcccagctgacctccacctgt gagttcttcaaaggcttcAtctgcaagttcttgggcgggtct ggtttgatgatgtccctgatgaagaagctggtgctgTagttc tcatatttattctgctgccgtgcttccaacgccagttcaatg ggcagggtctccTcggcagttgggcaggtgacatcctcctgg caggacactgaatacttctcatagtcccttTggtccagtgtg accttctctgcagacagagacgccattccacatgtcactgcc cgagagTcaggggaactgctactgctcttgatgttgaacttc aagtccatgtttctttgcaccagcCatgagcacgtgaaccgt ccggagtaatttggtgcttcacacttcaggaaagtcttgttt Ttgaaattttttaaaatttcagtggaccaaattccattttcc ttcttgtggagcagcagaTgtgagtggctcagagtctcgcct cctttgtggcaggtgtactggccagcatctagaaacTctttg acagtgatggtcagggtctttccagagcctatgactccatgt ctctggtctgagGtccaggtgatgtcatcttcttcaggcgtg tcacaggtgaggttcactgtttctccagggGcatcgggagtc cagtccacctctacaacataaacgtctttctccagctcccac atggccAtgagtggagacaccagcaaaacgatggcaaaccag gagatggttagcttctgaggacacAtggtggcgaccggtagc gctaggacgcgtaggacttgaggtcactgtgaggagtgatta Gcaagggtgataggcagctcttcagcatgggaggcaatttat actgttaatgctggaaaaAtaatatgggggtgtcacgatgtt cccgggacaattgaacgccttctgtatgaaacaaatTttcct cttaacgccttctgtatgaaacaaattttcctcttaacgcct tctgtatgaaacAaattttcctcttaacgccttctgtatgaa acaaattttcctcttaacgccttctgtatgaaacaaattttc ctcttaacgccttctgtatgaaacaaattttcctctt |

All documents mentioned in the present invention are cited as references in this application, as if each document is individually cited as a reference. In addition, it should be understood that after reading the above teaching contents of the present invention, a skilled person in the art can make various changes or modifications to the present invention, and these equivalent forms shall also fall within the scope defined by the appended claims of the present application.

## Claims

1. An immune effector cell expressing a receptor and exogenous IL12, wherein the receptor specifically binds to GPC3.

2. The immune effector cell of claim 1, wherein the receptor and the IL12 are operably linked.

3. The immune effector cell of claim 1, wherein the immune effector cell includes: T cell, natural killer cell, cytotoxic T lymphocyte, natural killer T cell, DNT cell, and/or regulatory T cell.

4. The immune effector cell of claim 1, wherein the exogenous IL12 is constitutively or inductively expressed; preferably, the promoter used to express the IL12 includes: an immune cell inducible promoter; and preferably, the immune cell inducible promoter is NFAT6 promoter.

5. The immune effector cell of any one of preceding claims, wherein the receptor has an extracellular domain specifically binding to GPC3, a transmembrane domain, and an intracellular signal domain.

6. The immune effector cell of claim 5, wherein the receptor is a chimeric antigen receptor, wherein the intracellular domain contains a cell stimulating signal molecule or a combination of a cell stimulating signal molecule and a cell activating co-stimulatory molecule;

Preferably, the cell stimulating signal molecule is selected from the functional signaling domain of a protein: CD3$\zeta$, CD3$\gamma$, CD3$\delta$, CD3$\varepsilon$, Fc$\varepsilon$RI$\gamma$, FcR$\beta$, CD79a, CD79b, Fc$\gamma$RIIa, DAP10, or DAP 12; more preferably CD3$\zeta$; or Preferably, the cell activating co-stimulatory molecule is selected from the functional signaling domain of the following proteins: CD27, CD28, CD137, CD134, ICOS, OX40, CD30, CD40, PD-1, lymphocyte function related antigen -1 (LFA-1), CD2, CD7, LIGHT, NKG2C, B7-H3, a ligand that specifically binds to CD83, CDS, ICAM-1, GITR, BAFFR, HVEM (LIGHTR), SLAMF7, NKp80 (KLRF1), CD160 , CD19, CD4, CD8$\alpha$, CD8$\beta$, IL2R$\beta$, IL2R$\gamma$, IL7R$\alpha$, ITGA4, VLA1, CD49a, ITGA4, IA4, CD49D, ITGA6, VLA-6, CD49f, ITGAD, CD11d, ITGAE, CD103, ITGAL, CD11a, LFA-1 , ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, NKp44, NKp30, NKp46, and NKG2D, more preferably, CD27, CD28, CD137, CD134, ICOS.

7. The immune effector cell of claim 5 or 6, wherein the extracellular domain of the receptor contains an amino acid sequence that is at least 90%, 95%, 96%, 97%, 98%, or 99% identical with the amino acid sequence shown in SEQ ID NO: 25.

8. The immune effector cell of any one of preceding claims, wherein the receptor contains the amino acid sequence shown in SEQ ID NO: 21, 22, 23, or 24; or contains an amino acid sequence that is at least 90%, 95%, 96%, 97%, 98%, or 99% identical with the amino acid sequence shown in SEQ ID NO: 21, 22, 23 or 24.

9. The immune effector cell of claim 8, wherein the receptor and the exogenous IL12 are encoded by a nucleotide sequence that is at least 90%, 95%, 96%, 97%, 98%, or 99% identity with SEQ ID NO: 28, 29, 30, 31, 32, 33, 34 or 35;

preferably, encoded by a nucleotide sequence that is at least 90%, 95%, 96%, 97%, 98%, or 99% identity with SEQ ID NO: 28, 29, 30 or 31;
more preferably, encoded by a nucleotide sequence of SEQ ID NO: 28, 29, 30 or 31.

10. The immune effector cell of any one of preceding claims, wherein the immune effector cell does not contain exogenous co-stimulatory ligands.

11. The immune effector cell of any one of preceding claims, wherein the receptor and/or the IL12 are constitutively or inducibly expressed on the surface of the immune effector cell.

12. The immune effector cell of any one of preceding claims, wherein the immune effector cell contains an expression construct, which includes: an expression cassette of the antigen-binding receptor; and an expression cassette of the IL12.

13. The immune effector cell of any one of preceding claims, wherein the receptor and/or IL12 are expressed by using a viral vector; and preferably, the viral vector includes: a lentiviral vector, retroviral vector or adenoviral vector.

14. The immune effector cell of any one of claims 1-13, wherein after the immune effector cell is administered to an individual, the number of CAR-T cells in the peripheral blood of the individual is increased by at least 50%, compared with the case where the exogenous IL12 is not present.

15. The immune effector cell of any one of claims 1-13, wherein about 7 days after the immune effector cell is administered to the individual, the sum of the number of CAR-T cells in the individual's peripheral blood is greater than 6,000/$\mu$L;

and about 10 days after the immune effector cell is administered, the sum of the number of CAR-T cells in the individual's peripheral blood is greater than 6,000/μL.

**16.** An expression construct, including an expression cassette of a receptor and an expression cassette of IL12, which are connected in sequence; wherein the antigen-binding receptor and IL12 are defined as in any one of preceding claims.

**17.** Use of the immune effector cell of any one of claims 1-13 in the preparation of a pharmaceutical composition for treating tumors in an individual in need thereof.

**18.** The use of claim 17, wherein the tumor includes: liver cancer, stomach cancer, lung cancer, breast cancer, head and neck cancer, bladder cancer, ovarian cancer, cervical cancer, kidney cancer, pancreatic cancer, cervical cancer, liposarcoma, melanoma, adrenal carcinoma, Schwannoma, malignant fibrous histiocytoma, esophageal cancer; and preferably, the tumor is liver cancer, gastric cancer, lung cancer, or breast cancer.

**19.** The use of claim 17 or 18, wherein the immune effector cells reduce the tumor by at least 50%.

**20.** The use of claim 19, wherein the immune effector cells reduce the tumor by at least 70%, and more preferably, the immune effector cells reduce the tumor by at least 80%.

**21.** A pharmaceutical composition, comprising:

the immune effector cell of any one of claims 1-13; and
a pharmaceutically acceptable carrier or excipient.

**22.** A kit comprising:

the immune effector cell of any one of claims 1-13; and
instructions on how to administer the immune effector cell to an individual.

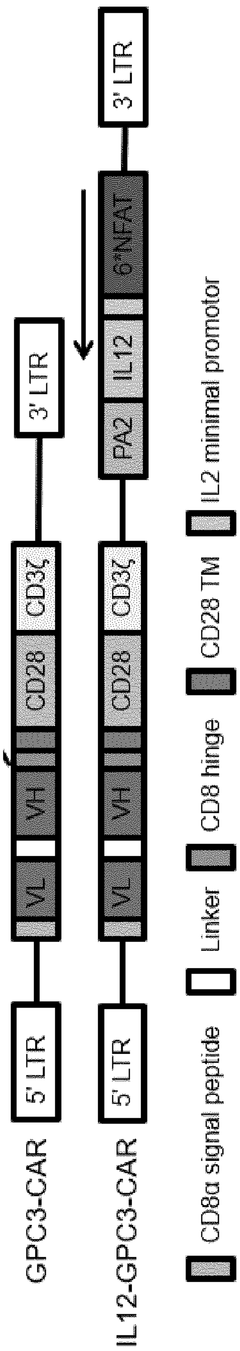
GPC3-CAR
IL12-GPC3-CAR
5' LTR
VL
VH
CD28
CD3ζ
3' LTR
PA2
IL12
6*NFAT
CD8α signal peptide
Linker
CD8 hinge
CD28 TM
IL2 minimal promotor

Fig. 1A

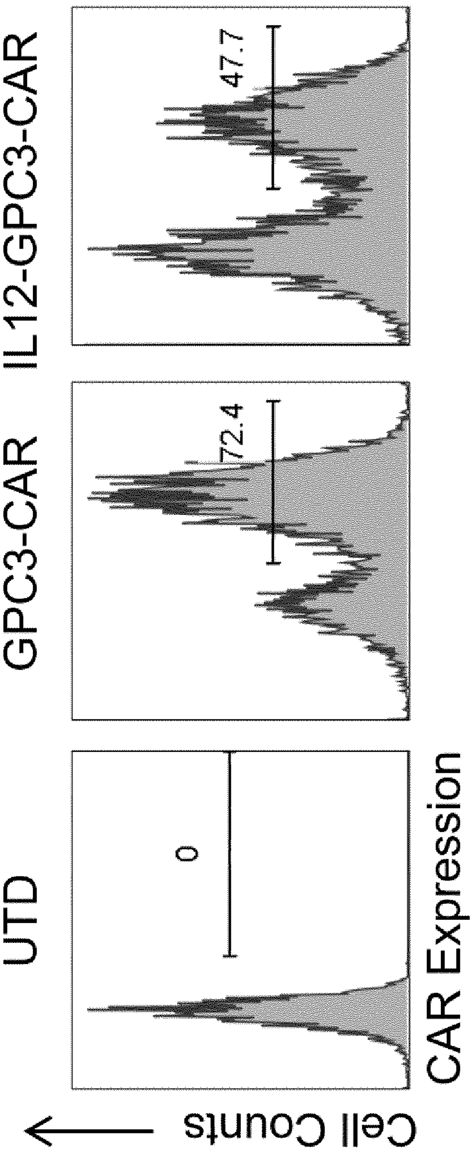
UTD
GPC3-CAR
IL12-GPC3-CAR
0
72.4
47.7
Cell Counts
CAR Expression

Fig. 1B

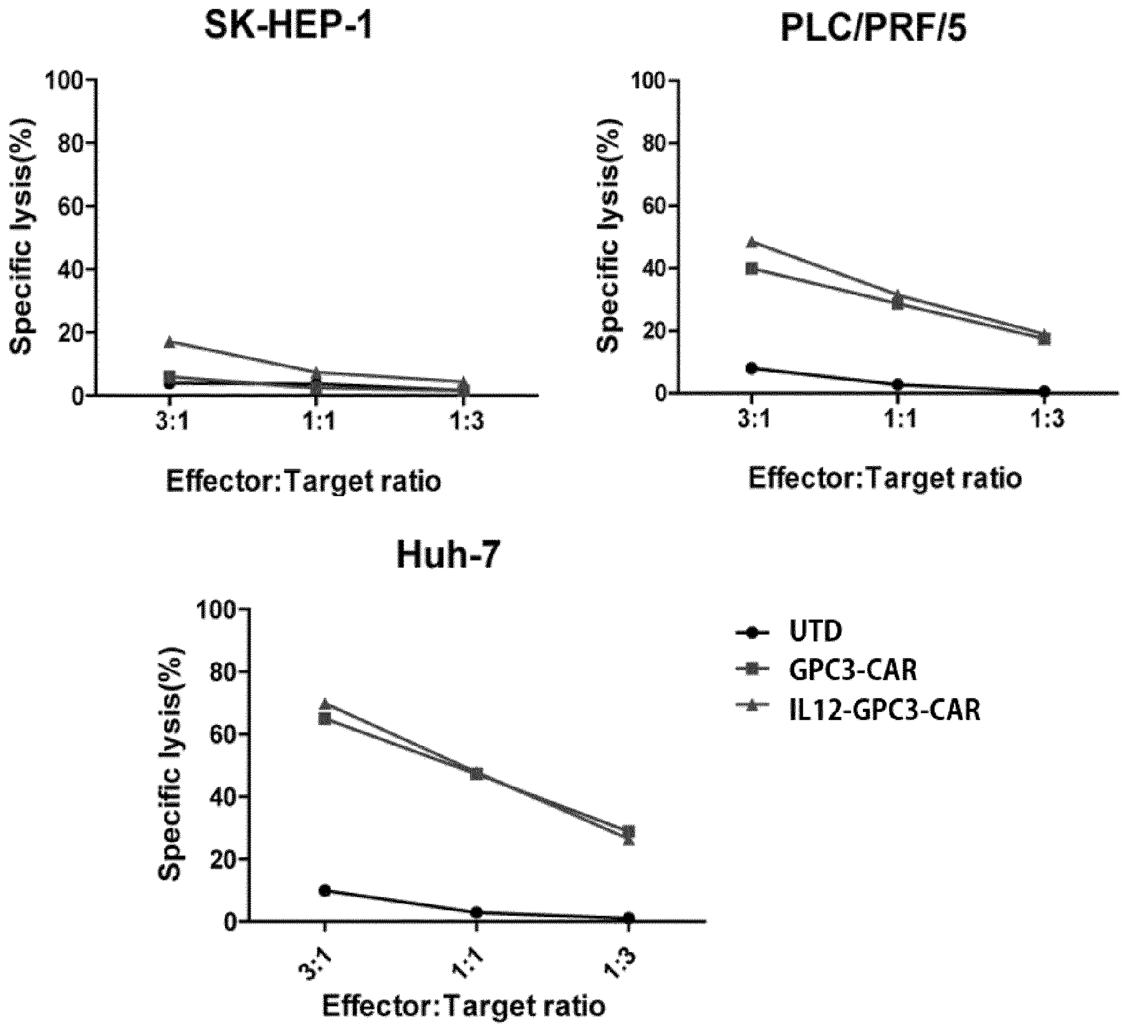
SK-HEP-1
PLC/PRF/5
Huh-7
Specific lysis(%)
100
80
60
40
20
0
3:1
1:1
1:3
Effector:Target ratio
UTD
GPC3-CAR
IL12-GPC3-CAR

Fig. 2

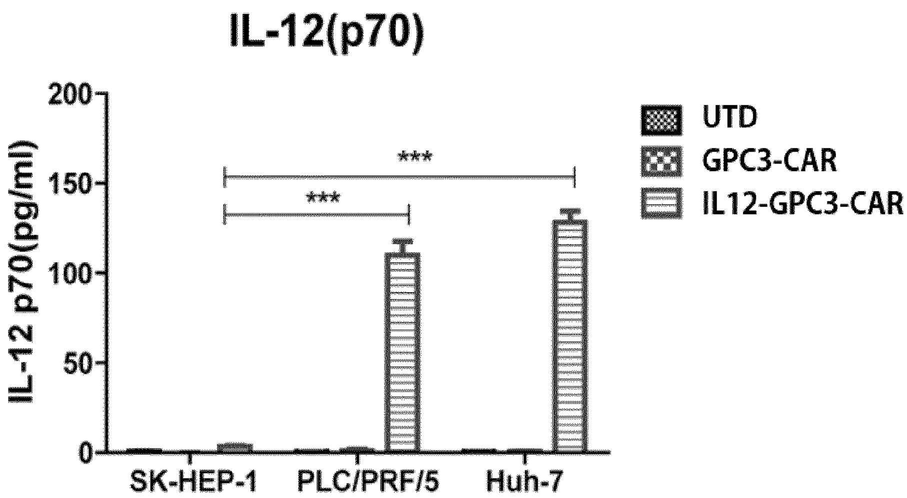
IL-12(p70)
IL-12 p70(pg/ml)
200
150
100
50
0
***
***
SK-HEP-1
PLC/PRF/5
Huh-7
UTD
GPC3-CAR
IL12-GPC3-CAR

Fig. 3A

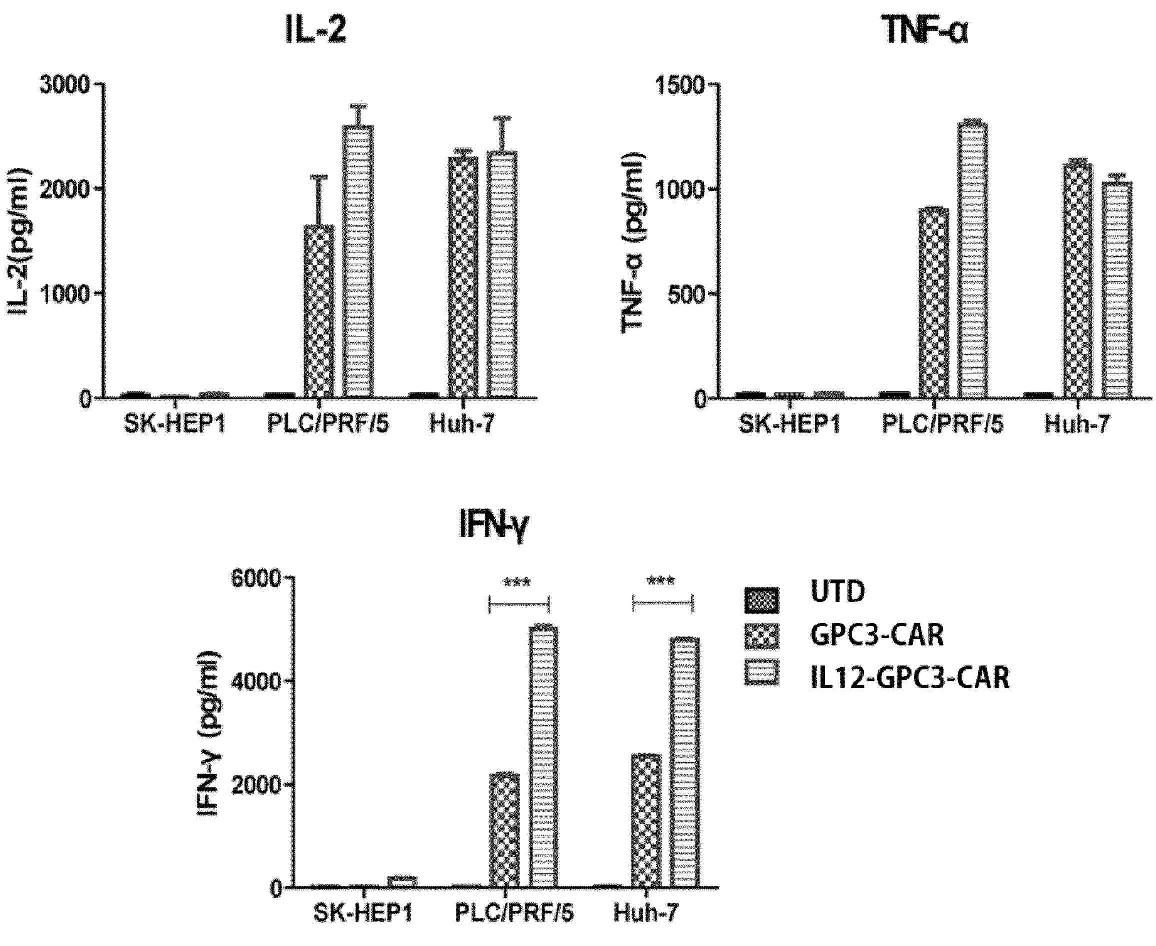
IL-2
IL-2(pg/ml)
3000
2000
1000
0
SK-HEP1
PLC/PRF/5
Huh-7
TNF-α
TNF-α (pg/ml)
1500
1000
500
0
SK-HEP1
PLC/PRF/5
Huh-7
IFN-γ
IFN-γ (pg/ml)
6000
4000
2000
0
***
***
SK-HEP1
PLC/PRF/5
Huh-7
UTD
GPC3-CAR
IL12-GPC3-CAR

Fig. 3B

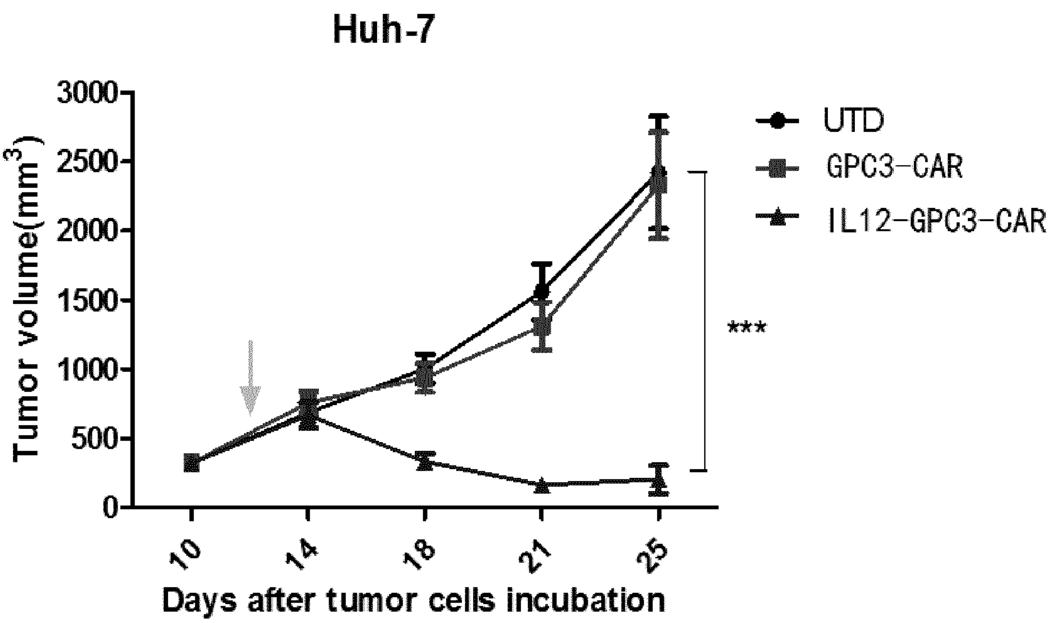
Huh-7
Tumor volume(mm3)
3000
2500
2000
1500
1000
500
0
10
14
18
21
25
Days after tumor cells incubation
UTD
GPC3-CAR
IL12-GPC3-CAR
***

Fig. 4A

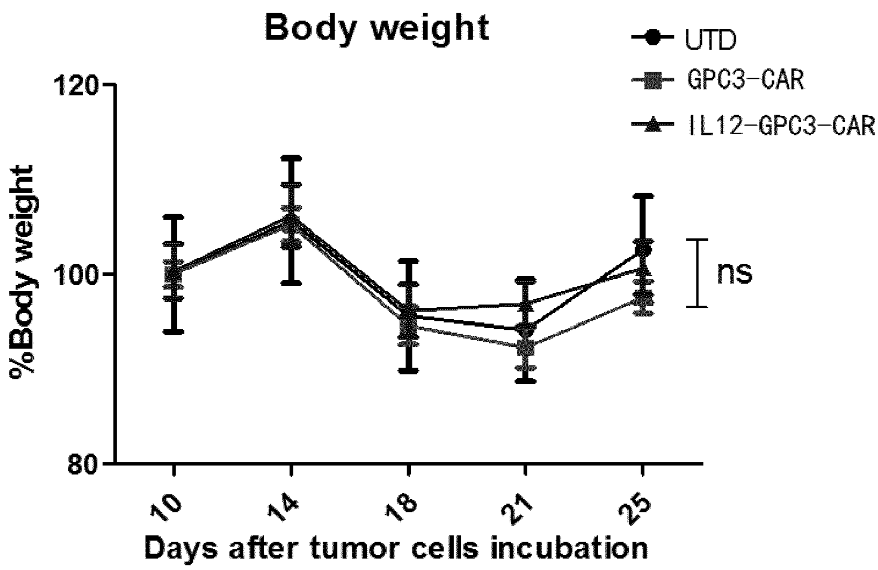


Fig. 4B

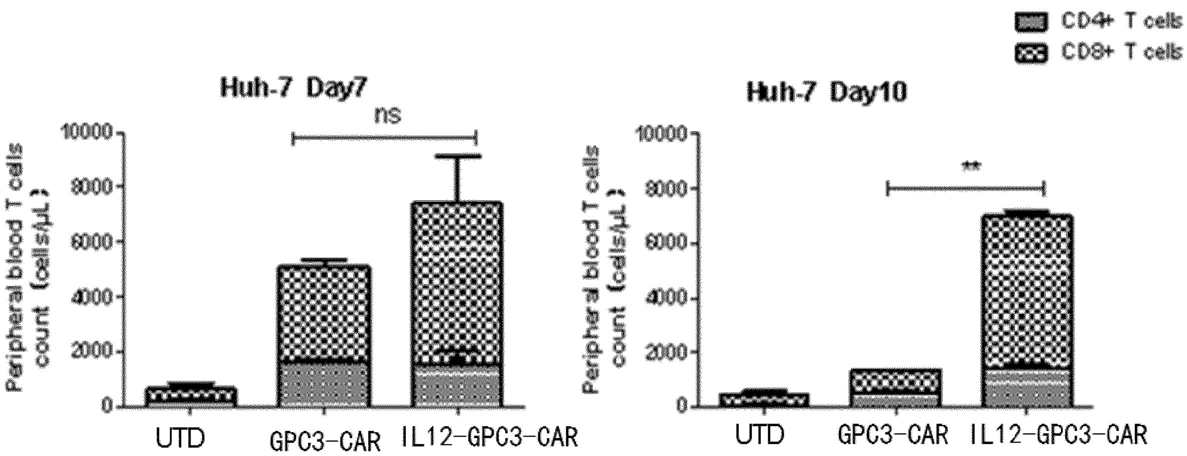


Fig. 5

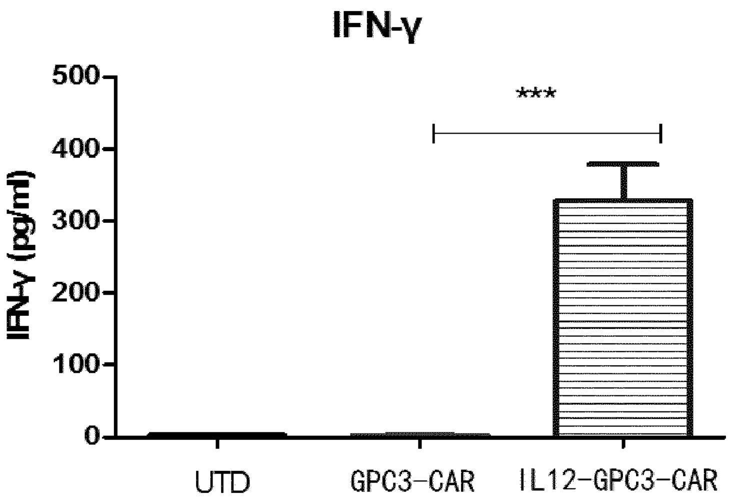


Fig. 6

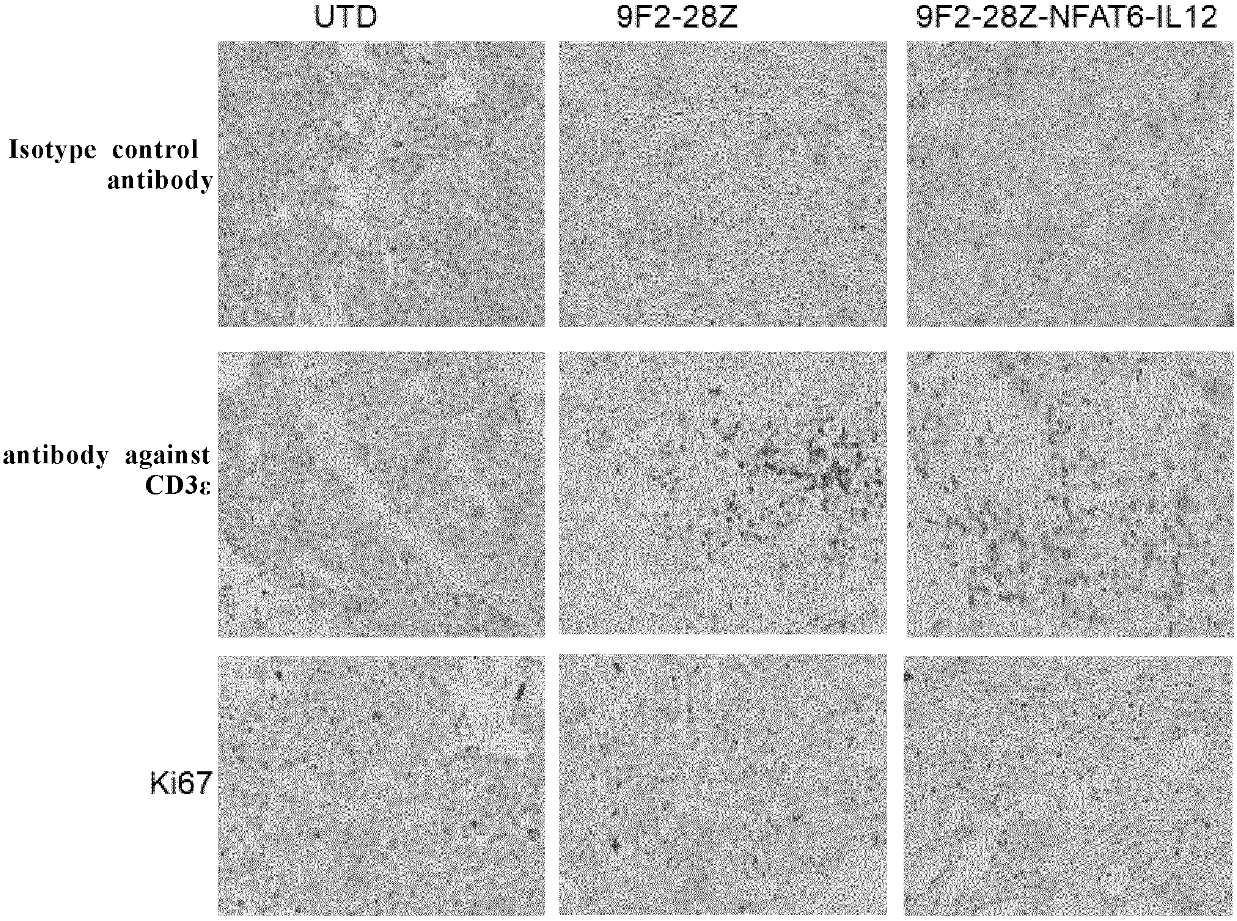
UTD
9F2-28Z
9F2-28Z-NFAT6-IL12
Isotype control antibody
antibody against CD3ε
Ki67

Fig. 7

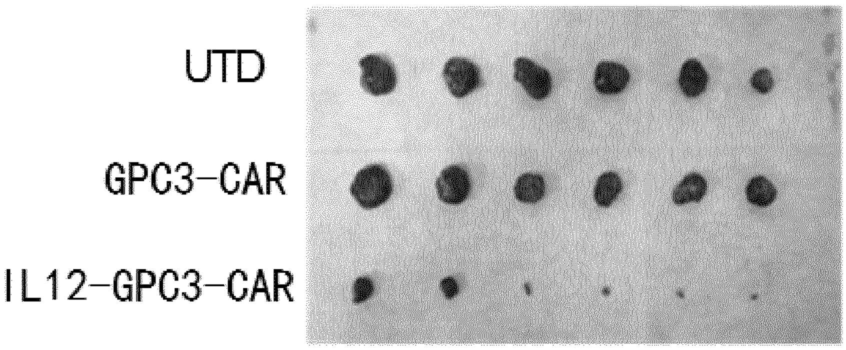
UTD
GPC3-CAR
IL12-GPC3-CAR

Fig. 8A

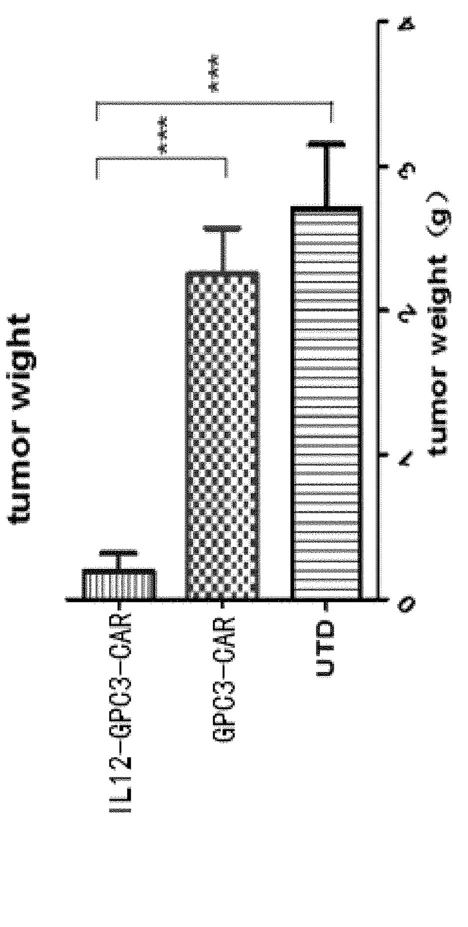
tumor wight
IL12-GPC3-CAR
GPC3-CAR
UTD
***
***
0
1
2
3
4
tumor weight（g）

Fig. 8B

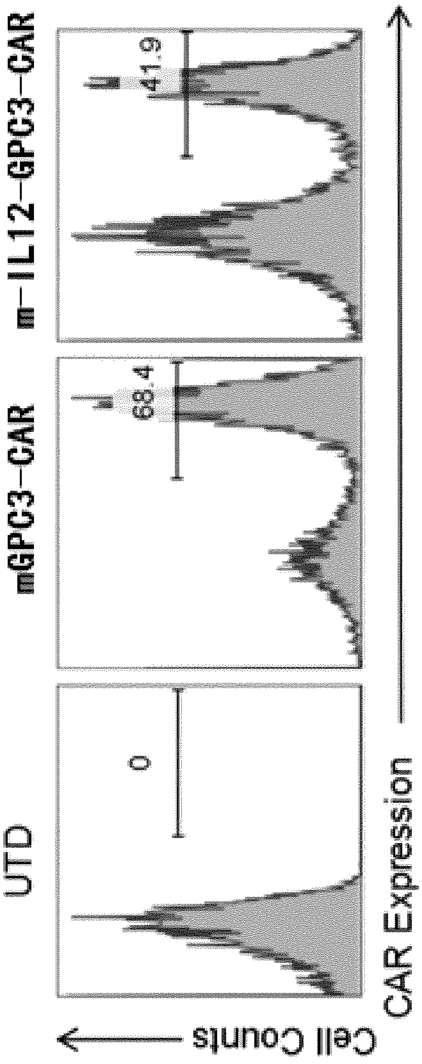
UTD
mGPC3-CAR
m-IL12-GPC3-CAR
0
68.4
41.9
Cell Counts
CAR Expression

Fig. 9

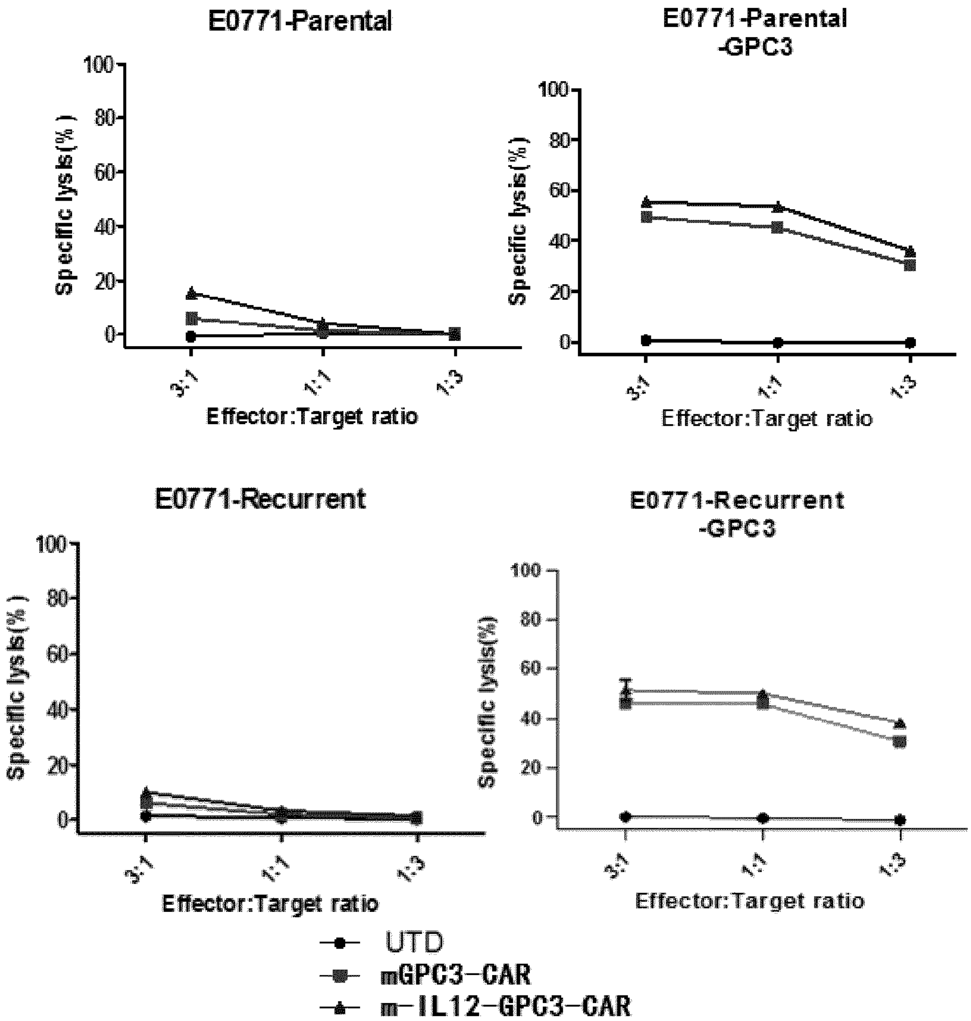


Fig. 10

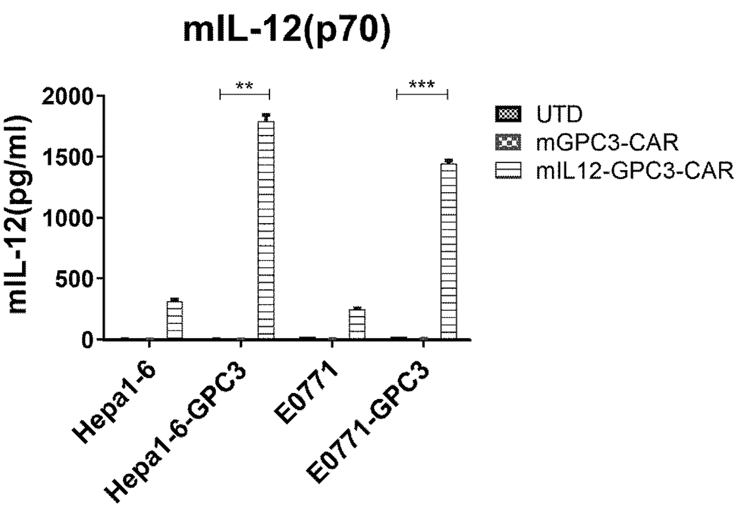


Fig. 11A

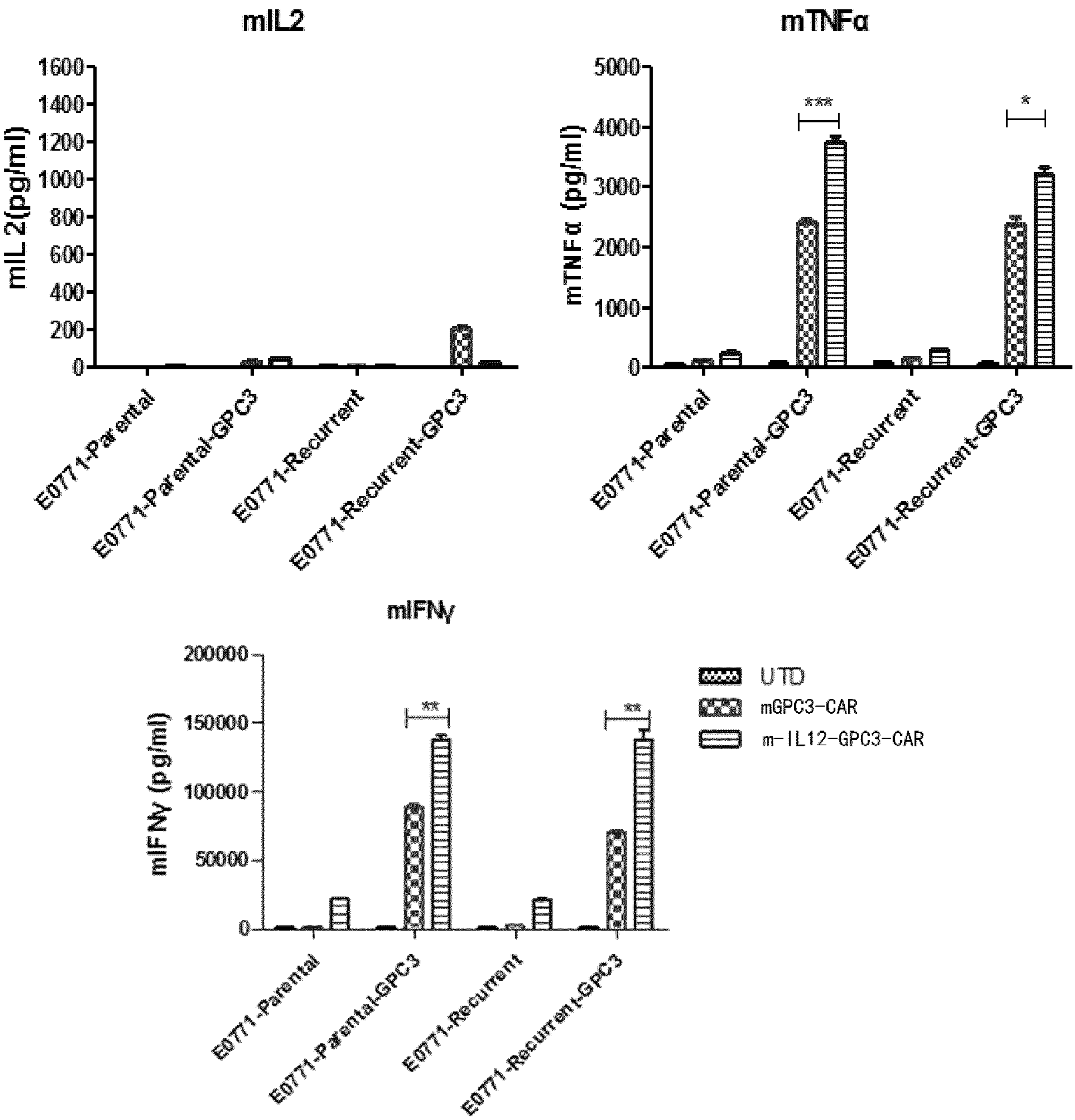
mIL2
mIL 2(pg/ml)
1600
1400
1200
1000
800
600
400
200
0
E0771-Parental
E0771-Parental-GPC3
E0771-Recurrent
E0771-Recurrent-GPC3
mTNFα
mTNFα (pg/ml)
5000
4000
3000
2000
1000
0
***
*
mIFNγ
mIFNγ (pg/ml)
200000
150000
100000
50000
0
**
**
UTD
mGPC3-CAR
m-IL12-GPC3-CAR

Fig. 11B

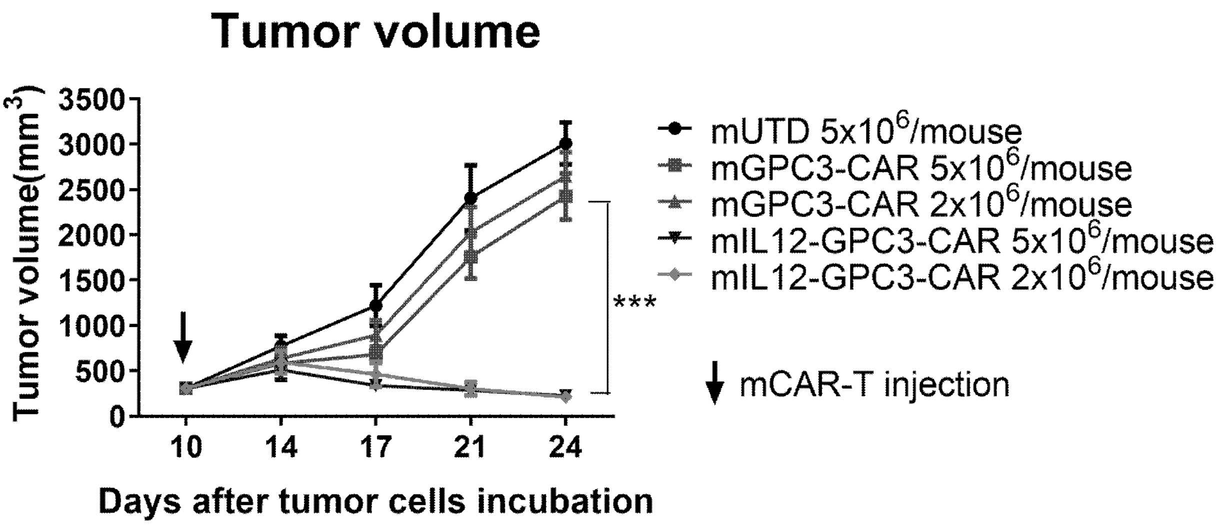


Fig. 12A

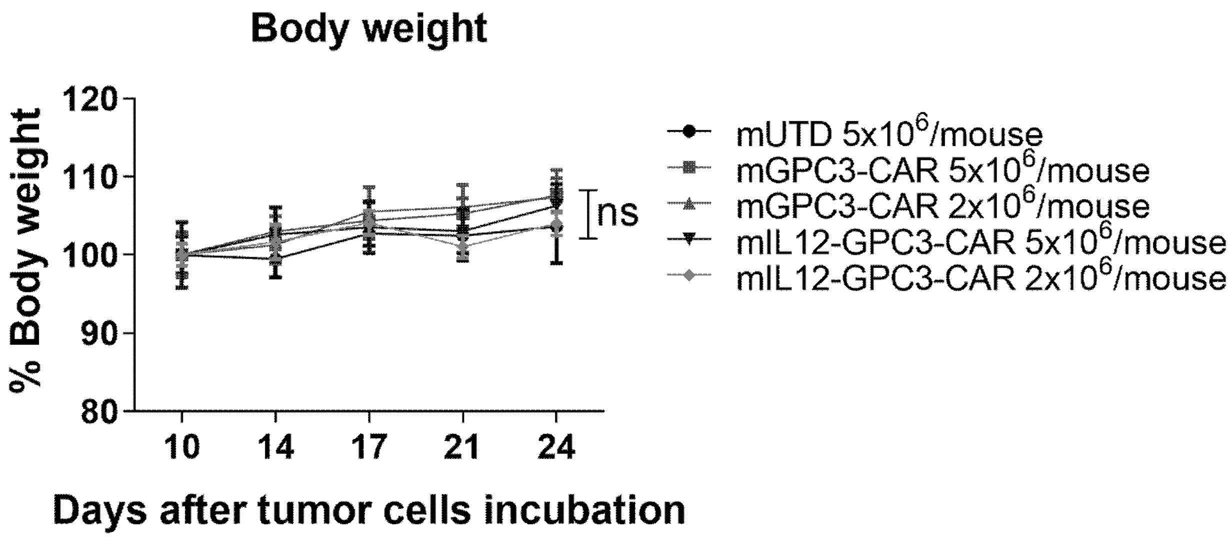


Fig. 12B

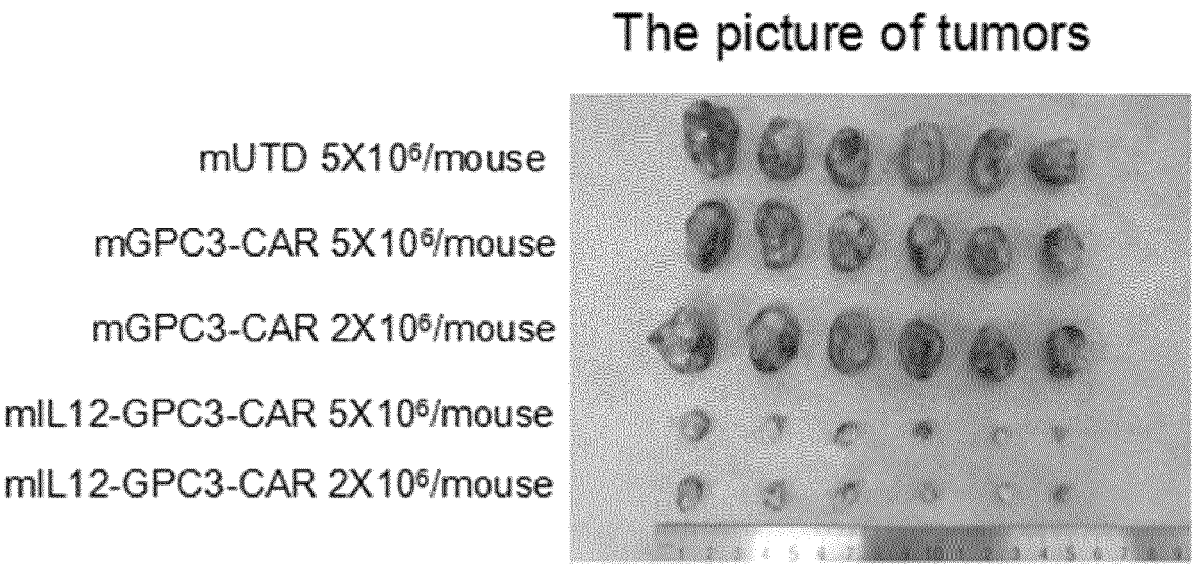


Fig. 13A

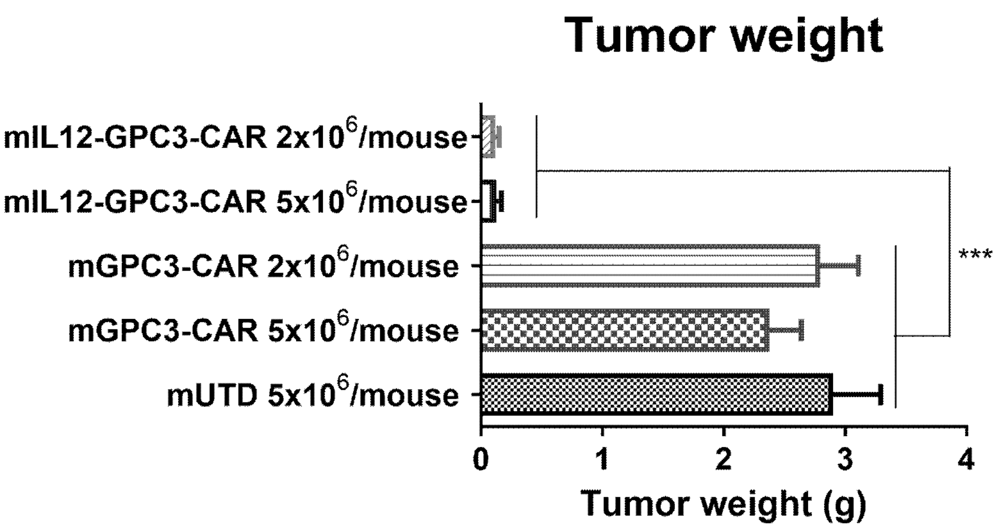


Fig. 13B

## INTERNATIONAL SEARCH REPORT

International application No.
**PCT/CN2018/120938**

**A. CLASSIFICATION OF SUBJECT MATTER**

C12N 5/10(2006.01)i； C12N 15/867(2006.01)i

According to International Patent Classification (IPC) or to both national classification and IPC

**B. FIELDS SEARCHED**

Minimum documentation searched (classification system followed by classification symbols)

C12N

Documentation searched other than minimum documentation to the extent that such documents are included in the fields searched

Electronic data base consulted during the international search (name of data base and, where practicable, search terms used)

CNABS; CNTXT; CJFD; CNKI; DWPI; SIPOABS; WOTXT; USTXT; EPTXT; 万方; WANFANG; 读秀; DUXIU; ISI Web of Science; 中国专利生物序列检索系统; Chinese Patent Biological Sequence Retrieval System; GenBank; EMBL: 科济生物医药, 上海市肿瘤研究所, 李宗海, 蒋华, 嵌合抗原受体, 磷脂酰肌醇蛋白多糖3, 膦脂酰肌醇蛋白多糖3, T 细胞, 白介素2, 乳腺癌, CAR, chimeric antigen receptor, GPC3, Glypican 3, T cell, IL2, interleukin 2, breast cancer, 基于序列21-25, 28-35的检索, search based on SEQs 21-25, 28-35

**C. DOCUMENTS CONSIDERED TO BE RELEVANT**

| Category* | Citation of document, with indication, where appropriate, of the relevant passages | Relevant to claim No. |
|---|---|---|
| X | 刘莹 等 (LIU, Ying et al.). "可诱导表达IL-12的GPC3靶向性CAR-T细胞在免疫健全小鼠中的抗乳腺癌作用 (Anti-breast Cancer Activity of GPC3-specific CAR-T Cells Inducing Interleukin-12 Expression in Immunocompetent Mice)" *肿瘤 (Tumor),* Vol. 38, No. 7, 31 July 2018 (2018-07-31), pp. 631-642 | 1-22 |
| X | CN 106397593 A (CARSGEN THERAPEUTICS (SHANGHAI) CO., LTD.) 15 February 2017 (2017-02-15) claims 1-37 | 1-22 |
| E | CN 109468279 A (CARSGEN THERAPEUTICS (SHANGHAI) CO., LTD. et al.) 15 March 2019 (2019-03-15) claims 1-22 | 1-22 |
| A | CN 108884459 A (CARSGEN THERAPEUTICS (SHANGHAI) CO., LTD. et al.) 23 November 2018 (2018-11-23) entire document | 1-22 |

☐ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **05 May 2019** | **21 June 2019** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2018/120938**

**Box No. I** **Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/CN2018/120938**

| Patent document cited in search report | | | Publication date (day/month/year) | Patent family member(s) | | | Publication date (day/month/year) |
|---|---|---|---|---|---|---|---|
| CN | 106397593 | A | 15 February 2017 | KR | 20180054590 | A | 24 May 2018 |
| | | | | JP | 2018522564 | A | 16 August 2018 |
| | | | | US | 2019046659 | A1 | 14 February 2019 |
| | | | | BR | 112018002386 | A2 | 18 September 2018 |
| | | | | EP | 3333192 | A4 | 27 March 2019 |
| | | | | SG | 11201800989R | A | 28 March 2018 |
| | | | | EP | 3333192 | A1 | 13 June 2018 |
| | | | | WO | 2017020812 | A1 | 09 February 2017 |
| | | | | AU | 2016304060 | A1 | 29 March 2018 |
| | | | | CA | 2994751 | A1 | 09 February 2017 |
| | | | | HK | 1251587 | A0 | 01 February 2019 |
| CN | 109468279 | A | 15 March 2019 | CN | 109468278 | A | 15 March 2019 |
| | | | | WO | 2019047932 | A1 | 14 March 2019 |
| CN | 108884459 | A | 23 November 2018 | WO | 2017186121 | A1 | 02 November 2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HOOIJBERG E ; BAKKER AQ ; RUIZENDAAL JJ ; SPITS H.** NFAT-controlled expression of GFP permits visualization and Isolation of antigen-stimulated primary human Tcells. *Blood,* 15 July 2000, vol. 96 (2), 459-66
- **ZHANG L ; KERKAR SP ; YU Z ; ZHENG Z ; YANG S ; RESTIFONP ; ROSENBERG SA ; MORGAN RA.** Improving adoptive T cell therapy by targeting and controlling IL-12 expression to the tumor environment. *Mol Ther.,* April 2011, vol. 19 (4), 751-9

## Sequence listing

<110> CARSGEN THERAPEUTICS CO., LTD.
SHANGHAI CANCER INSTITUTE

<120> IMMUNE EFFECTOR CELL TARGETING GPC3 AND APPLICATION THEREOF

<130> P2018-2398

<160> 35

<170> PatentIn version 3.5

<210> 1
<211> 729
<212> DNA
<213> Artificial sequence

<220>
<223> Synthesized polynucleotide

<400> 1
```
gaggtgcagc tggtgcagag cggcgccgag gtgaagaagc ccggcgccag cgtgaaggtg      60

agctgcaagg ccagcggcta caccttcagc gactacgaga tgcactgggt gcggcaggcc     120

cccggccagg gcctggagtg gatgggcgcc atccaccccg gcagcggcga caccgcctac     180

aaccagcggt tcaagggccg ggtgaccatc accgccgaca agagcaccag caccgcctac     240

atggagctga gcagcctgcg gagcgaggac accgccgtgt actactgcgc ccggttctac     300

agctacgcct actggggcca gggcaccctg gtgaccgtga gcgccggtgg aggcggttca     360

ggcggaggtg gttctggcgg tggcggatcg gacatcgtga tgacccagac cccccctgagc     420

ctgcccgtga cccccggcga gcccgccagc atcagctgcc ggagcagcca gagcctggtg     480

cacagcaacg gcaacaccta cctgcagtgg tacctgcaga agcccggcca gagcccccag     540

ctgctgatct acaaggtgag caaccggttc agcggcgtgc ccgaccggtt cagcggcagc     600

ggcagcggca ccgacttcac cctgaagatc agccgggtgg aggccgagga cgtgggcgtg     660

tactactgca gccagagcat ctacgtgccc tacaccttcg gccagggcac caagctggag     720

atcaaacgt                                                             729
```

<210> 2
<211> 63
<212> DNA
<213> Artificial sequence

<220>
<223> Synthesized polynucleotide

<400> 2
```
atggccttac cagtgaccgc cttgctcctg ccgctggcct tgctgctcca cgccgccagg      60

ccg                                                                    63
```

<210> 3
<211> 135
<212> DNA
<213> Artificial sequence

<220>
<223> Synthesized polynucleotide

<400> 3
accacgacgc cagcgccgcg accaccaaca ccggcgccca ccatcgcgtc gcagcccctg 60

tccctgcgcc cagaggcgtg ccggccagcg gcggggggcg cagtgcacac gagggggctg 120

gacttcgcct gtgat 135

<210> 4
<211> 123
<212> DNA
<213> Artificial sequence

<220>
<223> Synthesized polynucleotide

<400> 4
aggagtaaga ggagcaggct cctgcacagt gactacatga acatgactcc ccgccgcccc 60

gggccaaccc gcaagcatta ccagccctat gccccaccac gcgacttcgc agcctatcgc 120

tcc 123

<210> 5
<211> 339
<212> DNA
<213> Artificial sequence

<220>
<223> Synthesized polynucleotide

<400> 5
agagtgaagt tcagcaggag cgcagacgcc cccgcgtacc agcagggcca gaaccagctc 60

tataacgagc tcaatctagg acgaagagag gagtacgatg ttttggacaa gagacgtggc 120

cgggaccctg agatgggggg aaagccgcag agaaggaaga accctcagga aggcctgtac 180

aatgaactgc agaaagataa gatggcggag gcctacagtg agattgggat gaaaggcgag 240

cgccggaggg gcaaggggca cgatggcctt taccagggtc tcagtacagc caccaaggac 300

acctacgacg cccttcacat gcaggccctg ccccctcgc 339

<210> 6
<211> 81
<212> DNA
<213> Artificial sequence

<220>
<223> Synthesized polynucleotide

<400> 6

ttttgggtgc tggtggtggt tggtggagtc ctggcttgct atagcttgct agtaacagtg 60

gcctttatta ttttctgggt g 81

<210> 7
<211> 194
<212> DNA
<213> Artificial sequence

<220>
<223> Synthesized polynucleotide

<400> 7
ggaggaaaaa ctgtttcata cagaaggcgt ggaggaaaaa ctgtttcata cagaaggcgt 60

ggaggaaaaa ctgtttcata cagaaggcgt caattgtcct cgacggagga aaaactgttt 120

catacagaag gcgtggagga aaaactgttt catacagaag gcgtggagga aaaactgttt 180

catacagaag gcgt 194

<210> 8
<211> 114
<212> DNA
<213> Artificial sequence

<220>
<223> Synthesized polynucleotide

<400> 8
acattttgac acccccataa tatttttcca gaattaacag tataaattgc atctcttgtt 60

caagagttcc ctatcactct ctttaatcac tactcacagt aacctcaact cctg 114

<210> 9
<211> 45
<212> DNA
<213> Artificial sequence

<220>
<223> Synthesized polynucleotide

<400> 9
ggtggaggcg gttcaggcgg aggtggttct ggcggtggcg gatcg 45

<210> 10
<211> 984
<212> DNA
<213> Artificial sequence

<220>
<223> Synthesized polynucleotide

<400> 10
atgtgtcacc agcagttggt catctcttgg ttttccctgg tttttctggc atctcccctc 60

gtggccatat gggaactgaa gaaagatgtt tatgtcgtag aattggattg gtatccggat 120

gcccctggag aaatggtggt cctcacctgt gacacccctg aagaagatgg tatcacctgg 180

acccttggacc agagcagtga ggtcttaggc tctggcaaaa ccctgaccat ccaagtcaaa 240

gagtttggag atgctggcca gtacacctgt cacaaaggag gcgaggttct aagccattcg 300

ctcctgctgc ttcacaaaaa ggaagatgga atttggtcca ctgatatttt aaaggaccag 360

aaagaaccca aaaataagac ctttctaaga tgcgaggcca agaattattc tggacgtttc 420

acctgctggt ggctgacgac aatcagtact gatttgacat tcagtgtcaa aagcagcaga 480

ggctcttctg acccccaagg ggtgacgtgc ggagctgcta cactctctgc agagagagtc 540

agaggggaca acaaggagta tgagtactca gtggagtgcc aggaggacag tgcctgccca 600

gctgctgagg agagtctgcc cattgaggtc atggtggatg ccgttcacaa gctcaagtat 660

gaaaactaca ccagcagctt cttcatcagg gacatcatca aacctgaccc acccaagaac 720

ttgcagctga agccattaaa gaattctcgg caggtggagg tcagctggga gtaccctgac 780

acctggagta ctccacattc ctacttctcc ctgacattct gcgttcaggt ccagggcaag 840

agcaagagag aaaagaaaga tagagtcttc acggacaaga cctcagccac ggtcatctgc 900

cgcaaaaatg ccagcattag cgtgcgggcc caggaccgct actatagctc atcttggagc 960

gaatgggcat ctgtgccctg cagt 984

<210> 11
<211> 594
<212> DNA
<213> Artificial sequence

<220>
<223> Synthesized polynucleotide

<400> 11
agaaacctcc ccgtggccac tccagaccca ggaatgttcc catgccttca ccactcccaa 60

aacctgctga gggccgtcag caacatgctc cagaaggcca gacaaactct agaattttac 120

ccttgcactt ctgaagagat tgatcatgaa gatatcacaa aagataaaac cagcacagtg 180

gaggcctgtt taccattgga attaaccaag aatgagagtt gcctaaattc cagagagacc 240

tctttcataa ctaatgggag ttgcctggcc tccagaaaga cctcttttat gatggccctg 300

tgccttagta gtatttatga agacttgaag atgtaccagg tggagttcaa gaccatgaat 360

gcaaagcttc tgatggatcc taagaggcag atctttctag atcaaaacat gctggcagtt 420

attgatgagc tgatgcaggc cctgaatttc aacagtgaga ctgtgccaca aaaatcctcc 480

cttgaagaac cggattttta taaaactaaa atcaagctct gcatacttct tcatgctttc 540

agaattcggg cagtgactat tgatagagtg atgagctatc tgaatgcttc ctaa 594

<210> 12
<211> 81
<212> DNA

<213> Artificial sequence

<220>
<223> Synthesized polynucleotide

<400> 12
atggcctcac cgttgacccg ctttctgtcg ctgaacctgc tgctgctggg tgagtcgatt 60

atcctgggga gtggagaagc t 81

<210> 13
<211> 216
<212> DNA
<213> Artificial sequence

<220>
<223> Synthesized polynucleotide

<400> 13
actactacca agccagtgct gcgaactccc tcacctgtgc accctaccgg gacatctcag 60

ccccagagac cagaagattg tcggccccgt ggctcagtga aggggaccgg attggacttc 120

gcctgtgata tttacatctg ggcacccttg gccggaatct gcgtggccct tctgctgtcc 180

ttgatcatca ctctcatctg ctaccacagg agccga 216

<210> 14
<211> 123
<212> DNA
<213> Artificial sequence

<220>
<223> Synthesized polynucleotide

<400> 14
aatagtagaa ggaacagact ccttcaaagt gactacatga acatgactcc ccggaggcct 60

gggctcactc gaaagcctta ccagccctac gcccctgcca gagactttgc agcgtaccgc 120

ccc 123

<210> 15
<211> 321
<212> DNA
<213> Artificial sequence

<220>
<223> Synthesized polynucleotide

<400> 15
agcaggagtg cagagactgc tgccaacctg caggacccca accagctcta caatgagctc 60

aatctagggc gaagagagga atatgacgtc ttggagaaga agcgggctcg ggatccagag 120

atgggaggca aacagcagag gaggaggaac cccccaggaag gcgtatacaa tgcactgcag 180

aaagacaaga tggcagaagc ctacagtgag atcggcacaa aaggcgagag gcggagaggc 240

aaggggcacg atggccttta ccagggtctc agcactgcca ccaaggacac ctatgatgcc 300

ctgcatatgc agaccctggc c 321

<210> 16
<211> 192
<212> DNA
<213> Artificial sequence

<220>
<223> Synthesized polynucleotide

<400> 16
aagaggaaaa tttgtttcat acagaaggcg ttaagaggaa aatttgtttc atacagaagg 60

cgttaagagg aaaatttgtt tcatacagaa ggcgttaaga ggaaaatttg tttcatacag 120

aaggcgttaa gaggaaaatt tgtttcatac agaaggcgtt aagaggaaaa tttgtttcat 180

acagaaggcg tt 192

<210> 17
<211> 114
<212> DNA
<213> Artificial sequence

<220>
<223> Synthesized polynucleotide

<400> 17
aacatcgtga cacccccata ttatttttcc agcattaaca gtataaattg cctcccatgc 60

tgaagagctg cctatcaccc ttgctaatca ctcctcacag tgacctcaag tcct 114

<210> 18
<211> 1005
<212> DNA
<213> Artificial sequence

<220>
<223> Synthesized polynucleotide

<400> 18
atgtgtcctc agaagctaac catctcctgg tttgccatcg ttttgctggt gtctccactc 60

atggccatgt gggagctgga gaaagacgtt tatgttgtag aggtggactg gactcccgat 120

gcccctggag aaacagtgaa cctcacctgt gacacgcctg aagaagatga catcacctgg 180

acctcagacc agagacatgg agtcataggc tctggaaaga ccctgaccat cactgtcaaa 240

gagtttctag atgctggcca gtacacctgc cacaaaggag gcgagactct gagccactca 300

catctgctgc tccacaagaa ggaaaatgga atttggtcca ctgaaatttt aaaaaatttc 360

aaaaacaaga ctttcctgaa gtgtgaagca ccaaattact ccggacggtt cacgtgctca 420

tggctggtgc aaagaaacat ggacttgaag ttcaacatca agagcagtag cagttcccct 480

gactctcggg cagtgacatg tggaatggcg tctctgtctg cagagaaggt cacactggac 540

```
caaagggact atgagaagta ttcagtgtcc tgccaggagg atgtcacctg cccaactgcc      600

gaggagaccc tgcccattga actggcgttg gaagcacggc agcagaataa atatgagaac      660

tacagcacca gcttcttcat cagggacatc atcaaaccag acccgcccaa gaacttgcag      720

atgaagcctt tgaagaactc acaggtggag gtcagctggg agtaccctga ctcctggagc      780

actccccatt cctacttctc cctcaagttc tttgttcgaa tccagcgcaa gaaagaaaag      840

atgaaggaga cagaggaggg gtgtaaccag aaaggtgcgt tcctcgtaga gaagacatct      900

accgaagtcc aatgcaaagg cgggaatgtc tgcgtgcaag ctcaggatcg ctattacaat      960

tcctcatgca gcaagtgggc atgtgttccc tgcagggtcc gatcc                     1005
```

<210> 19
<211> 579
<212> DNA
<213> Artificial sequence

<220>
<223> Synthesized polynucleotide

<400> 19

```
agggtcattc cagtctctgg acctgccagg tgtcttagcc agtcccgaaa cctgctgaag       60

accacagatg acatggtgaa gacggccaga gaaaaactga aacattattc ctgcactgct      120

gaagacatcg atcatgaaga catcacacgg gaccaaacca gcacattgaa gacctgttta      180

ccactggaac tacacaagaa cgagagttgc ctggctacta gagagacttc ttccacaaca      240

agagggagct gcctgccccc acagaagacg tctttgatga tgaccctgtg ccttggtagc      300

atctatgagg acttgaagat gtaccagaca gagttccagg ccatcaacgc agcacttcag      360

aatcacaacc atcagcagat cattctagac aagggcatgc tggtggccat cgatgagctg      420

atgcagtctc tgaatcataa tggcgagact ctgcgccaga aacctcctgt gggagaagca      480

gacccttaca gagtgaaaat gaagctctgc atcctgcttc acgccttcag cacccgcgtc      540

gtgaccatca acagggtgat gggctatctg agctccgcc                             579
```

<210> 20
<211> 51
<212> DNA
<213> Artificial sequence

<220>
<223> Synthesized polynucleotide

<400> 20

```
aataaaatat ctttattttc attacatctg tgtgttggtt ttttgtgtga g                51
```

<210> 21
<211> 469
<212> PRT
<213> Artificial sequence

<220>
<223>  Synthesized polypeptide

<400>  21

```
Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
```

```
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Arg Val Lys Phe Ser Arg Ser Ala Asp Ala Pro Ala
        355                 360                 365

Tyr Gln Gln Gly Gln Asn Gln Leu Tyr Asn Glu Leu Asn Leu Gly Arg
    370                 375                 380

Arg Glu Glu Tyr Asp Val Leu Asp Lys Arg Arg Gly Arg Asp Pro Glu
385                 390                 395                 400

Met Gly Gly Lys Pro Gln Arg Arg Lys Asn Pro Gln Glu Gly Leu Tyr
                405                 410                 415

Asn Glu Leu Gln Lys Asp Lys Met Ala Glu Ala Tyr Ser Glu Ile Gly
            420                 425                 430

Met Lys Gly Glu Arg Arg Arg Gly Lys Gly His Asp Gly Leu Tyr Gln
        435                 440                 445

Gly Leu Ser Thr Ala Thr Lys Asp Thr Tyr Asp Ala Leu His Met Gln
    450                 455                 460

Ala Leu Pro Pro Arg
465
```

<210> 22
<211> 467
<212> PRT
<213> Artificial sequence

<220>
<223> Synthesized polypeptide

<400> 22

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val
145                 150                 155                 160

His Ser Asn Gly Asn Thr Tyr Leu Gln Trp Tyr Leu Gln Lys Pro Gly
                165                 170                 175

Gln Ser Pro Gln Leu Leu Ile Tyr Lys Val Ser Asn Arg Phe Ser Gly
            180                 185                 190

Val Pro Asp Arg Phe Ser Gly Ser Gly Ser Gly Thr Asp Phe Thr Leu
        195                 200                 205

Lys Ile Ser Arg Val Glu Ala Glu Asp Val Gly Val Tyr Tyr Cys Ser

```
    210                 215                 220

Gln Ser Ile Tyr Val Pro Tyr Thr Phe Gly Gln Gly Thr Lys Leu Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Ile Tyr Ile Trp Ala Pro Leu Ala Gly Thr Cys Gly Val Leu Leu Leu
    290                 295                 300

Ser Leu Val Ile Thr Leu Tyr Cys Lys Arg Gly Arg Lys Lys Leu Leu
305                 310                 315                 320

Tyr Ile Phe Lys Gln Pro Phe Met Arg Pro Val Gln Thr Thr Gln Glu
                325                 330                 335

Glu Asp Gly Cys Ser Cys Arg Phe Pro Glu Glu Glu Glu Gly Gly Cys
            340                 345                 350

Glu Leu Arg Val Lys Phe Ser Arg Ser Ala Asp Ala Pro Ala Tyr Gln
        355                 360                 365

Gln Gly Gln Asn Gln Leu Tyr Asn Glu Leu Asn Leu Gly Arg Arg Glu
    370                 375                 380

Glu Tyr Asp Val Leu Asp Lys Arg Arg Gly Arg Asp Pro Glu Met Gly
385                 390                 395                 400

Gly Lys Pro Gln Arg Arg Lys Asn Pro Gln Glu Gly Leu Tyr Asn Glu
                405                 410                 415

Leu Gln Lys Asp Lys Met Ala Glu Ala Tyr Ser Glu Ile Gly Met Lys
            420                 425                 430

Gly Glu Arg Arg Arg Gly Lys Gly His Asp Gly Leu Tyr Gln Gly Leu
        435                 440                 445

Ser Thr Ala Thr Lys Asp Thr Tyr Asp Ala Leu His Met Gln Ala Leu
    450                 455                 460
```

Pro Pro Arg
465

<210> 23
<211> 511
<212> PRT
<213> Artificial sequence

<220>
<223> Synthesized polypeptide

<400> 23

Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Ser Asp Tyr
20 25 30

Glu Met His Trp Val Arg Gln Ala Pro Gly Gln Gly Leu Glu Trp Met
35 40 45

Gly Ala Ile His Pro Gly Ser Gly Asp Thr Ala Tyr Asn Gln Arg Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Phe Tyr Ser Tyr Ala Tyr Trp Gly Gln Gly Thr Leu Val Thr
100 105 110

Val Ser Ala Gly Gly Gly Gly Ser Gly Gly Gly Gly Ser Gly Gly Gly
115 120 125

Gly Ser Asp Ile Val Met Thr Gln Thr Pro Leu Ser Leu Pro Val Thr
130 135 140

Pro Gly Glu Pro Ala Ser Ile Ser Cys Arg Ser Ser Gln Ser Leu Val
145 150 155 160

His Ser Asn Gly Asn Thr Tyr Leu Gln Trp Tyr Leu Gln Lys Pro Gly
165 170 175

Gln Ser Pro Gln Leu Leu Ile Tyr Lys Val Ser Asn Arg Phe Ser Gly
180 185 190

Val Pro Asp Arg Phe Ser Gly Ser Gly Ser Gly Thr Asp Phe Thr Leu

```
        195                 200                 205

Lys Ile Ser Arg Val Glu Ala Glu Asp Val Gly Val Tyr Tyr Cys Ser
    210                 215                 220

Gln Ser Ile Tyr Val Pro Tyr Thr Phe Gly Gln Gly Thr Lys Leu Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Lys Arg Gly Arg Lys Lys Leu Leu Tyr Ile Phe Lys
        355                 360                 365

Gln Pro Phe Met Arg Pro Val Gln Thr Thr Gln Glu Glu Asp Gly Cys
    370                 375                 380

Ser Cys Arg Phe Pro Glu Glu Glu Glu Gly Gly Cys Glu Leu Arg Val
385                 390                 395                 400

Lys Phe Ser Arg Ser Ala Asp Ala Pro Ala Tyr Gln Gln Gly Gln Asn
                405                 410                 415

Gln Leu Tyr Asn Glu Leu Asn Leu Gly Arg Arg Glu Glu Tyr Asp Val
            420                 425                 430

Leu Asp Lys Arg Arg Gly Arg Asp Pro Glu Met Gly Gly Lys Pro Gln
        435                 440                 445
```

Arg Arg Lys Asn Pro Gln Glu Gly Leu Tyr Asn Glu Leu Gln Lys Asp
    450                 455                 460

Lys Met Ala Glu Ala Tyr Ser Glu Ile Gly Met Lys Gly Glu Arg Arg
465                 470                 475                 480

Arg Gly Lys Gly His Asp Gly Leu Tyr Gln Gly Leu Ser Thr Ala Thr
                485                 490                 495

Lys Asp Thr Tyr Asp Ala Leu His Met Gln Ala Leu Pro Pro Arg
            500                 505                 510

<210> 24
<211> 356
<212> PRT
<213> Artificial sequence

<220>
<223> Synthesized polypeptide

<400> 24

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Ser Asp Tyr
            20                  25                  30

Glu Met His Trp Val Arg Gln Ala Pro Gly Gln Gly Leu Glu Trp Met
        35                  40                  45

Gly Ala Ile His Pro Gly Ser Gly Asp Thr Ala Tyr Asn Gln Arg Phe
    50                  55                  60

Lys Gly Arg Val Thr Ile Thr Ala Asp Lys Ser Thr Ser Thr Ala Tyr
65                  70                  75                  80

Met Glu Leu Ser Ser Leu Arg Ser Glu Asp Thr Ala Val Tyr Tyr Cys
                85                  90                  95

Ala Arg Phe Tyr Ser Tyr Ala Tyr Trp Gly Gln Gly Thr Leu Val Thr
            100                 105                 110

Val Ser Ala Gly Gly Gly Gly Ser Gly Gly Gly Gly Ser Gly Gly Gly
        115                 120                 125

Gly Ser Asp Ile Val Met Thr Gln Thr Pro Leu Ser Leu Pro Val Thr
    130                 135                 140

Pro Gly Glu Pro Ala Ser Ile Ser Cys Arg Ser Ser Gln Ser Leu Val

145 150 155 160

His Ser Asn Gly Asn Thr Tyr Leu Gln Trp Tyr Leu Gln Lys Pro Gly
165 170 175

Gln Ser Pro Gln Leu Leu Ile Tyr Lys Val Ser Asn Arg Phe Ser Gly
180 185 190

Val Pro Asp Arg Phe Ser Gly Ser Gly Ser Gly Thr Asp Phe Thr Leu
195 200 205

Lys Ile Ser Arg Val Glu Ala Glu Asp Val Gly Val Tyr Tyr Cys Ser
210 215 220

Gln Ser Ile Tyr Val Pro Tyr Thr Phe Gly Gln Gly Thr Lys Leu Glu
225 230 235 240

Ile Lys Arg Arg Val Lys Phe Ser Arg Ser Ala Asp Ala Pro Ala Tyr
245 250 255

Gln Gln Gly Gln Asn Gln Leu Tyr Asn Glu Leu Asn Leu Gly Arg Arg
260 265 270

Glu Glu Tyr Asp Val Leu Asp Lys Arg Arg Gly Arg Asp Pro Glu Met
275 280 285

Gly Gly Lys Pro Gln Arg Arg Lys Asn Pro Gln Glu Gly Leu Tyr Asn
290 295 300

Glu Leu Gln Lys Asp Lys Met Ala Glu Ala Tyr Ser Glu Ile Gly Met
305 310 315 320

Lys Gly Glu Arg Arg Arg Gly Lys Gly His Asp Gly Leu Tyr Gln Gly
325 330 335

Leu Ser Thr Ala Thr Lys Asp Thr Tyr Asp Ala Leu His Met Gln Ala
340 345 350

Leu Pro Pro Arg
355

<210> 25
<211> 243
<212> PRT
<213> Artificial sequence

<220>
<223> Synthesized polypeptide

<400> 25

Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Ser Asp Tyr
20 25 30

Glu Met His Trp Val Arg Gln Ala Pro Gly Gln Gly Leu Glu Trp Met
35 40 45

Gly Ala Ile His Pro Gly Ser Gly Asp Thr Ala Tyr Asn Gln Arg Phe
50 55 60

Lys Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Phe Tyr Ser Tyr Ala Tyr Trp Gly Gln Gly Thr Leu Val Thr
100 105 110

Val Ser Ala Gly Gly Gly Gly Ser Gly Gly Gly Gly Ser Gly Gly Gly
115 120 125

Gly Ser Asp Ile Val Met Thr Gln Thr Pro Leu Ser Leu Pro Val Thr
130 135 140

Pro Gly Glu Pro Ala Ser Ile Ser Cys Arg Ser Ser Gln Ser Leu Val
145 150 155 160

His Ser Asn Gly Asn Thr Tyr Leu Gln Trp Tyr Leu Gln Lys Pro Gly
165 170 175

Gln Ser Pro Gln Leu Leu Ile Tyr Lys Val Ser Asn Arg Phe Ser Gly
180 185 190

Val Pro Asp Arg Phe Ser Gly Ser Gly Ser Gly Thr Asp Phe Thr Leu
195 200 205

Lys Ile Ser Arg Val Glu Ala Glu Asp Val Gly Val Tyr Tyr Cys Ser
210 215 220

Gln Ser Ile Tyr Val Pro Tyr Thr Phe Gly Gln Gly Thr Lys Leu Glu
225 230 235 240

Ile Lys Arg

<210> 26
<211> 540
<212> PRT
<213> Artificial sequence

<220>
<223> Synthesized polypeptide

<400> 26

Met Cys His Gln Gln Leu Val Ile Ser Trp Phe Ser Leu Val Phe Leu
1 5 10 15

Ala Ser Pro Leu Val Ala Ile Trp Glu Leu Lys Lys Asp Val Tyr Val
20 25 30

Val Glu Leu Asp Trp Tyr Pro Asp Ala Pro Gly Glu Met Val Val Leu
35 40 45

Thr Cys Asp Thr Pro Glu Glu Asp Gly Ile Thr Trp Thr Leu Asp Gln
50 55 60

Ser Ser Glu Val Leu Gly Ser Gly Lys Thr Leu Thr Ile Gln Val Lys
65 70 75 80

Glu Phe Gly Asp Ala Gly Gln Tyr Thr Cys His Lys Gly Gly Glu Val
85 90 95

Leu Ser His Ser Leu Leu Leu Leu His Lys Lys Glu Asp Gly Ile Trp
100 105 110

Ser Thr Asp Ile Leu Lys Asp Gln Lys Glu Pro Lys Asn Lys Thr Phe
115 120 125

Leu Arg Cys Glu Ala Lys Asn Tyr Ser Gly Arg Phe Thr Cys Trp Trp
130 135 140

Leu Thr Thr Ile Ser Thr Asp Leu Thr Phe Ser Val Lys Ser Ser Arg
145 150 155 160

Gly Ser Ser Asp Pro Gln Gly Val Thr Cys Gly Ala Ala Thr Leu Ser
165 170 175

Ala Glu Arg Val Arg Gly Asp Asn Lys Glu Tyr Glu Tyr Ser Val Glu
180 185 190

Cys Gln Glu Asp Ser Ala Cys Pro Ala Ala Glu Glu Ser Leu Pro Ile
195 200 205

Glu Val Met Val Asp Ala Val His Lys Leu Lys Tyr Glu Asn Tyr Thr
210 215 220

Ser Ser Phe Phe Ile Arg Asp Ile Ile Lys Pro Asp Pro Pro Lys Asn
225 230 235 240

Leu Gln Leu Lys Pro Leu Lys Asn Ser Arg Gln Val Glu Val Ser Trp
245 250 255

Glu Tyr Pro Asp Thr Trp Ser Thr Pro His Ser Tyr Phe Ser Leu Thr
260 265 270

Phe Cys Val Gln Val Gln Gly Lys Ser Lys Arg Glu Lys Lys Asp Arg
275 280 285

Val Phe Thr Asp Lys Thr Ser Ala Thr Val Ile Cys Arg Lys Asn Ala
290 295 300

Ser Ile Ser Val Arg Ala Gln Asp Arg Tyr Tyr Ser Ser Ser Trp Ser
305 310 315 320

Glu Trp Ala Ser Val Pro Cys Ser Gly Gly Gly Gly Ser Gly Gly Gly
325 330 335

Gly Ser Gly Gly Gly Gly Ser Arg Asn Leu Pro Val Ala Thr Pro Asp
340 345 350

Pro Gly Met Phe Pro Cys Leu His His Ser Gln Asn Leu Leu Arg Ala
355 360 365

Val Ser Asn Met Leu Gln Lys Ala Arg Gln Thr Leu Glu Phe Tyr Pro
370 375 380

Cys Thr Ser Glu Glu Ile Asp His Glu Asp Ile Thr Lys Asp Lys Thr
385 390 395 400

Ser Thr Val Glu Ala Cys Leu Pro Leu Glu Leu Thr Lys Asn Glu Ser
405 410 415

Cys Leu Asn Ser Arg Glu Thr Ser Phe Ile Thr Asn Gly Ser Cys Leu
420 425 430

Ala Ser Arg Lys Thr Ser Phe Met Met Ala Leu Cys Leu Ser Ser Ile
435 440 445

Tyr Glu Asp Leu Lys Met Tyr Gln Val Glu Phe Lys Thr Met Asn Ala

450 455 460

Lys Leu Leu Met Asp Pro Lys Arg Gln Ile Phe Leu Asp Gln Asn Met
465 470 475 480

Leu Ala Val Ile Asp Glu Leu Met Gln Ala Leu Asn Phe Asn Ser Glu
485 490 495

Thr Val Pro Gln Lys Ser Ser Leu Glu Glu Pro Asp Phe Tyr Lys Thr
500 505 510

Lys Ile Lys Leu Cys Ile Leu Leu His Ala Phe Arg Ile Arg Ala Val
515 520 525

Thr Ile Asp Arg Val Met Ser Tyr Leu Asn Ala Ser
530 535 540

<210> 27
<211> 543
<212> PRT
<213> Artificial sequence

<220>
<223> Synthesized polypeptide

<400> 27

Met Cys Pro Gln Lys Leu Thr Ile Ser Trp Phe Ala Ile Val Leu Leu
1 5 10 15

Val Ser Pro Leu Met Ala Met Trp Glu Leu Glu Lys Asp Val Tyr Val
20 25 30

Val Glu Val Asp Trp Thr Pro Asp Ala Pro Gly Glu Thr Val Asn Leu
35 40 45

Thr Cys Asp Thr Pro Glu Glu Asp Asp Ile Thr Trp Thr Ser Asp Gln
50 55 60

Arg His Gly Val Ile Gly Ser Gly Lys Thr Leu Thr Ile Thr Val Lys
65 70 75 80

Glu Phe Leu Asp Ala Gly Gln Tyr Thr Cys His Lys Gly Gly Glu Thr
85 90 95

Leu Ser His Ser His Leu Leu Leu His Lys Lys Glu Asn Gly Ile Trp
100 105 110

Ser Thr Glu Ile Leu Lys Asn Phe Lys Asn Lys Thr Phe Leu Lys Cys
115 120 125

Glu Ala Pro Asn Tyr Ser Gly Arg Phe Thr Cys Ser Trp Leu Val Gln
    130                 135                 140

Arg Asn Met Asp Leu Lys Phe Asn Ile Lys Ser Ser Ser Ser Ser Pro
145                 150                 155                 160

Asp Ser Arg Ala Val Thr Cys Gly Met Ala Ser Leu Ser Ala Glu Lys
                165                 170                 175

Val Thr Leu Asp Gln Arg Asp Tyr Glu Lys Tyr Ser Val Ser Cys Gln
            180                 185                 190

Glu Asp Val Thr Cys Pro Thr Ala Glu Glu Thr Leu Pro Ile Glu Leu
        195                 200                 205

Ala Leu Glu Ala Arg Gln Gln Asn Lys Tyr Glu Asn Tyr Ser Thr Ser
    210                 215                 220

Phe Phe Ile Arg Asp Ile Ile Lys Pro Asp Pro Pro Lys Asn Leu Gln
225                 230                 235                 240

Met Lys Pro Leu Lys Asn Ser Gln Val Glu Val Ser Trp Glu Tyr Pro
                245                 250                 255

Asp Ser Trp Ser Thr Pro His Ser Tyr Phe Ser Leu Lys Phe Phe Val
            260                 265                 270

Arg Ile Gln Arg Lys Lys Glu Lys Met Lys Glu Thr Glu Glu Gly Cys
        275                 280                 285

Asn Gln Lys Gly Ala Phe Leu Val Glu Lys Thr Ser Thr Glu Val Gln
    290                 295                 300

Cys Lys Gly Gly Asn Val Cys Val Gln Ala Gln Asp Arg Tyr Tyr Asn
305                 310                 315                 320

Ser Ser Cys Ser Lys Trp Ala Cys Val Pro Cys Arg Val Arg Ser Gly
                325                 330                 335

Gly Gly Gly Ser Gly Gly Gly Gly Ser Gly Gly Gly Gly Ser Arg Val
            340                 345                 350

Ile Pro Val Ser Gly Pro Ala Arg Cys Leu Ser Gln Ser Arg Asn Leu
        355                 360                 365

Leu Lys Thr Thr Asp Asp Met Val Lys Thr Ala Arg Glu Lys Leu Lys

370 375 380

His Tyr Ser Cys Thr Ala Glu Asp Ile Asp His Glu Asp Ile Thr Arg
385 390 395 400

Asp Gln Thr Ser Thr Leu Lys Thr Cys Leu Pro Leu Glu Leu His Lys
405 410 415

Asn Glu Ser Cys Leu Ala Thr Arg Glu Thr Ser Ser Thr Thr Arg Gly
420 425 430

Ser Cys Leu Pro Pro Gln Lys Thr Ser Leu Met Met Thr Leu Cys Leu
435 440 445

Gly Ser Ile Tyr Glu Asp Leu Lys Met Tyr Gln Thr Glu Phe Gln Ala
450 455 460

Ile Asn Ala Ala Leu Gln Asn His Asn His Gln Gln Ile Ile Leu Asp
465 470 475 480

Lys Gly Met Leu Val Ala Ile Asp Glu Leu Met Gln Ser Leu Asn His
485 490 495

Asn Gly Glu Thr Leu Arg Gln Lys Pro Pro Val Gly Glu Ala Asp Pro
500 505 510

Tyr Arg Val Lys Met Lys Leu Cys Ile Leu Leu His Ala Phe Ser Thr
515 520 525

Arg Val Val Thr Ile Asn Arg Val Met Gly Tyr Leu Ser Ser Ala
530 535 540

<210> 28
<211> 4415
<212> DNA
<213> Artificial sequence

<220>
<223> Synthesized polynucleotide

<400> 28
cgatggctcc ggtgcccgtc agtgggcaga gcgcacatcg cccacagtcc ccgagaagtt 60

gggggggaggg gtcggcaatt gaaccggtgc ctagagaagg tggcgcgggg taaactggga 120

aagtgatgtc gtgtactggc tccgcctttt tcccgagggt gggggagaac cgtatataag 180

tgcagtagtc gccgtgaacg ttcttttcg caacgggttt gccgccagaa cacaggtgtc 240

gtgacgcgga tccaggccta agcttacgcg tcctagcgct accggtcgcc accatggcct 300

taccagtgac cgccttgctc ctgccgctgg ccttgctgct ccacgccgcc aggccggagg 360

tgcagctggt gcagagcggc gccgaggtga agaagcccgg cgccagcgtg aaggtgagct 420
gcaaggccag cggctacacc ttcagcgact acgagatgca ctgggtgcgg caggcccccg 480
gccagggcct ggagtggatg ggcgccatcc accccggcag cggcgacacc gcctacaacc 540
agcggttcaa gggccgggtg accatcaccg ccgacaagag caccagcacc gcctacatgg 600
agctgagcag cctgcggagc gaggacaccg ccgtgtacta ctgcgcccgg ttctacagct 660
acgcctactg gggccagggc accctggtga ccgtgagcgc cggtggaggc ggttcaggcg 720
gaggtggttc tggcggtggc ggatcggaca tcgtgatgac ccagaccccc ctgagcctgc 780
ccgtgacccc cggcgagccc gccagcatca gctgccggag cagccagagc ctggtgcaca 840
gcaacggcaa cacctacctg cagtggtacc tgcagaagcc cggccagagc ccccagctgc 900
tgatctacaa ggtgagcaac cggttcagcg gcgtgcccga ccggttcagc ggcagcggca 960
gcggcaccga cttcaccctg aagatcagcc gggtggaggc cgaggacgtg ggcgtgtact 1020
actgcagcca gagcatctac gtgccctaca ccttcggcca gggcaccaag ctggagatca 1080
aacgtaccac gacgccagcg ccgcgaccac caacaccggc gcccaccatc gcgtcgcagc 1140
ccctgtccct gcgcccagag gcgtgccggc cagcggcggg gggcgcagtg cacacgaggg 1200
ggctggactt cgcctgtgat ttttgggtgc tggtggtggt tggtggagtc ctggcttgct 1260
atagcttgct agtaacagtg gcctttatta ttttctgggt gaggagtaag aggagcaggc 1320
tcctgcacag tgactacatg aacatgactc cccgccgccc cgggccaacc cgcaagcatt 1380
accagcccta tgccccacca cgcgacttcg cagcctatcg ctccagagtg aagttcagca 1440
ggagcgcaga cgcccccgcg taccagcagg gccagaacca gctctataac gagctcaatc 1500
taggacgaag agaggagtac gatgttttgg acaagagacg tggccgggac cctgagatgg 1560
ggggaaagcc gcagagaagg aagaaccctc aggaaggcct gtacaatgaa ctgcagaaag 1620
ataagatggc ggaggcctac agtgagattg ggatgaaagg cgagcgccgg aggggcaagg 1680
ggcacgatgg cctttaccag ggtctcagta cagccaccaa ggacacctac gacgcccttc 1740
acatgcaggc cctgccccct cgctaggtcg acaatcaacc tctggattac aaaatttgtg 1800
aaagattgac tggtattctt aactatgttg ctccttttac gctatgtgga tacgctgctt 1860
taatgccttt gtatcatgct attgcttccc gtatggcttt cattttctcc tccttgtata 1920
aatcctggtt gctgtctctt tatgaggagt tgtggcccgt tgtcaggcaa cgtggcgtgg 1980
tgtgcactgt gtttgctgac gcaaccccca ctggttgggg cattgccacc acctgtcagc 2040
tcctttccgg gactttcgct ttccccctcc ctattgccac ggcggaactc atcgccgcct 2100
gccttgcccg ctgctggaca ggggctcggc tgttgggcac tgacaattcc gtggtgttgt 2160
cggggaagct gacgtccttt ccatggctgc tcgcctgtgt tgccacctgg attctgcgcg 2220

ggacgtcctt ctgctacgtc ccttcggccc tcaatccagc ggaccttcct tcccgcggcc 2280
tgctgccggc tctgcggcct cttccgcgtc ttcgccttcg ccctcagacg agtcggatct 2340
ccctttgggc cgcctccccg cctggaattc gctagcctcg agctcacaca aaaaaccaac 2400
acacagatgt aatgaaaata aagatatttt attgcggccg ctttaggaag cattcagata 2460
gctcatcact ctatcaatag tcactgcccg aattctgaaa gcatgaagaa gtatgcagag 2520
cttgatttta gttttataaa aatccggttc ttcaagggag gatttttgtg gcacagtctc 2580
actgttgaaa ttcagggcct gcatcagctc atcaataact gccagcatgt tttgatctag 2640
aaagatctgc ctcttaggat ccatcagaag ctttgcattc atggtcttga actccacctg 2700
gtacatcttc aagtcttcat aaatactact aaggcacagg gccatcataa aagaggtctt 2760
tctggaggcc aggcaactcc cattagttat gaaagaggtc tctctggaat ttaggcaact 2820
ctcattcttg gttaattcca atggtaaaca ggcctccact gtgctggttt tatcttttgt 2880
gatatcttca tgatcaatct cttcagaagt gcaagggtaa aattctagag tttgtctggc 2940
cttctggagc atgttgctga cggccctcag caggttttgg gagtggtgaa ggcatgggaa 3000
cattcctggg tctggagtgg ccacggggag gtttctagat ccgccgccac ccgacccacc 3060
accgcccgag ccaccgccac cactgcaggg cacagatgcc cattcgctcc aagatgagct 3120
atagtagcgg tcctgggccc gcacgctaat gctggcattt ttgcggcaga tgaccgtggc 3180
tgaggtcttg tccgtgaaga ctctatcttt cttttctctc ttgctcttgc cctggacctg 3240
aacgcagaat gtcagggaga agtaggaatg tggagtactc caggtgtcag ggtactccca 3300
gctgacctcc acctgccgag aattctttaa tggcttcagc tgcaagttct tgggtgggtc 3360
aggtttgatg atgtccctga tgaagaagct gctggtgtag ttttcatact tgagcttgtg 3420
aacggcatcc accatgacct caatgggcag actctcctca gcagctgggc aggcactgtc 3480
ctcctggcac tccactgagt actcatactc cttgttgtcc cctctgactc tctctgcaga 3540
gagtgtagca gctccgcacg tcaccccttg gggtcagaa gagcctctgc tgcttttgac 3600
actgaatgtc aaatcagtac tgattgtcgt cagccaccag caggtgaaac gtccagaata 3660
attcttggcc tcgcatctta gaaaggtctt atttttgggt tctttctggt cctttaaaat 3720
atcagtggac caaattccat cttccttttt gtgaagcagc aggagcgaat ggcttagaac 3780
ctcgcctcct ttgtgacagg tgtactggcc agcatctcca aactctttga cttggatggt 3840
cagggttttg ccagagccta agacctcact gctctggtcc aaggtccagg tgataccatc 3900
ttcttcaggg gtgtcacagg tgaggaccac catttctcca ggggcatccg gataccaatc 3960
caattctacg acataaacat ctttcttcag ttcccatatg gccacgaggg gagatgccag 4020
aaaaaccagg gaaaaccaag agatgaccaa ctgctggtga cacatggtgg cgaccggtag 4080
cgctaggtca tatgcaggag ttgaggttac tgtgagtagt gattaaagag agtgataggg 4140

aactcttgaa caagagatgc aatttatact gttaattctg gaaaaatatt atgggggtgt 4200

caaaatgtcc cgggacaatt gacgccttct gtatgaaaca gtttttcctc cacgccttct 4260

gtatgaaaca gtttttcctc cacgccttct gtatgaaaca gtttttcctc cgtcgaggac 4320

aattgacgcc ttctgtatga aacagttttt cctccacgcc ttctgtatga aacagttttt 4380

cctccacgcc ttctgtatga aacagttttt cctcc 4415

<210> 29
<211> 4418
<212> DNA
<213> Artificial sequence

<220>
<223> Synthesized polynucleotide

<400> 29
cgatggctcc ggtgcccgtc agtgggcaga gcgcacatcg cccacagtcc ccgagaagtt 60

ggggggaggg gtcggcaatt gaaccggtgc ctagagaagg tggcgcgggg taaactggga 120

aagtgatgtc gtgtactggc tccgcctttt tcccgagggt gggggagaac cgtatataag 180

tgcagtagtc gccgtgaacg ttctttttcg caacgggttt gccgccagaa cacaggtgtc 240

gtgacgcgga tccaggccta agcttacgcg tcctagcgct accggtcgcc accatggcct 300

taccagtgac cgccttgctc ctgccgctgg ccttgctgct ccacgccgcc aggccggagg 360

tgcagctggt gcagagcggc gccgaggtga agaagcccgg cgccagcgtg aaggtgagct 420

gcaaggccag cggctacacc ttcagcgact acgagatgca ctgggtgcgg caggcccccg 480

gccagggcct ggagtggatg ggcgccatcc accccggcag cggcgacacc gcctacaacc 540

agcggttcaa gggccgggtg accatcaccg ccgacaagag caccagcacc gcctacatgg 600

agctgagcag cctgcggagc gaggacaccg ccgtgtacta ctgcgcccgg ttctacagct 660

acgcctactg gggccagggc accctggtga ccgtgagcgc cggtggaggc ggttcaggcg 720

gaggtggttc tggcggtggc ggatcggaca tcgtgatgac ccagaccccc ctgagcctgc 780

ccgtgacccc cggcgagccc gccagcatca gctgccggag cagccagagc ctggtgcaca 840

gcaacggcaa cacctacctg cagtggtacc tgcagaagcc cggccagagc ccccagctgc 900

tgatctacaa ggtgagcaac cggttcagcg gcgtgcccga ccggttcagc ggcagcggca 960

gcggcaccga cttcaccctg aagatcagcc gggtggaggc cgaggacgtg ggcgtgtact 1020

actgcagcca gagcatctac gtgccctaca ccttcggcca gggcaccaag ctggagatca 1080

aacgtaccac gacgccagcg ccgcgaccac caacaccggc gcccaccatc gcgtcgcagc 1140

ccctgtccct gcgcccagag gcgtgccggc cagcggcggg gggcgcagtg cacacgaggg 1200

ggctggactt cgcctgtgat ttttgggtgc tggtggtggt tggtggagtc ctggcttgct 1260

atagcttgct agtaacagtg gcctttatta ttttctgggt gaaacggggc agaaagaaac 1320
tcctgtatat attcaaacaa ccatttatga gaccagtaca aactactcaa gaggaagatg 1380
gctgtagctg ccgatttcca gaagaagaag aaggaggatg tgaactgaga gtgaagttca 1440
gcaggagcgc agacgccccc gcgtaccagc agggccagaa ccagctctat aacgagctca 1500
atctaggacg aagagaggag tacgatgttt tggacaagag acgtggccgg gaccctgaga 1560
tgggggggaaa gccgcagaga aggaagaacc ctcaggaagg cctgtacaat gaactgcaga 1620
aagataagat ggcggaggcc tacagtgaga ttgggatgaa aggcgagcgc cggaggggca 1680
aggggcacga tggcctttac cagggtctca gtacagccac caaggacacc tacgacgccc 1740
ttcacatgca ggccctgccc cctcgctagg tcgacaatca acctctggat tacaaaattt 1800
gtgaaagatt gactggtatt cttaactatg ttgctccttt tacgctatgt ggatacgctg 1860
ctttaatgcc tttgtatcat gctattgctt cccgtatggc tttcattttc tcctccttgt 1920
ataaatcctg gttgctgtct ctttatgagg agttgtggcc cgttgtcagg caacgtggcg 1980
tggtgtgcac tgtgtttgct gacgcaaccc ccactggttg gggcattgcc accacctgtc 2040
agctcctttc cgggactttc gctttccccc tccctattgc cacggcggaa ctcatcgccg 2100
cctgccttgc ccgctgctgg acaggggctc ggctgttggg cactgacaat tccgtggtgt 2160
tgtcggggaa gctgacgtcc tttccatggc tgctcgcctg tgttgccacc tggattctgc 2220
gcgggacgtc cttctgctac gtcccttcgg ccctcaatcc agcggacctt ccttcccgcg 2280
gcctgctgcc ggctctgcgg cctcttccgc gtcttcgcct tcgccctcag acgagtcgga 2340
tctccctttg ggccgcctcc ccgcctggaa ttcgctagcc tcgagctcac acaaaaaacc 2400
aacacacaga tgtaatgaaa ataaagatat tttattgcgg ccgctttagg aagcattcag 2460
atagctcatc actctatcaa tagtcactgc ccgaattctg aaagcatgaa gaagtatgca 2520
gagcttgatt ttagttttat aaaaatccgg ttcttcaagg gaggattttt gtggcacagt 2580
ctcactgttg aaattcaggg cctgcatcag ctcatcaata actgccagca tgttttgatc 2640
tagaaagatc tgcctcttag gatccatcag aagctttgca ttcatggtct tgaactccac 2700
ctggtacatc ttcaagtctt cataaatact actaaggcac agggccatca taaaagaggt 2760
ctttctggag gccaggcaac tcccattagt tatgaaagag gtctctctgg aatttaggca 2820
actctcattc ttggttaatt ccaatggtaa acaggcctcc actgtgctgg ttttatcttt 2880
tgtgatatct tcatgatcaa tctcttcaga agtgcaaggg taaaattcta gagtttgtct 2940
ggccttctgg agcatgttgc tgacggccct cagcaggttt tgggagtggt gaaggcatgg 3000
gaacattcct gggtctggag tggccacggg gaggtttcta gatccgccgc cacccgaccc 3060
accaccgccc gagccaccgc caccactgca gggcacagat gcccattcgc tccaagatga 3120
gctatagtag cggtcctggg cccgcacgct aatgctggca tttttgcggc agatgaccgt 3180

ggctgaggtc ttgtccgtga agactctatc tttcttttct ctcttgctct tgccctggac 3240

ctgaacgcag aatgtcaggg agaagtagga atgtggagta ctccaggtgt cagggtactc 3300

ccagctgacc tccacctgcc gagaattctt taatggcttc agctgcaagt tcttgggtgg 3360

gtcaggtttg atgatgtccc tgatgaagaa gctgctggtg tagttttcat acttgagctt 3420

gtgaacggca tccaccatga cctcaatggg cagactctcc tcagcagctg ggcaggcact 3480

gtcctcctgg cactccactg agtactcata ctccttgttg tcccctctga ctctctctgc 3540

agagagtgta gcagctccgc acgtcacccc ttgggggtca gaagagcctc tgctgctttt 3600

gacactgaat gtcaaatcag tactgattgt cgtcagccac cagcaggtga aacgtccaga 3660

ataattcttg gcctcgcatc ttagaaaggt cttatttttg ggttctttct ggtcctttaa 3720

aatatcagtg gaccaaattc catcttcctt tttgtgaagc agcaggagcg aatggcttag 3780

aacctcgcct cctttgtgac aggtgtactg gccagcatct ccaaactctt tgacttggat 3840

ggtcagggtt ttgccagagc ctaagacctc actgctctgg tccaaggtcc aggtgatacc 3900

atcttcttca ggggtgtcac aggtgaggac caccatttct ccaggggcat ccggatacca 3960

atccaattct acgacataaa catctttctt cagttcccat atggccacga ggggagatgc 4020

cagaaaaacc agggaaaacc aagagatgac caactgctgg tgacacatgg tggcgaccgg 4080

tagcgctagg tcatatgcag gagttgaggt tactgtgagt agtgattaaa gagagtgata 4140

gggaactctt gaacaagaga tgcaatttat actgttaatt ctggaaaaat attatggggg 4200

tgtcaaaatg tcccgggaca attgacgcct tctgtatgaa acagtttttc ctccacgcct 4260

tctgtatgaa acagtttttc ctccacgcct tctgtatgaa acagtttttc ctccgtcgag 4320

gacaattgac gccttctgta tgaaacagtt tttcctccac gccttctgta tgaaacagtt 4380

tttcctccac gccttctgta tgaaacagtt tttcctcc 4418

<210> 30
<211> 4541
<212> DNA
<213> Artificial sequence

<220>
<223> Synthesized polynucleotide

<400> 30
cgatggctcc ggtgcccgtc agtgggcaga gcgcacatcg cccacagtcc ccgagaagtt 60

ggggggaggg gtcggcaatt gaaccggtgc ctagagaagg tggcgcgggg taaactggga 120

aagtgatgtc gtgtactggc tccgcctttt tcccgagggt gggggagaac cgtatataag 180

tgcagtagtc gccgtgaacg ttctttttcg caacgggttt gccgccagaa cacaggtgtc 240

gtgacgcgga tccaggccta agcttacgcg tcctagcgct accggtcgcc accatggcct 300

taccagtgac cgccttgctc ctgccgctgg ccttgctgct ccacgccgcc aggccggagg 360
tgcagctggt gcagagcggc gccgaggtga agaagcccgg cgccagcgtg aaggtgagct 420
gcaaggccag cggctacacc ttcagcgact acgagatgca ctgggtgcgg caggcccccg 480
gccagggcct ggagtggatg ggcgccatcc accccggcag cggcgacacc gcctacaacc 540
agcggttcaa gggccgggtg accatcaccg ccgacaagag caccagcacc gcctacatgg 600
agctgagcag cctgcggagc gaggacaccg ccgtgtacta ctgcgcccgg ttctacagct 660
acgcctactg gggccagggc accctggtga ccgtgagcgc cggtggaggc ggttcaggcg 720
gaggtggttc tggcggtggc ggatcggaca tcgtgatgac ccagaccccc ctgagcctgc 780
ccgtgacccc cggcgagccc gccagcatca gctgccggag cagccagagc ctggtgcaca 840
gcaacggcaa cacctacctg cagtggtacc tgcagaagcc cggccagagc ccccagctgc 900
tgatctacaa ggtgagcaac cggttcagcg gcgtgcccga ccggttcagc ggcagcggca 960
gcggcaccga cttcaccctg aagatcagcc gggtggaggc cgaggacgtg ggcgtgtact 1020
actgcagcca gagcatctac gtgccctaca ccttcggcca gggcaccaag ctggagatca 1080
aacgtaccac gacgccagcg ccgcgaccac caacaccggc gcccaccatc gcgtcgcagc 1140
ccctgtccct gcgcccagag gcgtgccggc cagcggcggg gggcgcagtg cacacgaggg 1200
ggctggactt cgcctgtgat ttttgggtgc tggtggtggt tggtggagtc ctggcttgct 1260
atagcttgct agtaacagtg gcctttatta ttttctgggt gaggagtaag aggagcaggc 1320
tcctgcacag tgactacatg aacatgactc cccgccgccc cgggccaacc cgcaagcatt 1380
accagcccta tgccccacca cgcgacttcg cagcctatcg ctccaaacgg ggcagaaaga 1440
aactcctgta tatattcaaa caaccattta tgagaccagt acaaactact caagaggaag 1500
atggctgtag ctgccgattt ccagaagaag aagaaggagg atgtgaactg agagtgaagt 1560
tcagcaggag cgcagacgcc cccgcgtacc agcagggcca gaaccagctc tataacgagc 1620
tcaatctagg acgaagagag gagtacgatg ttttggacaa gagacgtggc cgggaccctg 1680
agatgggggg aaagccgcag agaaggaaga accctcagga aggcctgtac aatgaactgc 1740
agaaagataa gatggcggag gcctacagtg agattgggat gaaaggcgag cgccggaggg 1800
gcaaggggca cgatggcctt taccagggtc tcagtacagc caccaaggac acctacgacg 1860
cccttcacat gcaggccctg ccccctcgct aggtcgacaa tcaacctctg gattacaaaa 1920
tttgtgaaag attgactggt attcttaact atgttgctcc ttttacgcta tgtggatacg 1980
ctgctttaat gcctttgtat catgctattg cttcccgtat ggctttcatt ttctcctcct 2040
tgtataaatc ctggttgctg tctctttatg aggagttgtg gcccgttgtc aggcaacgtg 2100
gcgtggtgtg cactgtgttt gctgacgcaa cccccactgg ttggggcatt gccaccacct 2160
gtcagctcct ttccgggact ttcgctttcc ccctccctat tgccacggcg gaactcatcg 2220

ccgcctgcct tgcccgctgc tggacagggg ctcggctgtt gggcactgac aattccgtgg 2280
tgttgtcggg gaagctgacg tcctttccat ggctgctcgc ctgtgttgcc acctggattc 2340
tgcgcgggac gtccttctgc tacgtccctt cggccctcaa tccagcggac cttccttccc 2400
gcggcctgct gccggctctg cggcctcttc cgcgtcttcg ccttcgccct cagacgagtc 2460
ggatctccct ttgggccgcc tccccgcctg gaattcgcta gcctcgagct cacacaaaaa 2520
accaacacac agatgtaatg aaaataaaga tattttattg cggccgcttt aggaagcatt 2580
cagatagctc atcactctat caatagtcac tgcccgaatt ctgaaagcat gaagaagtat 2640
gcagagcttg attttagttt tataaaaatc cggttcttca agggaggatt tttgtggcac 2700
agtctcactg ttgaaattca gggcctgcat cagctcatca ataactgcca gcatgttttg 2760
atctagaaag atctgcctct taggatccat cagaagcttt gcattcatgg tcttgaactc 2820
cacctggtac atcttcaagt cttcataaat actactaagg cacagggcca tcataaaaga 2880
ggtctttctg gaggccaggc aactcccatt agttatgaaa gaggtctctc tggaatttag 2940
gcaactctca ttcttggtta attccaatgg taaacaggcc tccactgtgc tggttttatc 3000
ttttgtgata tcttcatgat caatctcttc agaagtgcaa gggtaaaatt ctagagtttg 3060
tctggccttc tggagcatgt tgctgacggc cctcagcagg ttttgggagt ggtgaaggca 3120
tgggaacatt cctgggtctg gagtggccac ggggaggttt ctagatccgc cgccacccga 3180
cccaccaccg cccgagccac cgccaccact gcagggcaca gatgcccatt cgctccaaga 3240
tgagctatag tagcggtcct gggcccgcac gctaatgctg gcatttttgc ggcagatgac 3300
cgtggctgag gtcttgtccg tgaagactct atctttcttt tctctcttgc tcttgccctg 3360
gacctgaacg cagaatgtca gggagaagta ggaatgtgga gtactccagg tgtcagggta 3420
ctcccagctg acctccacct gccgagaatt ctttaatggc ttcagctgca agttcttggg 3480
tgggtcaggt ttgatgatgt ccctgatgaa gaagctgctg gtgtagtttt catacttgag 3540
cttgtgaacg gcatccacca tgacctcaat gggcagactc tcctcagcag ctgggcaggc 3600
actgtcctcc tggcactcca ctgagtactc atactccttg ttgtcccctc tgactctctc 3660
tgcagagagt gtagcagctc cgcacgtcac cccttggggg tcagaagagc ctctgctgct 3720
tttgacactg aatgtcaaat cagtactgat tgtcgtcagc caccagcagg tgaaacgtcc 3780
agaataattc ttggcctcgc atcttagaaa ggtcttattt ttgggttctt tctggtcctt 3840
taaaatatca gtggaccaaa ttccatcttc ctttttgtga agcagcagga gcgaatggct 3900
tagaacctcg cctcctttgt gacaggtgta ctggccagca tctccaaact ctttgacttg 3960
gatggtcagg gttttgccag agcctaagac ctcactgctc tggtccaagg tccaggtgat 4020
accatcttct tcaggggtgt cacaggtgag gaccaccatt tctccagggg catccggata 4080

ccaatccaat tctacgacat aaacatcttt cttcagttcc catatggcca cgaggggaga 4140

tgccagaaaa accagggaaa accaagagat gaccaactgc tggtgacaca tggtggcgac 4200

cggtagcgct aggtcatatg caggagttga ggttactgtg agtagtgatt aaagagagtg 4260

atagggaact cttgaacaag agatgcaatt tatactgtta attctggaaa aatattatgg 4320

gggtgtcaaa atgtcccggg acaattgacg ccttctgtat gaaacagttt ttcctccacg 4380

ccttctgtat gaaacagttt ttcctccacg ccttctgtat gaaacagttt ttcctccgtc 4440

gaggacaatt gacgccttct gtatgaaaca gtttttcctc cacgccttct gtatgaaaca 4500

gtttttcctc cacgccttct gtatgaaaca gtttttcctc c 4541

<210> 31
<211> 4292
<212> DNA
<213> Artificial sequence

<220>
<223> Synthesized polynucleotide

<400> 31
cgatggctcc ggtgcccgtc agtgggcaga gcgcacatcg cccacagtcc ccgagaagtt 60

ggggggaggg gtcggcaatt gaaccggtgc ctagagaagg tggcgcgggg taaactggga 120

aagtgatgtc gtgtactggc tccgcctttt tcccgagggt gggggagaac cgtatataag 180

tgcagtagtc gccgtgaacg ttctttttcg caacgggttt gccgccagaa cacaggtgtc 240

gtgacgcgga tccaggccta agcttacgcg tcctagcgct accggtcgcc accatggcct 300

taccagtgac cgccttgctc ctgccgctgg ccttgctgct ccacgccgcc aggccggagg 360

tgcagctggt gcagagcggc gccgaggtga agaagcccgg cgccagcgtg aaggtgagct 420

gcaaggccag cggctacacc ttcagcgact acgagatgca ctgggtgcgg caggcccccg 480

gccagggcct ggagtggatg ggcgccatcc accccggcag cggcgacacc gcctacaacc 540

agcggttcaa gggccgggtg accatcaccg ccgacaagag caccagcacc gcctacatgg 600

agctgagcag cctgcggagc gaggacaccg ccgtgtacta ctgcgcccgg ttctacagct 660

acgcctactg gggccagggc accctggtga ccgtgagcgc cggtggaggc ggttcaggcg 720

gaggtggttc tggcggtggc ggatcggaca tcgtgatgac ccagaccccc ctgagcctgc 780

ccgtgacccc cggcgagccc gccagcatca gctgccggag cagccagagc ctggtgcaca 840

gcaacggcaa cacctacctg cagtggtacc tgcagaagcc cggccagagc ccccagctgc 900

tgatctacaa ggtgagcaac cggttcagcg gcgtgcccga ccggttcagc ggcagcggca 960

gcggcaccga cttcaccctg aagatcagcc gggtggaggc cgaggacgtg ggcgtgtact 1020

actgcagcca gagcatctac gtgccctaca ccttcggcca gggcaccaag ctggagatca 1080

aacgtaccac gacgccagcg ccgcgaccac caacaccggc gcccaccatc gcgtcgcagc 1140

ccctgtccct gcgcccagag gcgtgccggc cagcggcggg gggcgcagtg cacacgaggg 1200
ggctggactt cgcctgtgat tttgggtgc tggtggtggt tggtggagtc ctggcttgct 1260
atagcttgct agtaacagtg gcctttatta ttttctgggt gagagtgaag ttcagcagga 1320
gcgcagacgc ccccgcgtac cagcagggcc agaaccagct ctataacgag ctcaatctag 1380
gacgaagaga ggagtacgat gttttggaca agagacgtgg ccgggaccct gagatggggg 1440
gaaagccgca gagaaggaag aaccctcagg aaggcctgta caatgaactg cagaaagata 1500
agatggcgga ggcctacagt gagattggga tgaaaggcga gcgccggagg ggcaaggggc 1560
acgatggcct ttaccagggt ctcagtacag ccaccaagga cacctacgac gcccttcaca 1620
tgcaggccct gccccctcgc taggtcgaca atcaacctct ggattacaaa atttgtgaaa 1680
gattgactgg tattcttaac tatgttgctc cttttacgct atgtggatac gctgctttaa 1740
tgcctttgta tcatgctatt gcttcccgta tggctttcat tttctcctcc ttgtataaat 1800
cctggttgct gtctctttat gaggagttgt ggcccgttgt caggcaacgt ggcgtggtgt 1860
gcactgtgtt tgctgacgca acccccactg gttggggcat tgccaccacc tgtcagctcc 1920
tttccgggac tttcgctttc cccctcccta ttgccacggc ggaactcatc gccgcctgcc 1980
ttgcccgctg ctggacaggg gctcggctgt tgggcactga caattccgtg gtgttgtcgg 2040
ggaagctgac gtcctttcca tggctgctcg cctgtgttgc cacctggatt ctgcgcggga 2100
cgtccttctg ctacgtccct tcggccctca atccagcgga ccttccttcc cgcggcctgc 2160
tgccggctct gcggcctctt ccgcgtcttc gccttcgccc tcagacgagt cggatctccc 2220
tttgggccgc ctccccgcct ggaattcgct agcctcgagc tcacacaaaa aaccaacaca 2280
cagatgtaat gaaaataaag atattttatt gcggccgctt taggaagcat tcagatagct 2340
catcactcta tcaatagtca ctgcccgaat tctgaaagca tgaagaagta tgcagagctt 2400
gattttagtt ttataaaaat ccggttcttc aagggaggat tttgtggca cagtctcact 2460
gttgaaattc agggcctgca tcagctcatc aataactgcc agcatgtttt gatctagaaa 2520
gatctgcctc ttaggatcca tcagaagctt tgcattcatg gtcttgaact ccacctggta 2580
catcttcaag tcttcataaa tactactaag gcacagggcc atcataaaag aggtctttct 2640
ggaggccagg caactcccat tagttatgaa agaggtctct ctggaattta ggcaactctc 2700
attcttggtt aattccaatg gtaaacaggc ctccactgtg ctggttttat cttttgtgat 2760
atcttcatga tcaatctctt cagaagtgca agggtaaaat tctagagttt gtctggcctt 2820
ctggagcatg ttgctgacgg ccctcagcag gttttgggag tggtgaaggc atgggaacat 2880
tcctgggtct ggagtggcca cggggaggtt tctagatccg ccgccacccg acccaccacc 2940
gcccgagcca ccgccaccac tgcagggcac agatgcccat tcgctccaag atgagctata 3000

gtagcggtcc tgggcccgca cgctaatgct ggcatttttg cggcagatga ccgtggctga 3060

ggtcttgtcc gtgaagactc tatctttctt ttctctcttg ctcttgccct ggacctgaac 3120

gcagaatgtc agggagaagt aggaatgtgg agtactccag gtgtcagggt actcccagct 3180

gacctccacc tgccgagaat tctttaatgg cttcagctgc aagttcttgg gtgggtcagg 3240

tttgatgatg tccctgatga agaagctgct ggtgtagttt tcatacttga gcttgtgaac 3300

ggcatccacc atgacctcaa tgggcagact ctcctcagca gctgggcagg cactgtcctc 3360

ctggcactcc actgagtact catactcctt gttgtcccct ctgactctct ctgcagagag 3420

tgtagcagct ccgcacgtca ccccttgggg gtcagaagag cctctgctgc ttttgacact 3480

gaatgtcaaa tcagtactga ttgtcgtcag ccaccagcag gtgaaacgtc cagaataatt 3540

cttggcctcg catcttagaa aggtcttatt tttgggttct ttctggtcct ttaaaatatc 3600

agtggaccaa attccatctt cctttttgtg aagcagcagg agcgaatggc ttagaacctc 3660

gcctcctttg tgacaggtgt actggccagc atctccaaac tctttgactt ggatggtcag 3720

ggttttgcca gagcctaaga cctcactgct ctggtccaag gtccaggtga taccatcttc 3780

ttcaggggtg tcacaggtga ggaccaccat ttctccaggg gcatccggat accaatccaa 3840

ttctacgaca taaacatctt tcttcagttc ccatatggcc acgaggggag atgccagaaa 3900

aaccagggaa aaccaagaga tgaccaactg ctggtgacac atggtggcga ccggtagcgc 3960

taggtcatat gcaggagttg aggttactgt gagtagtgat taaagagagt gatagggaac 4020

tcttgaacaa gagatgcaat ttatactgtt aattctggaa aaatattatg ggggtgtcaa 4080

aatgtcccgg gacaattgac gccttctgta tgaaacagtt tttcctccac gccttctgta 4140

tgaaacagtt tttcctccac gccttctgta tgaaacagtt tttcctccgt cgaggacaat 4200

tgacgccttc tgtatgaaac agtttttcct ccacgccttc tgtatgaaac agtttttcct 4260

ccacgccttc tgtatgaaac agtttttcct cc 4292

<210> 32
<211> 3391
<212> DNA
<213> Artificial sequence

<220>
<223> Synthesized polynucleotide

<400> 32
atggcctcac cgttgacccg ctttctgtcg ctgaacctgc tgctgctggg tgagtcgatt 60

atcctgggga gtggagaagc tgaggtgcag ctggtgcaga gcggcgccga ggtgaagaag 120

cccggcgcca gcgtgaaggt gagctgcaag gccagcggct acaccttcag cgactacgag 180

atgcactggg tgcggcaggc ccccggccag ggcctggagt ggatgggcgc catccacccc 240

ggcagcggcg acaccgccta caaccagcgg ttcaagggcc gggtgaccat caccgccgac 300

aagagcacca gcaccgccta catggagctg agcagcctgc ggagcgagga caccgccgtg 360

tactactgcg cccggttcta cagctacgcc tactggggcc agggcaccct ggtgaccgtg 420

agcgccggtg gaggcggttc aggcggaggt ggttctggcg gtggcggatc ggacatcgtg 480

atgacccaga ccccccctgag cctgcccgtg acccccggcg agcccgccag catcagctgc 540

cggagcagcc agagcctggt gcacagcaac ggcaacacct acctgcagtg gtacctgcag 600

aagcccggcc agagcccca gctgctgatc tacaaggtga gcaaccggtt cagcggcgtg 660

cccgaccggt tcagcggcag cggcagcggc accgacttca ccctgaagat cagccgggtg 720

gaggccgagg acgtgggcgt gtactactgc agccagagca tctacgtgcc ctacaccttc 780

ggccagggca ccaagctgga gatcaaacgt actactacca agccagtgct gcgaactccc 840

tcacctgtgc accctaccgg gacatctcag ccccagagac cagaagattg tcggccccgt 900

ggctcagtga aggggaccgg attggacttc gcctgtgata tttacatctg ggcacccttg 960

gccggaatct gcgtggccct tctgctgtcc ttgatcatca ctctcatctg ctaccacagg 1020

agccgaagca ggagtgcaga gactgctgcc aacctgcagg accccaacca gctctacaat 1080

gagctcaatc tagggcgaag agaggaatat gacgtcttgg agaagaagcg ggctcgggat 1140

ccagagatgg gaggcaaaca gcagaggagg aggaaccccc aggaaggcgt atacaatgca 1200

ctgcagaaag acaagatggc agaagcctac agtgagatcg gcacaaaagg cgagaggcgg 1260

agaggcaagg ggcacgatgg cctttaccag ggtctcagca ctgccaccaa ggacacctat 1320

gatgccctgc atatgcagac cctggcctag gtcgactcac acaaaaaacc aacacacaga 1380

tgtaatgaaa ataaagatat tttattcgta cgttaggcgg agctcagata gcccatcacc 1440

ctgttgatgg tcacgacgcg ggtgctgaag gcgtgaagca ggatgcagag cttcattttc 1500

actctgtaag ggtctgcttc tcccacagga ggtttctggc gcagagtctc gccattatga 1560

ttcagagact gcatcagctc atcgatggcc accagcatgc ccttgtctag aatgatctgc 1620

tgatggttgt gattctgaag tgctgcgttg atggcctgga actctgtctg gtacatcttc 1680

aagtcctcat agatgctacc aaggcacagg gtcatcatca aagacgtctt ctgtgggggc 1740

aggcagctcc ctcttgttgt ggaagaagtc tctctagtag ccaggcaact ctcgttcttg 1800

tgtagttcca gtggtaaaca ggtcttcaat gtgctggttt ggtcccgtgt gatgtcttca 1860

tgatcgatgt cttcagcagt gcaggaataa tgtttcagtt tttctctggc cgtcttcacc 1920

atgtcatctg tggtcttcag caggtttcgg gactggctaa gacacctggc aggtccagag 1980

actggaatga ccctagatcc gccgccaccc gacccaccac cgcccgagcc accgccaccg 2040

gatcggaccc tgcagggaac acatgcccac ttgctgcatg aggaattgta atagcgatcc 2100

tgagcttgca cgcagacatt cccgcctttg cattggactt cggtagatgt cttctctacg 2160

aggaacgcac ctttctggtt acacccctcc tctgtctcct tcatcttttc tttcttgcgc 2220

tggattcgaa caaagaactt gagggagaag taggaatggg gagtgctcca ggagtcaggg 2280

tactcccagc tgacctccac ctgtgagttc ttcaaaggct tcatctgcaa gttcttgggc 2340

gggtctggtt tgatgatgtc cctgatgaag aagctggtgc tgtagttctc atatttattc 2400

tgctgccgtg cttccaacgc cagttcaatg ggcagggtct cctcggcagt tgggcaggtg 2460

acatcctcct ggcaggacac tgaatacttc tcatagtccc tttggtccag tgtgaccttc 2520

tctgcagaca gagacgccat tccacatgtc actgcccgag agtcagggga actgctactg 2580

ctcttgatgt tgaacttcaa gtccatgttt ctttgcacca gccatgagca cgtgaaccgt 2640

ccggagtaat ttggtgcttc acacttcagg aaagtcttgt ttttgaaatt ttttaaaatt 2700

tcagtggacc aaattccatt ttccttcttg tggagcagca gatgtgagtg gctcagagtc 2760

tcgcctcctt tgtggcaggt gtactggcca gcatctagaa actctttgac agtgatggtc 2820

agggtctttc cagagcctat gactccatgt ctctggtctg aggtccaggt gatgtcatct 2880

tcttcaggcg tgtcacaggt gaggttcact gtttctccag gggcatcggg agtccagtcc 2940

acctctacaa cataaacgtc tttctccagc tcccacatgg ccatgagtgg agacaccagc 3000

aaaacgatgg caaaccagga gatggttagc ttctgaggac acatggtggc gaccggtagc 3060

gctaggacgc gtaggacttg aggtcactgt gaggagtgat tagcaagggt gataggcagc 3120

tcttcagcat gggaggcaat ttatactgtt aatgctggaa aaataatatg ggggtgtcac 3180

gatgttcccg ggacaattga acgccttctg tatgaaacaa attttcctct taacgccttc 3240

tgtatgaaac aaattttcct cttaacgcct tctgtatgaa acaaattttc ctcttaacgc 3300

cttctgtatg aaacaaattt tcctcttaac gccttctgta tgaaacaaat tttcctctta 3360

acgccttctg tatgaaacaa attttcctct t 3391

<210> 33
<211> 3514
<212> DNA
<213> Artificial sequence

<220>
<223> Synthesized polynucleotide

<400> 33
atggcctcac cgttgacccg ctttctgtcg ctgaacctgc tgctgctggg tgagtcgatt 60

atcctgggga gtggagaagc tgaggtgcag ctggtgcaga gcggcgccga ggtgaagaag 120

cccggcgcca gcgtgaaggt gagctgcaag gccagcggct acaccttcag cgactacgag 180

atgcactggg tgcggcaggc ccccggccag ggcctggagt ggatgggcgc catccacccc 240

ggcagcggcg acaccgccta caaccagcgg ttcaagggcc gggtgaccat caccgccgac 300

aagagcacca gcaccgccta catggagctg agcagcctgc ggagcgagga caccgccgtg 360

```
tactactgcg cccggttcta cagctacgcc tactggggcc agggcaccct ggtgaccgtg      420
agcgccggtg gaggcggttc aggcggaggt ggttctggcg gtggcggatc ggacatcgtg      480
atgacccaga ccccccctgag cctgcccgtg acccccggcg agcccgccag catcagctgc      540
cggagcagcc agagcctggt gcacagcaac ggcaacacct acctgcagtg gtacctgcag      600
aagcccggcc agagccccca gctgctgatc tacaaggtga gcaaccggtt cagcggcgtg      660
cccgaccggt tcagcggcag cggcagcggc accgacttca ccctgaagat cagccgggtg      720
gaggccgagg acgtgggcgt gtactactgc agccagagca tctacgtgcc ctacaccttc      780
ggccagggca ccaagctgga gatcaaacgt actactacca agccagtgct gcgaactccc      840
tcacctgtgc accctaccgg gacatctcag ccccagagac cagaagattg tcggccccgt      900
ggctcagtga aggggaccgg attggacttc gcctgtgata tttacatctg ggcacccttg      960
gccggaatct gcgtggccct tctgctgtcc ttgatcatca ctctcatctg ctaccacagg     1020
agccgaaata gtagaaggaa cagactcctt caaagtgact acatgaacat gactccccgg     1080
aggcctgggc tcactcgaaa gccttaccag ccctacgccc ctgccagaga ctttgcagcg     1140
taccgcccca gcaggagtgc agagactgct gccaacctgc aggaccccaa ccagctctac     1200
aatgagctca atctagggcg aagagaggaa tatgacgtct tggagaagaa gcgggctcgg     1260
gatccagaga tgggaggcaa acagcagagg aggaggaacc cccaggaagg cgtatacaat     1320
gcactgcaga aagacaagat ggcagaagcc tacagtgaga tcggcacaaa aggcgagagg     1380
cggagaggca aggggcacga tggcctttac cagggtctca gcactgccac caaggacacc     1440
tatgatgccc tgcatatgca gaccctggcc taggtcgact cacacaaaaa accaacacac     1500
agatgtaatg aaaataaaga tattttattc gtacgttagg cggagctcag atagcccatc     1560
accctgttga tggtcacgac gcgggtgctg aaggcgtgaa gcaggatgca gagcttcatt     1620
ttcactctgt aagggtctgc ttctcccaca ggaggtttct ggcgcagagt ctcgccatta     1680
tgattcagag actgcatcag ctcatcgatg gccaccagca tgcccttgtc tagaatgatc     1740
tgctgatggt tgtgattctg aagtgctgcg ttgatggcct ggaactctgt ctggtacatc     1800
ttcaagtcct catagatgct accaaggcac agggtcatca tcaaagacgt cttctgtggg     1860
ggcaggcagc tccctcttgt tgtggaagaa gtctctctag tagccaggca actctcgttc     1920
ttgtgtagtt ccagtggtaa acaggtcttc aatgtgctgg tttggtcccg tgtgatgtct     1980
tcatgatcga tgtcttcagc agtgcaggaa taatgtttca gtttttctct ggccgtcttc     2040
accatgtcat ctgtggtctt cagcaggttt cgggactggc taagacacct ggcaggtcca     2100
gagactggaa tgaccctaga tccgccgcca cccgacccac caccgcccga gccaccgcca     2160
ccggatcgga ccctgcaggg aacacatgcc cacttgctgc atgaggaatt gtaatagcga     2220
```

tcctgagctt gcacgcagac attcccgcct ttgcattgga cttcggtaga tgtcttctct 2280

acgaggaacg cacctttctg gttacacccc tcctctgtct ccttcatctt ttctttcttg 2340

cgctggattc gaacaaagaa cttgagggag aagtaggaat ggggagtgct ccaggagtca 2400

gggtactccc agctgacctc cacctgtgag ttcttcaaag gcttcatctg caagttcttg 2460

ggcgggtctg gtttgatgat gtccctgatg aagaagctgg tgctgtagtt ctcatattta 2520

ttctgctgcc gtgcttccaa cgccagttca atgggcaggg tctcctcggc agttgggcag 2580

gtgacatcct cctggcagga cactgaatac ttctcatagt ccctttggtc cagtgtgacc 2640

ttctctgcag acagagacgc cattccacat gtcactgccc gagagtcagg ggaactgcta 2700

ctgctcttga tgttgaactt caagtccatg tttctttgca ccagccatga gcacgtgaac 2760

cgtccggagt aatttggtgc ttcacacttc aggaaagtct tgtttttgaa attttttaaa 2820

atttcagtgg accaaattcc attttccttc ttgtggagca gcagatgtga gtggctcaga 2880

gtctcgcctc ctttgtggca ggtgtactgg ccagcatcta gaaactcttt gacagtgatg 2940

gtcagggtct ttccagagcc tatgactcca tgtctctggt ctgaggtcca ggtgatgtca 3000

tcttcttcag gcgtgtcaca ggtgaggttc actgtttctc caggggcatc gggagtccag 3060

tccacctcta caacataaac gtctttctcc agctcccaca tggccatgag tggagacacc 3120

agcaaaacga tggcaaacca ggagatggtt agcttctgag gacacatggt ggcgaccggt 3180

agcgctagga cgcgtaggac ttgaggtcac tgtgaggagt gattagcaag ggtgataggc 3240

agctcttcag catgggaggc aatttatact gttaatgctg gaaaaataat atgggggtgt 3300

cacgatgttc ccgggacaat tgaacgcctt ctgtatgaaa caaattttcc tcttaacgcc 3360

ttctgtatga aacaaatttt cctcttaacg ccttctgtat gaaacaaatt ttcctcttaa 3420

cgccttctgt atgaaacaaa ttttcctctt aacgccttct gtatgaaaca aattttcctc 3480

ttaacgcctt ctgtatgaaa caaattttcc tctt 3514

<210> 34
<211> 3526
<212> DNA
<213> Artificial sequence

<220>
<223> Synthesized polynucleotide

<400> 34
atggcctcac cgttgacccg ctttctgtcg ctgaacctgc tgctgctggg tgagtcgatt 60

atcctgggga gtggagaagc tgaggtgcag ctggtgcaga gcggcgccga ggtgaagaag 120

cccggcgcca gcgtgaaggt gagctgcaag gccagcggct acaccttcag cgactacgag 180

atgcactggg tgcggcaggc ccccggccag ggcctggagt ggatgggcgc catccacccc 240

ggcagcggcg acaccgccta caaccagcgg ttcaagggcc gggtgaccat caccgccgac 300

```
aagagcacca gcaccgccta catggagctg agcagcctgc ggagcgagga caccgccgtg      360
tactactgcg cccggttcta cagctacgcc tactggggcc agggcaccct ggtgaccgtg      420
agcgccggtg gaggcggttc aggcggaggt ggttctggcg gtggcggatc ggacatcgtg      480
atgacccaga ccccccctgag cctgcccgtg acccccggcg agcccgccag catcagctgc     540
cggagcagcc agagcctggt gcacagcaac ggcaacacct acctgcagtg gtacctgcag      600
aagcccggcc agagccccca gctgctgatc tacaaggtga gcaaccggtt cagcggcgtg      660
cccgaccggt tcagcggcag cggcagcggc accgacttca ccctgaagat cagccgggtg      720
gaggccgagg acgtgggcgt gtactactgc agccagagca tctacgtgcc ctacaccttc      780
ggccagggca ccaagctgga gatcaaacgt actactacca agccagtgct gcgaactccc      840
tcacctgtgc accctaccgg gacatctcag ccccagagac cagaagattg tcggccccgt      900
ggctcagtga aggggaccgg attggacttc gcctgtgata tttacatctg ggcacccttg      960
gccggaatct gcgtggccct tctgctgtcc ttgatcatca ctctcatctg ctaccacagg     1020
agccgaaaat ggatcaggaa aaaattcccc cacatattca agcaaccatt taagaagacc     1080
actggagcag ctcaagagga agatgcttgt agctgccgat gtccacagga agaagaagga     1140
ggaggaggag gctatgagct gagcaggagt gcagagactg ctgccaacct gcaggacccc     1200
aaccagctct acaatgagct caatctaggg cgaagagagg aatatgacgt cttggagaag     1260
aagcgggctc gggatccaga gatgggaggc aaacagcaga ggaggaggaa cccccaggaa     1320
ggcgtataca atgcactgca gaaagacaag atggcagaag cctacagtga gatcggcaca     1380
aaaggcgaga ggcggagagg caaggggcac gatggccttt accagggtct cagcactgcc     1440
accaaggaca cctatgatgc cctgcatatg cagaccctgg cctaggtcga ctcacacaaa     1500
aaaccaacac acagatgtaa tgaaaataaa gatattttat tcgtacgtta ggcggagctc     1560
agatagccca tcaccctgtt gatggtcacg acgcgggtgc tgaaggcgtg aagcaggatg     1620
cagagcttca ttttcactct gtaagggtct gcttctccca caggaggttt ctggcgcaga     1680
gtctcgccat tatgattcag agactgcatc agctcatcga tggccaccag catgcccttg     1740
tctagaatga tctgctgatg gttgtgattc tgaagtgctg cgttgatggc ctggaactct     1800
gtctggtaca tcttcaagtc ctcatagatg ctaccaaggc acagggtcat catcaaagac     1860
gtcttctgtg gggcaggca gctccctctt gttgtggaag aagtctctct agtagccagg      1920
caactctcgt tcttgtgtag ttccagtggt aaacaggtct tcaatgtgct ggtttggtcc     1980
cgtgtgatgt cttcatgatc gatgtcttca gcagtgcagg aataatgttt cagtttttct     2040
ctggccgtct tcaccatgtc atctgtggtc ttcagcaggt ttcgggactg gctaagacac     2100
ctggcaggtc cagagactgg aatgacccta gatccgccgc cacccgaccc accaccgccc     2160
```

gagccaccgc caccggatcg gaccctgcag ggaacacatg cccacttgct gcatgaggaa 2220

ttgtaatagc gatcctgagc ttgcacgcag acattcccgc ctttgcattg gacttcggta 2280

gatgtcttct ctacgaggaa cgcacctttc tggttacacc cctcctctgt ctccttcatc 2340

ttttctttct tgcgctggat tcgaacaaag aacttgaggg agaagtagga atggggagtg 2400

ctccaggagt cagggtactc ccagctgacc tccacctgtg agttcttcaa aggcttcatc 2460

tgcaagttct tgggcgggtc tggtttgatg atgtccctga tgaagaagct ggtgctgtag 2520

ttctcatatt tattctgctg ccgtgcttcc aacgccagtt caatgggcag ggtctcctcg 2580

gcagttgggc aggtgacatc ctcctggcag gacactgaat acttctcata gtccctttgg 2640

tccagtgtga ccttctctgc agacagagac gccattccac atgtcactgc ccgagagtca 2700

ggggaactgc tactgctctt gatgttgaac ttcaagtcca tgtttctttg caccagccat 2760

gagcacgtga accgtccgga gtaatttggt gcttcacact tcaggaaagt cttgtttttg 2820

aaattttttta aaatttcagt ggaccaaatt ccattttcct tcttgtggag cagcagatgt 2880

gagtggctca gagtctcgcc tcctttgtgg caggtgtact ggccagcatc tagaaactct 2940

ttgacagtga tggtcagggt ctttccagag cctatgactc catgtctctg gtctgaggtc 3000

caggtgatgt catcttcttc aggcgtgtca caggtgaggt tcactgtttc tccaggggca 3060

tcgggagtcc agtccacctc tacaacataa acgtctttct ccagctccca catggccatg 3120

agtggagaca ccagcaaaac gatggcaaac caggagatgg ttagcttctg aggacacatg 3180

gtggcgaccg gtagcgctag gacgcgtagg acttgaggtc actgtgagga gtgattagca 3240

agggtgatag gcagctcttc agcatgggag gcaatttata ctgttaatgc tggaaaaata 3300

atatgggggt gtcacgatgt tcccgggaca attgaacgcc ttctgtatga aacaaatttt 3360

cctcttaacg ccttctgtat gaaacaaatt ttcctcttaa cgccttctgt atgaaacaaa 3420

ttttcctctt aacgccttct gtatgaaaca aattttcctc ttaacgcctt ctgtatgaaa 3480

caaattttcc tcttaacgcc ttctgtatga aacaaatttt cctctt 3526

<210> 35
<211> 3649
<212> DNA
<213> Artificial sequence

<220>
<223> Synthesized polynucleotide

<400> 35
atggcctcac cgttgacccg ctttctgtcg ctgaacctgc tgctgctggg tgagtcgatt 60

atcctgggga gtggagaagc tgaggtgcag ctggtgcaga gcggcgccga ggtgaagaag 120

cccggcgcca gcgtgaaggt gagctgcaag gccagcggct acaccttcag cgactacgag 180

atgcactggg tgcggcaggc ccccggccag ggcctggagt ggatgggcgc catccacccc 240

```
ggcagcggcg acaccgccta caaccagcgg ttcaagggcc gggtgaccat caccgccgac      300
aagagcacca gcaccgccta catggagctg agcagcctgc ggagcgagga caccgccgtg      360
tactactgcg cccggttcta cagctacgcc tactggggcc agggcaccct ggtgaccgtg      420
agcgccggtg gaggcggttc aggcggaggt ggttctggcg gtggcggatc ggacatcgtg      480
atgacccaga ccccccctgag cctgcccgtg acccccggcg agcccgccag catcagctgc      540
cggagcagcc agagcctggt gcacagcaac ggcaacacct acctgcagtg gtacctgcag      600
aagcccggcc agagccccca gctgctgatc tacaaggtga gcaaccggtt cagcggcgtg      660
cccgaccggt tcagcggcag cggcagcggc accgacttca ccctgaagat cagccgggtg      720
gaggccgagg acgtgggcgt gtactactgc agccagagca tctacgtgcc ctacaccttc      780
ggccagggca ccaagctgga gatcaaacgt actactacca agccagtgct gcgaactccc      840
tcacctgtgc accctaccgg gacatctcag ccccagagac cagaagattg tcggccccgt      900
ggctcagtga aggggaccgg attggacttc gcctgtgata tttacatctg ggcacccttg      960
gccggaatct gcgtggccct tctgctgtcc ttgatcatca ctctcatctg ctaccacagg     1020
agccgaaata gtagaaggaa cagactcctt caaagtgact acatgaacat gactccccgg     1080
aggcctgggc tcactcgaaa gccttaccag ccctacgccc ctgccagaga ctttgcagcg     1140
taccgcccca aatggatcag gaaaaaattc ccccacatat tcaagcaacc atttaagaag     1200
accactggag cagctcaaga ggaagatgct tgtagctgcc gatgtccaca ggaagaagaa     1260
ggaggaggag gaggctatga gctgagcagg agtgcagaga ctgctgccaa cctgcaggac     1320
cccaaccagc tctacaatga gctcaatcta gggcgaagag aggaatatga cgtcttggag     1380
aagaagcggg ctcgggatcc agagatggga ggcaaacagc agaggaggag gaacccccag     1440
gaaggcgtat acaatgcact gcagaaagac aagatggcag aagcctacag tgagatcggc     1500
acaaaaggcg agaggcggag aggcaagggg cacgatggcc tttaccaggg tctcagcact     1560
gccaccaagg acacctatga tgccctgcat atgcagaccc tggcctaggt cgactcacac     1620
aaaaaaccaa cacacagatg taatgaaaat aaagatattt tattcgtacg ttaggcggag     1680
ctcagatagc ccatcaccct gttgatggtc acgacgcggg tgctgaaggc gtgaagcagg     1740
atgcagagct tcattttcac tctgtaaggg tctgcttctc ccacaggagg tttctggcgc     1800
agagtctcgc cattatgatt cagagactgc atcagctcat cgatggccac cagcatgccc     1860
ttgtctagaa tgatctgctg atggttgtga ttctgaagtg ctgcgttgat ggcctggaac     1920
tctgtctggt acatcttcaa gtcctcatag atgctaccaa ggcacagggt catcatcaaa     1980
gacgtcttct gtgggggcag gcagctccct cttgttgtgg aagaagtctc tctagtagcc     2040
aggcaactct cgttcttgtg tagttccagt ggtaaacagg tcttcaatgt gctggtttgg     2100
```

```
tcccgtgtga tgtcttcatg atcgatgtct tcagcagtgc aggaataatg tttcagtttt     2160
tctctggccg tcttcaccat gtcatctgtg gtcttcagca ggtttcggga ctggctaaga     2220
cacctggcag gtccagagac tggaatgacc ctagatccgc cgccacccga cccaccaccg     2280
cccgagccac cgccaccgga tcggaccctg cagggaacac atgcccactt gctgcatgag     2340
gaattgtaat agcgatcctg agcttgcacg cagacattcc cgcctttgca ttggacttcg     2400
gtagatgtct tctctacgag gaacgcacct ttctggttac acccctcctc tgtctccttc     2460
atcttttctt tcttgcgctg gattcgaaca aagaacttga gggagaagta ggaatgggga     2520
gtgctccagg agtcagggta ctcccagctg acctccacct gtgagttctt caaaggcttc     2580
atctgcaagt tcttgggcgg gtctggtttg atgatgtccc tgatgaagaa gctggtgctg     2640
tagttctcat atttattctg ctgccgtgct tccaacgcca gttcaatggg cagggtctcc     2700
tcggcagttg ggcaggtgac atcctcctgg caggacactg aatacttctc atagtccctt     2760
tggtccagtg tgaccttctc tgcagacaga gacgccattc cacatgtcac tgcccgagag     2820
tcaggggaac tgctactgct cttgatgttg aacttcaagt ccatgtttct ttgcaccagc     2880
catgagcacg tgaaccgtcc ggagtaattt ggtgcttcac acttcaggaa agtcttgttt     2940
ttgaaatttt ttaaaatttc agtggaccaa attccatttt ccttcttgtg gagcagcaga     3000
tgtgagtggc tcagagtctc gcctcctttg tggcaggtgt actggccagc atctagaaac     3060
tctttgacag tgatggtcag ggtctttcca gagcctatga ctccatgtct ctggtctgag     3120
gtccaggtga tgtcatcttc ttcaggcgtg tcacaggtga ggttcactgt ttctccaggg     3180
gcatcgggag tccagtccac ctctacaaca taaacgtctt tctccagctc ccacatggcc     3240
atgagtggag acaccagcaa aacgatggca aaccaggaga tggttagctt ctgaggacac     3300
atggtggcga ccggtagcgc taggacgcgt aggacttgag gtcactgtga ggagtgatta     3360
gcaagggtga taggcagctc ttcagcatgg gaggcaattt atactgttaa tgctggaaaa     3420
ataatatggg ggtgtcacga tgttcccggg acaattgaac gccttctgta tgaaacaaat     3480
tttcctctta acgccttctg tatgaaacaa attttcctct taacgccttc tgtatgaaac     3540
aaattttcct cttaacgcct tctgtatgaa acaaattttc ctcttaacgc cttctgtatg     3600
aaacaaattt tcctcttaac gccttctgta tgaaacaaat tttcctctt                 3649
```